(19) Europäisches Patentamt
European Patent Office
Office européen des brevets

(11) Veröffentlichungsnummer: **0 232 224 B1**

(12) **EUROPÄISCHE PATENTSCHRIFT**

(45) Veröffentlichungstag der Patentschrift: **25.11.92**

(51) Int. Cl.5: **C07D 211/58**, C07D 401/14, C07D 401/12, C07F 9/58, C08K 5/34

(21) Anmeldenummer: **87810052.8**

(22) Anmeldetag: **27.01.87**

Die Akte enthält technische Angaben, die nach dem Eingang der Anmeldung eingereicht wurden und die nicht in dieser Patentschrift enthalten sind.

# (54) Neue Piperidinverbindungen.

(30) Priorität: **30.01.86 IT 1923086**

(43) Veröffentlichungstag der Anmeldung: **12.08.87 Patentblatt 87/33**

(45) Bekanntmachung des Hinweises auf die Patenterteilung: **25.11.92 Patentblatt 92/48**

(84) Benannte Vertragsstaaten: **BE DE FR GB IT**

(56) Entgegenhaltungen:
**EP-A- 0 163 245**
**DE-A- 2 517 284**
**DE-A- 2 612 314**
**GB-A- 1 394 770**
**GB-A- 2 176 482**

**JOURNAL OF MACROMOLECULAR SCIENCE. REVIEWS IN MACROMOLECULAR CHEMISTRY AND PHYSICS Band C22, Nr. 2, 1982-1983, Seiten 169-202, N.Y., US.; M. DAGONNEAU et al.: "Sterically hindered amines and nitroxyls as polymer stabilizers"**

(73) Patentinhaber: **CIBA-GEIGY AG**
**Klybeckstrasse 141**
**CH-4002 Basel(CH)**
(84) Benannte Vertragsstaaten: **BE DE FR GB**

(73) Patentinhaber: **CIBA-GEIGY S.p.A.**
**Strada Statale 233-Km. 20,5**
**Origgio(IT)**
(84) Benannte Vertragsstaaten: **IT**

(72) Erfinder: **Cantatore, Guiseppe, Dr.**
**Via Generale Planelli, 68**
**I-70032 Bitonto (Bari)(IT)**
Erfinder: **Borzatto, Valerio, Dr.**
**Via Novelli 2**
**Bologna(IT)**

EP 0 232 224 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Vorliegende Erfindung betrifft neue Piperidinverbindungen, deren Verwendung sowie das mittels dieser Verbindungen gegen thermischen, oxidativen und/oder licht-induzierten Abbau stabilisierte organische Material.

Es ist bekannt, dass synthetische Polymere fortschreitende Änderungen in ihren physikalischen Eigenschaften, wie Verlust der mechanischen Festigkeit und Farbänderungen, erleiden, wenn sie dem Sonnenlicht oder anderen Quellen ultravioletten Lichts ausgesetzt sind. Zur Verzögerung der schädlichen Wirkung des Sonnenlichts auf synthetische Polymere wurde die Verwendung verschiedener Zusätze mit Lichtschutzeigenschaften vorgeschlagen.

In FR-A-2 268 011 sind substituierte N,N'-Diphenylformamidinverbindungen und deren Verwendung als Lichtschuztmittel beschrieben. JP-A-85-84 258 offenbart ein Verfahren zur Herstellung substituierter N,N'-Formamidinderivate.

GB-A-1 394 770, DE-A-2 612 314 und EP-A-163 245 beschreiben Lichtschutzmittel, die 2,2,6,6-Tetraalkyl-4-piperidinylgruppen enthalten.

Gegenstand vorliegender Erfindung sind neue Piperidinverbindungen der Formel (I),

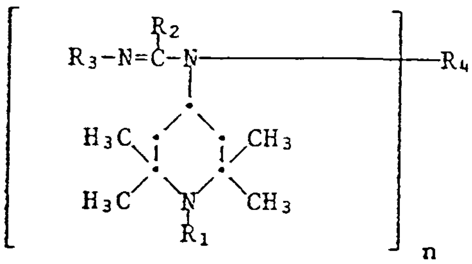

(I)

worin $R_1$ Wasserstoff, $O^\bullet$, CN, NO, Cyanomethyl, $C_1$-$C_{12}$-Alkyl, $C_3$-$C_{12}$-Alkenyl oder $C_3$-$C_{12}$-Alkinyl unter der Voraussetzung, dass das an das Stickstoffatom gebundene Kohlenstoffatom primär ist, $C_7$-$C_{12}$-Aralkyl, Methylbenzyl, t-Butylbenzyl, Hydroxybenzyl, $C_1$-$C_{12}$-Acyl, 2,3-Epoxypropyl, OH-mono-substituiertes $C_2$-$C_6$-Alkyl oder 2,3-Dihydroxypropyl ist, $R_2$ für Wasserstoff, $C_1$-$C_{18}$-Alkyl, $C_5$-$C_{18}$-Cycloalkyl, Methylcyclohexyl, Trimethylcyclohexyl, t-Butylcyclohexyl, $C_6$-$C_{18}$-Aryl, Methylphenyl, Dimethylphenyl, Trimethylphenyl, t-Butylphenyl, Methoxyphenyl, Ethoxyphenyl, Hydroxyphenyl, 3,5-Di-t-butyl-4-hydroxyphenyl, $C_7$-$C_{18}$-Aralkyl, Methylbenzyl, Hydroxybenzyl, 3,5-Di-t-butyl-4-hydroxybenzyl oder 2-(3,5-Di-t-butyl-4-hydroxyphenyl)-ethyl, $R_3$ für $C_6$-$C_{18}$-Aryl, Methylphenyl, Dimethylphenyl, Trimethylphenyl, Ethylphenyl, Diethylphenyl, Hydroxyphenyl, 3,5-Dit-butyl-4-hydroxyphenyl, Methoxyphenyl, Ethoxyphenyl, 4-Ethoxycarbonylphenyl, 4-(2,2,6,6-Tetramethyl-4-piperidyloxycarbonyl)-phenyl, 4-(1,2,2,6,6-Pentamethyl-4-piperidyloxycarbonyl)-phenyl oder eine Gruppe der Formel (II),

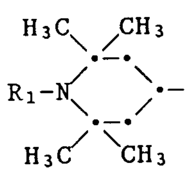

(II)

worin $R_1$ die oben angegebene Bedeutung hat, n für eine ganze Zahl von 1 bis 4 und, wenn n 1 ist, $R_4$ Wasserstoff,

CN, $-COR_5$, $-COOR_6$, $-CO-R_7-COOR_8$,

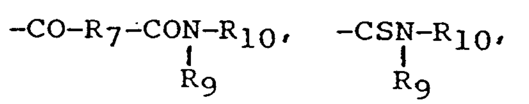

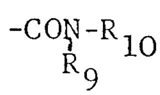 , $-SO_2R_{11}$, 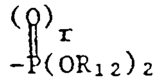 oder 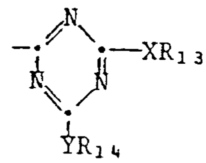 ist,

worin $R_5$ Wasserstoff, $C_1$-$C_{18}$-Alkyl, $C_5$-$C_{18}$-Cycloalkyl, Methylcyclohexyl, t-Butylcyclohexyl, $C_2$-$C_{18}$-Alkenyl, $C_6$-$C_{18}$-Aryl, Methylphenyl, Dimethylphenyl, Trimethylphenyl, Ethylphenyl, t-Butylphenyl, Methoxyphenyl, Ethoxyphenyl, Hydroxyphenyl, 3,5-Di-t-butyl-4-hydroxyphenyl, $C_7$-$C_{18}$-Aralkyl, 3,5-Di-t-butyl-4-hydroxybenzyl, 2-(3,5-Di-t-butyl-4-hydroxyphenyl)-ethyl oder durch $C_1$-$C_{18}$-Alkoxy, $C_2$-$C_{18}$-Dialkylamino oder eine Gruppe der Formel (III)

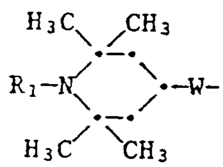 (III)

substituiertes $C_1$-$C_{10}$-Alkyl bedeutet, wobei $R_1$ die oben angegebene Bedeutung hat und W -O- oder $>$N-$R_{15}$ darstellt, worin $R_{15}$ für Wasserstoff, $C_1$-$C_{18}$-Alkyl, $C_5$-$C_{18}$-Cycloalkyl, Methylcyclohexyl, Dimethylcyclohexyl, Trimethylcyclohexyl, $C_3$-$C_{18}$-Alkenyl, $C_6$-$C_{18}$-Aryl, Methylphenyl, Dimethylphenyl, Trimethylphenyl, t-Butylphenyl, Methoxyphenyl, Ethoxyphenyl, Hydroxyphenyl, 3,5-Di-t-butyl-4-hydroxyphenyl, $C_7$-$C_{18}$-Aralkyl, Methylbenzyl, Hydroxybenzyl, 3,5-Di-t-butyl-4-hydroxybenzyl oder eine Gruppe der Formel (II) steht, $R_6$ $C_1$-$C_{18}$-Alkyl, durch OH, $C_1$-$C_{18}$-Alkoxy oder $C_2$-$C_{18}$-Dialkylamino substituiertes $C_2$-$C_6$-Alkyl, $C_5$-$C_{18}$-Cycloalkyl, Methylcyclohexyl, Dimethylcyclohexyl, Trimethylcyclohexyl, t-Butylcyclohexyl, $C_3$-$C_{18}$-Alkenyl, $C_6$-$C_{18}$-Aryl, Methylphenyl, Dimethylphenyl, Trimethylphenyl, Ethylphenyl, Isopropylphenyl, t-Butylphenyl, Di-t-butylphenyl, 2,6-Di-t-butyl-4-methylphenyl, Methoxyphenyl, Ethoxyphenyl, $C_7$-$C_{18}$-Aralkyl, Methylbenzyl, 3,5-Di-t-butyl-4-hydroxybenzyl oder eine Gruppe der Formel (II) und $R_7$ eine direkte Bindung, $C_1$-$C_{18}$-Alkylen, $C_2$-$C_{20}$-Alkyliden, $C_7$-$C_{20}$-Aralkyliden, $C_6$-$C_{10}$-Cycloalkylen, Methylcyclohexylen, $C_2$-$C_{18}$-Alkenylen oder $C_6$-$C_{10}$-Arylen bedeuten, $R_8$ die für $R_6$ angegebene Bedeutung hat oder Wasserstoff oder ein Alkalimetall darstellt, $R_9$ und $R_{10}$, die gleich oder verschieden sind, die für $R_{15}$ angegebene Bedeutung haben, oder $R_9$ und $R_{10}$ zusammen mit dem Stickstoffatom, an das sie gebunden sind, 1-Pyrrolidinyl, 1-Piperidyl, 4-Morpholinyl, 1-Hexahydroazepinyl oder 4-Methyl-1-piperazinyl bilden, $R_{11}$ $C_1$-$C_{12}$-Alkyl, $C_6$-$C_{12}$-Aryl, Methylphenyl, Dimethylphenyl, Trimethylphenyl, Methoxyphenyl oder Ethoxyphenyl, r 0 oder 1 und $R_{12}$ $C_1$-$C_{12}$-Alkyl oder eine Gruppe der Formel (II) bedeuten, oder die beiden Gruppen $R_{12}$ zusammen $C_2$-$C_{12}$-Alkylen oder eine Gruppe

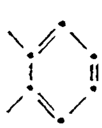 , 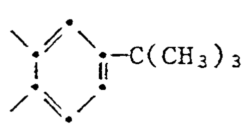 oder 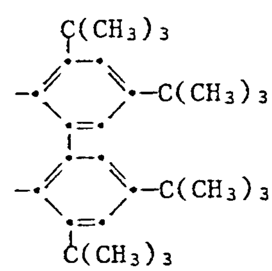

darstellen und zusammen mit dem Phosphoratom und den beiden Sauerstoffatomen einen 5- bis 7-gliedrigen Ring bilden, $R_{13}$ und $R_{14}$, die gleich oder verschieden sind, die für $R_6$ angegebene Bedeutung haben oder für Wasserstoff stehen, und X und Y, die gleich oder verschieden sind, eine direkte Bindung, -O- oder $>$N-$R_{16}$ bedeuten, wobei $R_{16}$ die für $R_{15}$ angegebene Bedeutung hat oder OH-monosubstituiertes $C_2$-$C_6$-Alkyl darstellt, oder die Gruppen $R_{13}$X- und $R_{14}$Y-, die gleich oder verschieden sind, 1-Pyrrolidinyl, 1-Piperidyl, 4-Morpholinyl, 1-Hexahydroazepinyl oder 4-Methyl-1-piperazinyl darstellen, und

wenn n = 2,
$R_4$ -CO-, $-SO_2-$, $-COO-R_{17}-OOC-$, $-COCOO-R_{17}-OOCOO-$, $-CO-R_{18}-CO-$,

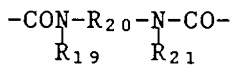 , 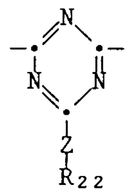 , 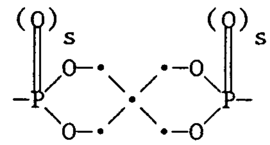 ,

$(-CO-R_7-COO)_2Me$ oder 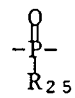 ist,

worin $R_{17}$ $C_2$-$C_{18}$-Alkylen, in der Kette durch 1 oder 2 Sauerstoffatome substituiertes $C_4$-$C_{12}$-Alkylen, $C_6$-$C_{18}$-Cycloalkylen, Cyclohexylendimethylen, Methylendicyclohexylen, Isopropylidendicyclohexylen, $C_4$-$C_8$-Alkenylen, $C_6$-$C_{18}$-Arylen, $C_8$-$C_{12}$-Aralkylen oder eine Gruppe der Formel (IV),

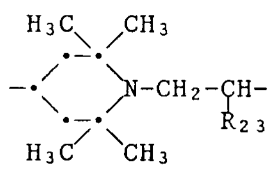 (IV)

worin $R_{23}$ für Wasserstoff, $C_1$-$C_4$-Alkyl oder Phenyl steht, und $R_{18}$ eine direkte Bindung, $C_1$-$C_{18}$-Alkylen, $C_2$-$C_{20}$-Alkyliden, $C_7$-$C_{20}$-Aralkyliden, $C_6$-$C_{10}$-Cycloalkylen,
Methylcyclohexylen, $C_6$-$C_{10}$-Arylen oder
in der Kette durch 1 oder 2 Sauerstoffatome oder 1 oder2 Gruppen $>N-R_{24}$, wobei $R_{24}$ die für $R_{15}$ angegebene Bedeutung hat, substituiertes $C_2$-$C_{12}$-Alkylen bedeuten, $R_{19}$ und $R_{21}$, die gleich oder verschieden sind, die für $R_{15}$ angegebene Bedeutung haben, $R_{20}$ $C_2$-$C_{18}$-Alkylen, $C_6$-$C_{18}$-Cycloalkylen, Cyclohexylendimethylen, Methylendicyclohexylen, $C_6$-$C_{18}$-Arylen oder $C_8$-$C_{12}$-Aralkylen bedeutet, $R_{22}$ die für $R_{13}$ und $R_{14}$ angegebene Bedeutung und Z die für X und Y angegebene Bedeutung haben, oder $-ZR_{22}$ 1-Pyrrolidinyl, 1-Piperidyl, 4-Morpholinyl, 1-Hexahydroazepinyl, 4-Methyl-1-piperazinyl oder Halogen darstellt, $R_{25}$ für $C_1$-$C_4$-Alkyl und s für 0 oder 1 stehen, $R_7$ die oben angegebene Bedeutung hat, Me für Ba, Ca, Co", Mg, Mn", Ni", Sn" oder Zn steht, und wenn n = 3, $R_4$ aliphatisches $C_4$-$C_{18}$-Triacyl, ein Triacylrest, welcher sich von Zitronensäure ableitet, durch ein Stickstoffatom substituiertes aliphatisches $C_6$-$C_{18}$-Triacyl, ein Triacylrest, welcher sich von 1,2,4-Benzoltricarbonsäure oder 1,3,5-Benzoltricarbonsäure ableitet, eine Gruppe

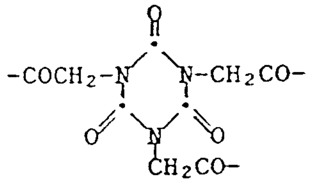 ,
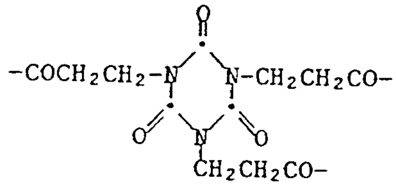 ,

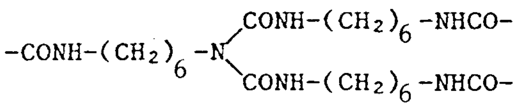 oder 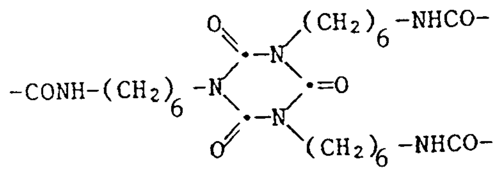

oder eine 1,3,5-Triazin-2,4,6-triylgruppe, eine ≡P-Gruppe, eine ≡PO-Gruppe oder eine Gruppe $(-CO-R_7-COO)_3Al$ mit $R_7$ in der oben angegebenen Bedeutung ist,
und wenn n = 4, $R_4$ aliphatisches $C_6$-$C_{18}$-Tetraacyl, durch zwei Stickstoffatome substituiertes aliphatisches $C_{10}$-$C_{18}$-Tetraacyl, aromatisches $C_{10}$-$C_{18}$-Tetraacyl, cycloaliphatisches $C_{10}$-$C_{22}$-Tetraacyl oder eine Gruppe $(-CO-R_7-COO)_4Sn^{IV}$ mit $R_7$ in der angegebenen Bedeutung ist.

$R_1$, $R_{11}$ und $R_{12}$ als $C_1$-$C_{12}$-Alkyl sind beispielsweise Methyl, Ethyl, Propyl, Isopropyl, Butyl, Isobutyl, t-Butyl, Pentyl, Hexyl, Heptyl, Octyl, 2-Ethylhexyl, Decyl oder Dodecyl. Geradkettiges oder verzweigtes $C_1$-$C_4$-Alkyl wird bevorzugt. Methyl ist besonders bevorzugt für $R_1$.

$R_1$ als $C_3$-$C_{12}$-Alkenyl ist beispielsweise Allyl, 2-Methallyl, 2-Butenyl, 2-Hexenyl oder 10-Undecenyl. Allyl wird besonders bevorzugt.

$R_1$ als $C_3$-$C_{12}$-Alkinyl kann vorzugsweise Propargyl sein.

$R_1$ als $C_7$-$C_{12}$-Aralkyl ist beispielsweise Benzyl.

$R_1$ als $C_1$-$C_{12}$-Acyl kann eine aliphatische oder aromatische $C_1$-$C_{12}$-Acylgruppe darstellen. $C_1$-$C_{12}$-Alkanoyl, $C_3$-$C_{12}$-Alkenoyl, $C_3$-$C_{12}$-Alkinoyl und gegebenenfalls durch $C_1$-$C_4$-Alkyl und/oder OH substituiertes Benzoyl sind bevorzugt. Beispiele dafür sind Formyl, Acetyl, Propionyl, Butyryl, Caproyl, Capryloyl, Caprinoyl, Lauroyl, Benzoyl, Acryloyl, Methacryloyl und Crotonyl.

$R_1$, $R_6$, $R_{13}$, $R_{14}$, $R_{16}$ und $R_{22}$ als OH-monosubstituiertes $C_2$-$C_6$-Alkyl sind beispielsweise 2-Hydroxyethyl, 2-Hydroxypropyl oder 3-Hydroxypropyl.

$R_2$, $R_5$, $R_6$, $R_8$, $R_9$, $R_{10}$, $R_{13}$, $R_{14}$, $R_{15}$, $R_{16}$, $R_{19}$, $R_{21}$, $R_{22}$ und $R_{24}$ als $C_1$-$C_{18}$-Alkyl sind beispielsweise Methyl, Ethyl, Propyl, Isopropyl, Butyl, Isobutyl, 2-Butyl, t-Butyl, Pentyl, Isopentyl , Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Octadecyl oder Heptadecyl. Vorzugsweise ist $R_2$ $C_1$-$C_4$-Alkyl, insbesondere Methyl.

$R_2$, $R_5$, $R_6$, $R_8$, $R_9$, $R_{10}$, $R_{13}$, $R_{14}$, $R_{15}$, $R_{16}$, $R_{19}$, $R_{21}$, $R_{22}$ und $R_{24}$ als $C_5$-$C_{18}$-Cycloalkyl sind beispielsweise Cyclopentyl, Cyclohexyl, Cyclooctyl und Cyclododecyl.

$R_2$, $R_3$, $R_5$, $R_6$, $R_8$, $R_9$, $R_{10}$, $R_{13}$, $R_{14}$, $R_{15}$, $R_{16}$, $R_{19}$, $R_{21}$, $R_{22}$ und $R_{24}$ als $C_6$-$C_{18}$-Aryl sind beispielsweise Phenyl oder Naphthyl.

$R_2$, $R_5$, $R_6$, $R_8$, $R_9$, $R_{10}$, $R_{13}$, $R_{14}$, $R_{15}$, $R_{16}$, $R_{19}$, $R_{21}$, $R_{22}$ und $R_{24}$ als $C_7$-$C_{18}$-Aralkyl können beispielsweise Benzyl sein.

$R_5$ als $C_2$-$C_{18}$-Alkenyl ist beispielsweise Vinyl, Propenyl, Allyl, Butenyl, Methylallyl, Hexenyl, Decenyl oder Heptadecenyl.

$R_5$ als durch $C_1$-$C_{18}$-Alkoxy oder $C_2$-$C_{18}$-Dialkylamino substituiertes $C_1$-$C_{10}$-Alkyl ist beispielsweise Ethoxymethyl, Butoxymethyl, Octoxyethyl, 2-Ethoxyethyl, 2-Butoxyethyl, 3-Methoxypropyl, 3-Butoxypropyl, Diethylaminomethyl, Dibutylaminomethyl, 2-Diethylaminoethyl, 2-Dibutylaminoethyl oder 3-Diethylaminopropyl.

$R_6$, $R_8$, $R_{13}$, $R_{14}$ und $R_{22}$ als durch $C_1$-$C_{18}$-Alkoxy oder $C_2$-$C_{18}$-Dialkylamino substituiertes $C_2$-$C_6$-Alkyl sind beispielsweise 2-Methoxyethyl, 2-Ethoxyethyl, 2-Butoxyethyl, 2-Octoxyethyl, 3-Ethoxypropyl, 3-Butoxypropyl, 4-Butoxybutyl, 2-Diethylaminoethyl, 2-Dibutylaminoethyl oder 3-Dibutylaminopropyl.

$R_6$, $R_8$, $R_9$, $R_{10}$, $R_{13}$, $R_{14}$, $R_{15}$, $R_{16}$, $R_{19}$, $R_{21}$, $R_{22}$ und $R_{24}$ als $C_3$-$C_{18}$-Alkenyl sind beispielsweise Allyl, Butenyl, Methylallyl, Hexenyl, Decenyl, Undecenyl oder Oleyl.

$R_7$ und $R_{18}$ als $C_1$-$C_{18}$-Alkylen sind beispielsweise Methylen, Ethylen, Propylen, Trimethylen, Tetramethylen, Pentamethylen, Hexamethylen, Heptamethylen, Octamethylen, Decamethylen oder Dodecamethylen. $C_1$-$C_6$-Alkylen wird dabei bevorzugt, und Ethylen ist besonders bevorzugt.

$R_7$ und $R_{18}$ als $C_2$-$C_{20}$-Alkyliden sind beispielsweise Ethyliden, Propyliden, Butyliden, Pentyliden, 4-Methylpentyliden, Heptyliden, Nonyliden, Tridecyliden, Nonadecyliden, 1-Methylethyliden, 1-Ethylpropyliden oder 1-Ethylpentyliden.

$R_7$ und $R_{18}$ als $C_7$-$C_{20}$-Aralkyliden können $C_7$-$C_{20}$-Phenylalkyliden sein, wie Z. B. Benzyliden, 2-Phenylethyliden oder 1-Phenyl-2-hexyliden.

$R_7$, $R_{17}$, $R_{18}$ und $R_{20}$ als $C_6$-$C_{18}$-Cycloalkylen sind beispielsweise Cyclohexylen.

$R_7$ als $C_2$-$C_{18}$-Alkenylen ist beispielsweise Vinylen, Methylvinylen, Octenylethylen oder Dodecenylethylen.

$R_7$ und $R_{18}$ als $C_6$-$C_{10}$-Arylen sind beispielsweise 1,2-Phenylen, 1,3-Phenylen oder 1,4-Phenylen. $R_{17}$ und $R_{20}$ als $C_6$-$C_{18}$-Arylen sind vorzugsweise Phenylen oder können Biphenylen oder Naphthylen sein.

$R_8$ als Alkalimetall kann Li, Na oder K sein.

$R_{11}$ als $C_6$-$C_{12}$-Aryl ist beispielsweise Phenyl oder Naphtyl.

Wenn die beiden Gruppen $R_{12}$ zusammen $C_2$-$C_{12}$-Alkylen darstellen und zusammen mit dem Phosphoratom und den beiden Sauerstoffatomen einen 5- bis 7-gliedrigen heterocyclischen Ring bilden, ist die $C_2$-$C_{12}$-Alkylengruppe vorzugsweise eine geradkettige oder verzweigte Alkylengruppe mit 2 bis 7 Kohlenstoffatomen. Beispiele für $R_4$ als eine Gruppe

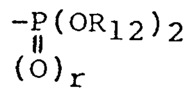

sind:

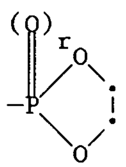 , 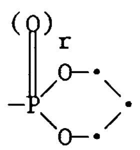 , 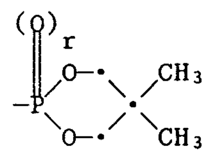 , 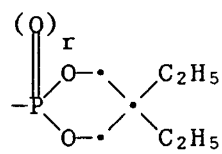 ,

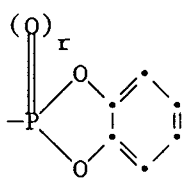 , 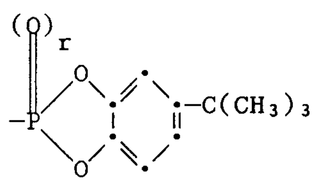 und 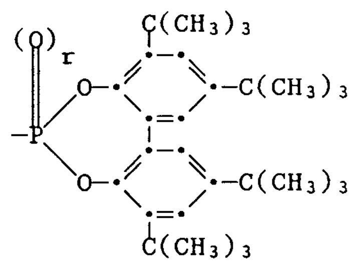 .

$-ZR_{22}$ kann auch Halogen, vorzugsweise Cl, sein.

$R_{17}$ und $R_{20}$ als $C_2$-$C_{18}$-Alkylen sind beispielsweise Ethylen, Propylen, Trimethylen, Tetramethylen, Pentamethylen, 2,2-Di-methyl-1,3-propandiyl, Hexamethylen, Octamethylen, Decamethylen oder Dodecamethylen. Dabei ist $C_2$-$C_6$-Alkylen bevorzugt.

$R_{17}$ als in der Kette durch 1 oder 2 Sauerstoffatome substituiertes $C_4$-$C_{12}$-Alkylen ist beispielsweise 3-Oxapentan-1,5-diyl oder 3,6-Dioxaoctan-1,8-diyl.

$R_{17}$ als $C_4$-$C_8$-Alkenylen ist beispielsweise 2-Buten-1,4-diyl.

$R_{17}$ und $R_{20}$ als $C_8$-$C_{12}$-Aralkylen sind beispielsweise Alkylenphenylen-alkylen mit 8 bis 12 Kohlenstoffatomen, vorzugsweise gegebenenfalls durch $C_1$-$C_4$-Alkyl substituiertes Dimethylenphenylen (Xylylen).

$R_{18}$ als in der Kette durch 1 oder 2 Sauerstoffatome substituiertes $C_2$-$C_{12}$-Alkylen ist beispielsweise 2-Oxapropan-1,3-diyl, 2,7-Dioxaoctan-1,8-diyl oder 2,6-Dioxa-4,4-dimethyl-1,7-heptandiyl.

$R_{23}$ und $R_{25}$ als $C_1$-$C_4$-Alkyl sind beispielsweise Methyl, Ethyl, Propyl oder Butyl.

$R_4$ als aliphatisches $C_4$- $C_{18}$-Triacyl kann $C_4$-$C_{18}$-Alkantrioyl sein. Bevorzugte Beispiele solcher Triacyle leiten sich von Methantricarbonsäure, 1,1,2-Ethantricarbonsäure, 1,2,3-Propantricarbonsäure, oder 1,2,3-Butantricarbonsäure ab.

$R_4$ als durch ein Stickstoffatom substituiertes aliphatisches $C_6$-$C_{18}$-Triacyl ist beispielsweise

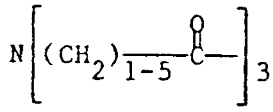

Die Gruppe $N(CH_2CO-)_3$ ist besonders bevorzugt.

$R_4$ als aliphatisches $C_6$-$C_{18}$-Tetraacyl kann $C_6$-$C_{18}$-Alkantetraoyl sein. Bevorzugte Beispiele sind solche Tetraacyle, die sich von 1,1,3,3-Propantetracarbonsäure oder 1,2,3,4-Butantetracarbonsäure ableiten.

$R_4$ als durch zwei Stickstoffatome substituiertes aliphatisches $C_{10}$-$C_{18}$-Tetraacyl ist beispielsweise eine Gruppe der Formel

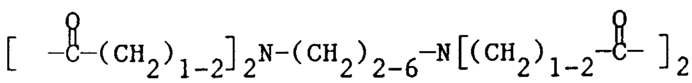

Bevorzugt wird sich von Ethylendiamintetraessigsäure ableitendes Tetraacyl.

$R_4$ als aromatisches $C_{10}$-$C_{18}$-Tetraacyl ist beispielsweise das sich von 1,2,4,5-Benzoltetracarbonsäure ableitende Tetraacyl.

$R_4$ als cycloaliphatisches $C_{10}$-$C_{22}$-Tetraacyl kann Cycloalkantetracarbonyl mit 10 bis 22 Kohlenstoffatomen oder Cycloalkentetracarbonyl mit 10 bis 22 Kohlenstoffatomen sein. Als Beispiele seien die folgenden Gruppen angegeben:

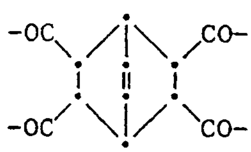 , 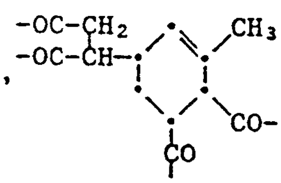 , 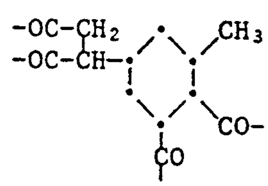 .

Solche Verbindungen der Formel (I) sind bevorzugt, worin $R_1$ für Wasserstoff, Methyl, Allyl, Benzyl oder Acetyl, $R_2$ für Wasserstoff, $C_1$-$C_{12}$-Alkyl $C_6$-$C_9$-Cycloalkyl oder $C_6$-$C_9$-Aryl, $R_3$ für $C_6$-$C_{12}$-Aryl oder eine Gruppe der Formel (II), n für 1, 2 oder 3, und, wenn n 1 ist, $R_4$ für Wasserstoff, $-COR_5$, $-COOR_6$, $-CO-R_7-COOR_8$,

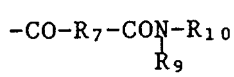 , 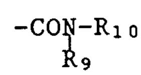 ,

$-SO_2R_{11}$ oder 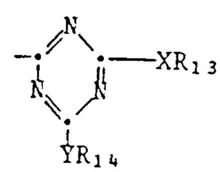

stehen, worin $R_5$ Wasserstoff, $C_1$-$C_{18}$-Alkyl, $C_6$-$C_9$-Cycloalkyl, $C_2$-$C_4$-Alkenyl, $C_6$-$C_8$-Aryl, $C_7$-$C_{16}$-Aralkyl oder durch eine Gruppe der Formel (III), worin W -O- oder $>N-R_{15}$ und $R_{15}$ Wasserstoff, $C_1$-$C_{12}$-Alkyl oder $C_6$-$C_9$-Cycloalkyl darstellen, substituiertes $C_1$-$C_3$-Alkyl, $R_6$ $C_1$-$C_{18}$-Alkyl, durch $C_1$-$C_4$-Alkoxy substituiertes $C_2$-$C_6$-Alkyl, $C_6$-$C_{12}$-Cycloalkyl oder eine Gruppe der Formel (II), $R_7$ eine direkte Bindung, $C_1$-$C_{10}$-Alkylen, $C_6$-$C_8$-Cycloalkylen, Vinylen oder Phenylen, $R_8$ Wasserstoff, $C_1$-$C_{12}$-Alkyl, $C_6$-$C_9$-Cycloalkyl, Allyl

oder eine Gruppe der Formel (II), $R_9$ und $R_{10}$, die gleich oder verschieden sind, Wasserstoff, $C_1$-$C_{12}$-Alkyl, $C_6$-$C_9$-Cycloalkyl, Allyl oder eine Gruppe der Formel (II) bedeuten oder zusammen mit dem Stickstoffatom, an das sie gebunden sind, 1-Pyrrolidinyl, 1-Piperidyl, 4-Morpholinyl, 1-Hexahydroazepinyl oder 4-Methyl-1-piperazinyl bilden, $R_{11}$ Methyl, Ethyl oder $C_6$-$C_9$-Aryl, $R_{13}$ und $R_{14}$, die gleich oder verschieden sind, Wasserstoff, $C_1$-$C_{12}$-Alkyl, $C_6$-$C_9$-Cycloalkyl, Allyl oder eine Gruppe der Formel (II) sowie X und Y, die gleich oder verschieden sind, -O- oder $>$N-$R_{16}$, wobei $R_{16}$ Wasserstoff, $C_1$-$C_{12}$-Alkyl, OH-monosubstituiertes $C_2$-$C_4$-Alkyl, $C_6$-$C_9$-Cycloalkyl, Allyl oder eine Gruppe der Formel (II) ist, bedeuten oder die Gruppen $R_{13}$X-und $R_{14}$Y-, die gleich oder verschieden sind, eine 1-Pyrrolidinyl-, 1-Piperidyl-, 4-Morpholinyl- oder 1-Hexahydroazepinylgruppe bedeuten, oder, wenn n 2 ist, $R_4$ für -CO-, -COO-$R_{17}$-OOC-, -CO-$R_{18}$-CO-,

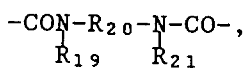,
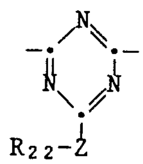
oder
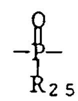

steht, worin $R_{17}$ $C_2$-$C_{12}$-Alkylen, in der Kette durch 1 oder 2 Sauerstoffatome substituiertes $C_4$-$C_{12}$-Alkylen, $C_6$-$C_{15}$-Cycloalkylen, 2-Buten-1,4-diyl oder eine Gruppe der Formel (IV), worin $R_{23}$ für Wasserstoff oder Methyl steht, $R_{18}$ eine direkte Bindung, $C_1$-$C_{12}$-Alkylen, $C_2$-$C_{12}$-Alkyliden, in der Kette durch eine oder zwei Gruppen $>$N-$R_{24}$ substituiertes $C_2$-$C_8$-Alkylen, wobei $R_{24}$ für $C_1$-$C_8$-Alkyl, $C_6$-$C_9$-Cycloalkyl oder eine Gruppe der Formel (II) steht, $R_{19}$ und $R_{21}$, die gleich oder verschieden sind, Wasserstoff, $C_1$-$C_8$-Alkyl, $C_6$-$C_9$-Cycloalkyl oder eine Gruppe der Formel (II), $R_{20}$ $C_2$-$C_{10}$-Alkylen, $C_6$-$C_{15}$-Cycloalkylen, $C_6$-$C_{15}$-Arylen oder Xylylen, $R_{22}$ Wasserstoff, $C_1$-$C_{12}$-Alkyl, OH-monosubstituiertes $C_2$-$C_4$-Alkyl, $C_6$-$C_9$-Cycloalkyl, Allyl oder eine Gruppe der Formel (II) und Z -O- oder $>$N-$R_{16}$ mit $R_{16}$ wie oben definiert bedeuten oder -$ZR_{22}$ Halogen und $R_{25}$ $C_1$-$C_4$-Alkyl bedeuten, und, wenn n 3 ist, $R_4$ aliphatisches $C_4$-$C_8$-Triacyl, eine Gruppe N-$(CH_2CO-)_3$,
einen Triacylrest, welcher sich von 1,2,4-Benzoltricarbonsäure oder 1,3,5-Benzoltricarbonsäure ableitet, oder für eine Gruppe

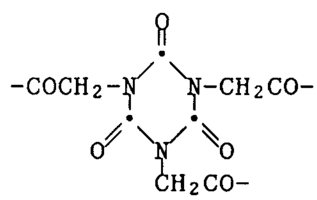
oder
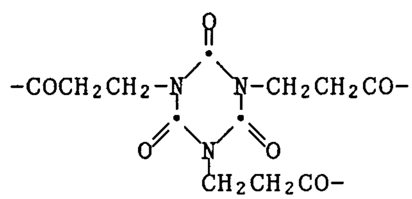

oder eine 1,3,5-Triazin-2,4,6-triylgruppe bedeutet.

Solche Verbindungen der Formel (I) sind bevorzugt, worin $R_1$ für Wasserstoff, Methyl, Allyl, Benzyl oder Acetyl, $R_2$ für Wasserstoff, $C_1$-$C_{12}$-Alkyl, $C_6$-$C_9$-Cycloalkyl oder $C_6$-$C_9$-Aryl, $R_3$ für $C_6$-$C_{12}$-Aryl oder eine Gruppe der Formel (II), n für 1, 2 oder 3 und, wenn n 1 ist, $R_4$ für Wasserstoff oder eine der Gruppen
-$COR_5$, -$COOR_6$, -CO-$R_7$-$COOR_8$,

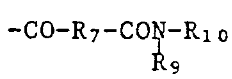,
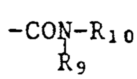,

$-SO_2R_{11}$ ,
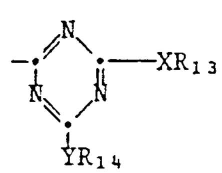

stehen, worin $R_5$ Wasserstoff, $C_1$-$C_{18}$-Alkyl, $C_6$-$C_9$-Cycloalkyl, $C_2$-$C_4$-Alkenyl, $C_6$-$C_8$-Aryl, $C_7$-$C_{16}$-Aralkyl oder durch eine Gruppe der Formel (III), wobei W -O- oder $>$N-$R_{15}$ und $R_{15}$ Wasserstoff, $C_1$-$C_{12}$-Alkyl oder $C_6$-$C_9$-Cycloalkyl darstellen, substituiertes $C_1$-$C_3$-Alkyl, $R_6$ $C_1$-$C_{12}$-Alkyl, $C_6$-$C_{12}$-Cycloalkyl oder eine Gruppe der Formel (II), $R_7$ eine direkte Bindung, $C_1$-$C_8$-Alkylen, $C_6$-$C_8$-Cycloalkylen, Vinylen oder Phenylen, $R_8$ $C_1$-$C_{12}$-Alkyl, $C_6$-$C_9$-Cycloalkyl, Allyl oder eine Gruppe der Formel (II) sowie $R_9$ und $R_{10}$, die gleich oder verschieden sein können, Wasserstoff, $C_1$-$C_{12}$-Alkyl, $C_6$-$C_9$-Cycloalkyl, Allyl oder eine Gruppe der Formel (II) bedeuten oder zusammen mit dem Stickstoffatom, an das sie gebunden sind, 1-Pyrrolidinyl, 1-Piperidyl, 4-Morpholinyl, 1-Hexahydroazepinyl oder 4-Methyl-1-piperazinyl bilden, $R_{11}$ Methyl Ethyl oder $C_6$-$C_9$-Aryl, $R_{13}$ und $R_{14}$, die gleich oder verschieden sein können, Wasserstoff, $C_1$-$C_{12}$-Alkyl, $C_6$-$C_9$-Cycloalkyl, Allyl oder eine Gruppe der Formel (II) sowie X und Y, die gleich oder verschieden sein können, -O- oder $>$N-$R_{16}$, wobei $R_{16}$ Wasserstoff, $C_1$-$C_{12}$-Alkyl, $C_6$-$C_9$-Cycloalkyl, Allyl oder eine Gruppe der Formel (II) darstellt, bedeuten oder die Gruppen $R_{13}$ X- und $R_{14}$Y-, die gleich oder verschieden sein können, eine 1-Pyrrolidinyl-, 1-Piperidyl-, 4-Morpholinyl- oder 1-Hexahydroazepinylgruppe bedeuten, oder, wenn n 2 ist, $R_4$ für eine -CO-Gruppe oder eine der Gruppen
-COO-$R_{17}$-OOC-, -CO-$R_{18}$-CO-,

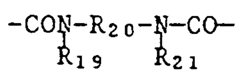 , 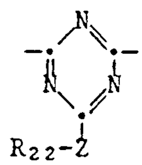

steht, worin $R_{17}$ $C_2$-$C_{10}$-Alkylen, $C_6$-$C_{15}$-Cycloalkylen, 2-Buten-1,4-diyl oder eine Gruppe der Formel (IV), wobei $R_{23}$ für Wasserstoff oder Methyl steht, $R_{18}$ eine direkte Bindung, $C_1$-$C_{12}$-Alkylen, $C_2$-$C_{12}$-Alkyliden oder in der Kette durch eine oder zwei Gruppen $>$N-$R_{24}$, wobei $R_{24}$ für $C_1$-$C_8$-Alkyl, $C_6$-$C_9$-Cycloalkyl oder eine Gruppe der Formel (II) steht, substituiertes $C_2$-$C_8$-Alkylen, $R_{19}$ und $R_{21}$, die gleich oder verschieden sein können, Wasserstoff, $C_1$-$C_8$-Alkyl, $C_6$-$C_9$-Cycloalkyl oder eine Gruppe der Formel (II), $R_{20}$ $C_2$-$C_{10}$-Alkylen, $C_6$-$C_{15}$-Cycloalkylen, $C_6$-$C_{15}$-Arylen oder Xylylen, $R_{22}$ Wasserstoff, $C_1$-$C_{12}$-Alkyl, $C_6$-$C_9$-Cycloalkyl, Allyl oder eine Gruppe der Formel (II) und Z -O- oder $>$N-$R_{16}$ mit $R_{16}$ wie oben definiert bedeuten, oder, wenn n 3 ist, $R_4$ für aliphatisches $C_4$-$C_8$-Triacyl, eine Gruppe $N(CH_2CO\text{-})_3$, einen Triacylrest, welcher sich von 1,2,4-Benzoltricarbonsäure oder 1,3,5-Benzoltricarbonsäure ableitet, oder für eine Gruppe

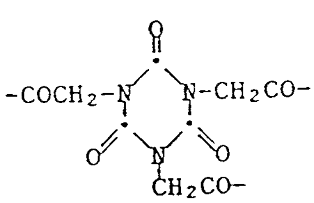 oder 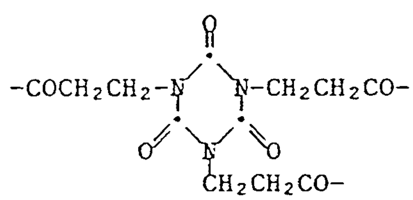

oder eine 1,3,5-Triazin-2,4,6-triylgruppe steht.

Solche Verbindungen der Formel (I) sind besonders bevorzugt, worin $R_1$ für Wasserstoff oder Methyl, $R_2$ für Wasserstoff oder Methyl, $R_3$ für 2,2,6,6-Tetramethyl-4-piperidyl oder 1,2,2,6,6-Pentamethyl-4-piperidyl, n für 1, 2 oder 3 und, wenn n 1 ist, $R_4$ für Wasserstoff oder eine der Gruppen
-$COR_5$, -$COOR_6$, -$COCOOR_8$,

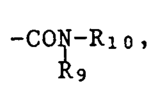 , 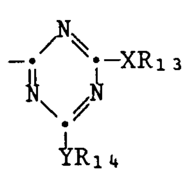

stehen, worin $R_5$ $C_1$-$C_{17}$-Alkyl, Cyclohexyl oder Phenyl, $R_6$ $C_2$-$C_{18}$-Alkyl, $C_6$-$C_{10}$-Cycloalkyl oder eine Gruppe der Formel (II), wobei $R_1$ für Wasserstoff oder Methyl steht, $R_8$ $C_2$-$C_{12}$-Alkyl, Cyclohexyl oder eine

Gruppe der Formel (II), wobei $R_1$ für Wasserstoff oder Methyl steht, $R_9$ Wasserstoff, $R_{10}$ $C_4$-$C_{12}$-Alkyl oder Cyclohexyl, $R_{13}$ und $R_{14}$, die gleich oder verschieden sein können, $C_1$-$C_{12}$-Alkyl, Cyclohexyl, Allyl oder eine Gruppe der Formel (II), wobei $R_1$ für Wasserstoff oder Methyl steht, sowie X und Y, die gleich oder verschieden sein können, eine Gruppe $>N-R_{16}$, wobei $R_{16}$ Wasserstoff, $C_1$-$C_{12}$-Alkyl, Cyclohexyl, Allyl oder eine Gruppe der Formel (II) darstellt, wobei $R_1$ für Wasserstoff oder Methyl steht, bedeuten und, wenn n 2 ist, $R_4$ für eine der Gruppen
$-COO-R_{17}-OOC-$, $-CO-R_{18}-CO-$,

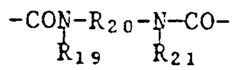 , 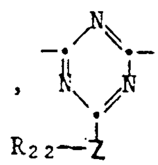

steht, worin $R_{17}$ $C_4$-$C_{10}$-Alkylen, $C_6$-$C_8$-Cycloalkylen oder eine Gruppe der Formel (IV), wobei $R_{23}$ für Wasserstoff steht, $R_{18}$ eine direkte Bindung, $C_1$-$C_8$-Alkylen, $C_2$-$C_8$-Alkyliden, Cyclohexylen oder Phenylen, $R_{19}$ und $R_{21}$ Wasserstoff, $R_{20}$ $C_6$-$C_9$-Alkylen oder $C_6$-$C_{13}$-Arylen, $R_{22}$ $C_1$-$C_{12}$-Alkyl, Cyclohexyl, Allyl, oder eine Gruppe der Formel (II), wobei $R_1$ für Wasserstoff oder Methyl steht, und Z -O- oder $>N-R_{16}$ mit $R_{16}$ wie oben definiert bedeuten und, wenn n 3 ist, $R_4$ für eine 1,3,5-Triazin-2,4,6-triylgruppe steht.

$R_1$ ist vorzugsweise Wasserstoff, $C_1$-$C_4$-Alkyl, Allyl, Benzyl oder Acetyl, insbesondere Wasserstoff oder Methyl.

Verbindungen der Formel (I), worin $R_2$ für Wasserstoff, $C_1$-$C_4$-Alkyl, Cyclohexyl, Phenyl oder Benzyl und $R_3$ für Phenyl, 4-Ethoxycarbonylphenyl oder eine Gruppe der Formel (II) stehen, sind bevorzugt.

Verbindungen der Formel (I), worin $R_1$ für Wasserstoff oder Methyl, $R_2$ für Wasserstoff oder $C_1$-$C_4$-Alkyl und $R_3$ für 4-Ethoxycarbonylphenyl oder eine Gruppe der Formel (II) stehen, sind besonders bevorzugt.

Solche Verbindungen der Formel (I) sind von Interesse, worin n 1, 2 oder 3 ist, und, wenn n = 1, $R_4$ für Wasserstoff, CN, $-COR_5$, $-COOR_6$, $-CO-R_7-COOR_8$,

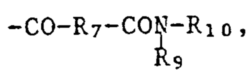, 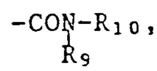, $-SO_2R_{11}$ oder 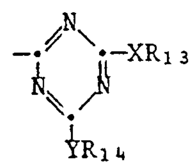

steht, worin $R_5$ Wasserstoff, $C_1$-$C_{18}$-Alkyl, Cyclopentyl, Cyclohexyl, $C_2$-$C_{18}$-Alkenyl, Phenyl, $C_7$-$C_{10}$-Phenylalkyl, durch $C_1$-$C_{18}$-Alkoxy, $C_2$-$C_{18}$-Dialkylamino oder eine Gruppe der Formel (III)

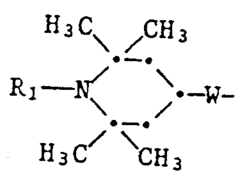 (III)

substituiertes $C_1$-$C_{10}$-Alkyl, wobei $R_1$ für Wasserstoff, $O^\bullet$, CN, NO, Cyanmethyl, $C_1$-$C_{12}$-Alkyl, $C_3$-$C_{12}$-Alkenyl oder $C_3$-$C_{12}$-Alkinyl, unter der Voraussetzung, dass das an das Stickstoffatom gebundene Kohlenstoffatom primär ist, $C_7$-$C_{12}$-Aralkyl, $C_1$-$C_{12}$-Acyl, 2,3-Epoxypropyl, OH-monosubstituiertes $C_2$-$C_6$-Alkyl oder 2,3-Dihydroxypropyl und W für -O- oder $>N-R_{15}$ stehen, wobei $R_{15}$ Wasserstoff, $C_1$-$C_{18}$-Alkyl, Cyclopentyl, Cyclohexyl, $C_3$-$C_{18}$-Alkenyl, Phenyl, $C_7$-$C_{10}$-Phenylalkyl oder eine Gruppe der Formel (II) darstellt, $R_6$ $C_1$-$C_{18}$-Alkyl, durch OH, $C_1$-$C_{18}$-Alkoxy oder $C_2$-$C_{18}$-Dialkylamino substituiertes $C_2$-$C_6$-Alkyl, Cyclopentyl, Cyclohexyl, $C_3$-$C_{18}$-Alkenyl, Phenyl, $C_7$-$C_{10}$-Phenylalkyl oder eine Gruppe der Formel (II) und $R_7$ eine direkte Bindung, $C_1$-$C_{18}$-Alkylen, $C_2$-$C_{20}$-Alkyliden, $C_7$-$C_{20}$-Phenylalkyliden, Cyclohexylen, $C_2$-$C_{18}$-Alkenylen oder Phenylen bedeuten, $R_8$ die für $R_6$ angegebene Bedeutung hat oder Wasserstoff oder Alkalimetall bedeutet, $R_9$ und $R_{10}$, die gleich oder verschieden sind, die für $R_{15}$ angegebene Bedeutung haben oder $R_9$ und $R_{10}$ zusammen mit dem Stickstoffatom, an das sie gebunden sind, eine Pyrrolidin-, Piperidin-,

Morpholin- oder Hexahydroazepingruppe bilden, $R_{11}$ $C_1$-$C_{12}$-Alkyl oder Phenyl bedeutet, $R_{13}$ und $R_{14}$, die gleich oder verschieden sind, die für $R_6$ angegebene Bedeutung haben oder Wasserstoff darstellen sowie X und Y, die gleich oder verschieden sind, eine direkte Bindung, -O- oder >N-$R_{16}$ bedeuten, wobei $R_{16}$ die für $R_{15}$ angegebene Bedeutung hat oder OH-monosubstituiertes $C_2$-$C_6$-Alkyl darstellt, oder die Gruppen $R_{13}$X- und $R_{14}$Y-, die gleich oder verschieden sind, eine 1-Pyrrolidinyl-, 1-Piperidyl-, 4-Morpholinyl-, 1-Hexahydroazepinyl- oder 4-Methyl-1-piperazinylgruppe bedeuten, oder, wenn n = 2, $R_4$ für -CO-, $-SO_2-$, -COO-$R_{17}$-OOC-, -CO-$R_{18}$-CO-,

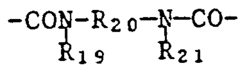 , 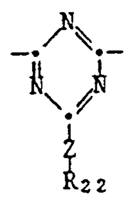 oder 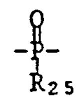

steht, worin $R_{17}$ $C_2$-$C_{18}$-Alkylen, in der Kette durch 1 oder 2 Sauerstoffatome substituiertes $C_4$-$C_{12}$-Alkylen, Cyclohexylen, $C_4$-$C_8$-Alkenylen, Phenylen, Xylylen oder eine Gruppe der Formel (IV)

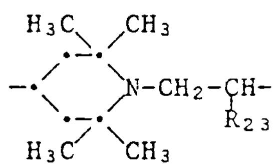 (IV)

worin $R_{23}$ für Wasserstoff $C_1$-$C_4$-Alkyl oder Phenyl steht, und $R_{18}$ eine direkte Bindung, $C_1$-$C_{18}$-Alkylen, $C_2$-$C_{20}$-Alkyliden, $C_7$-$C_{20}$-Phenylalkyliden, Cyclohexylen, Phenylen oder in der Kette durch 1 oder 2 Sauerstoffatome oder 1 oder 2 Gruppen >N-$R_{24}$,wobei $R_{24}$ die für $R_{15}$ angegebene Bedeutung hat, substituiertes $C_2$-$C_{12}$-Alkylen bedeuten, $R_{19}$ und $R_{21}$, die gleich oder verschieden sind, die für $R_{15}$ angegebene Bedeutung haben, $R_{20}$ $C_2$-$C_{18}$-Alkylen, Cyclohexylen, Phenylen oder Xylylen bedeutet, $R_{22}$ die für $R_{13}$ und $R_{14}$ sowie Z die für X und Y angegebenen Bedeutungen haben oder -Z$R_{22}$ 1-Pyrrolidinyl, 1-Piperidyl, 4-Morpholinyl, 1-Hexahydroazepinyl, 4-Methyl-1-piperazinyl oder Halogen und $R_{25}$ $C_1$-$C_4$-Alkyl bedeuten oder, wenn n = 3, $R_4$ fur $C_4$-$C_{18}$-Alkantrioyl, eine Gruppe

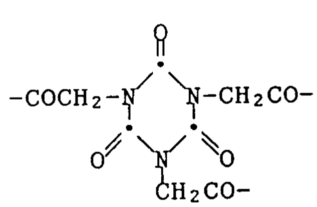 , 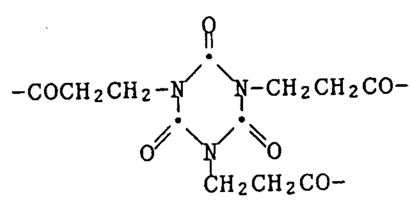 ,

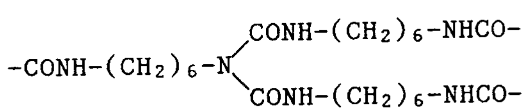 , 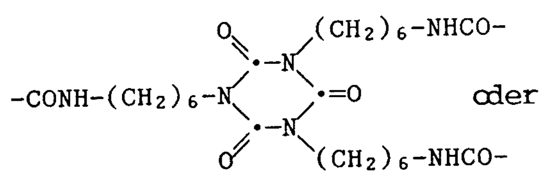 oder

$N(CH_2CO-)_3$ , Benzoltricarbonyl, Benzoltricarbamoyl, eine 1,3,5-Triazin-2,4,6-triylgruppe, eine ≡ P-Gruppe, eine ≡ PO-Gruppe oder eine Gruppe $(-CO-R_7-COO)_3Al$ mit $R_7$ wie oben definiert steht.

Vorzugsweise ist n 1, 2 oder 3, insbesondere 1 oder 2.

Verbindungen der Formel (I), worin $R_3$ für eine Gruppe der Formel (II) steht, sind bevorzugt.

Wenn n 1 ist, steht $R_4$ insbesondere für $-COR_5$, $-COOR_6$ oder

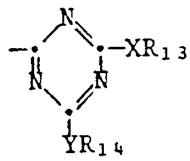 ,

und wenn n 2 ist, steht $R_4$ vorzugsweise für -COO-$R_{17}$-OOC-, -CO-$R_{18}$-CO- oder

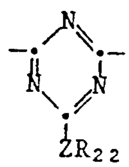

Solche Verbindungen der Formel (I) werden bevorzugt, worin n 1, 2 oder 3 ist, und, wenn n = 1, $R_4$ Wasserstoff,
-$COR_5$, -$COOR_6$, -CO-$R_7$-$COOR_8$,

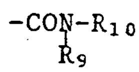 oder 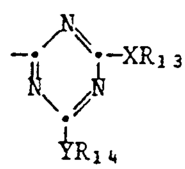 ist,

worin $R_5$ für $C_1$-$C_{18}$-Alkyl, $R_6$ für $C_1$-$C_{18}$-Alkyl, durch $C_1$-$C_4$-Alkoxy substituiertes $C_2$-$C_6$-Alkyl, Cyclohexyl, Methylcyclohexyl, Dimethylcyclohexyl, Trimethylcyclohexyl, t-Butylcyclohexyl oder eine Gruppe der Formel (II), $R_7$ für $C_1$-$C_{10}$-Alkylen, $R_8$ für Wasserstoff, $R_9$ und $R_{10}$ unabhängig voneinander für Wasserstoff, $C_1$-$C_4$-Alkyl oder Cyclohexyl, $R_{13}$ und $R_{14}$ unabhängig voneinander für Wasserstoff, $C_1$-$C_8$-Alkyl, Allyl oder eine Gruppe der Formel (II) sowie X und Y unabhängig voneinander für -O- oder $>$N-$R_{16}$, wobei $R_{16}$ Wasserstoff, $C_1$-$C_8$-Alkyl, OH-monosubstituiertes $C_2$-$C_4$-Alkyl, Allyl oder eine Gruppe der Formel (II) ist, stehen, und wenn n = 2 , $R_4$ -COO-$R_{17}$-OOC-, -CO-$R_{18}$-CO-,

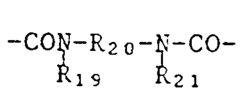 , 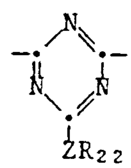 oder 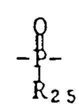 ist,

worin $R_{17}$ für $C_2$-$C_{12}$-Alkylen oder in der Kette durch 1 oder 2 Sauerstoffatome substituiertes $C_4$-$C_{12}$-Alkylen, $R_{18}$ für eine direkte Bindung oder $C_1$-$C_{10}$-Alkylen, $R_{19}$ und $R_{21}$ für Wasserstoff, $R_{20}$ für $C_2$-$C_{10}$-Alkylen oder Cyclohexylen, $R_{22}$ für $C_1$-$C_8$-Alkyl, OH-monosubstituiertes $C_2$-$C_4$-Alkyl oder Allyl und Z für -O- oder $>$N-$R_{16}$ mit $R_{16}$ in der oben angegebenen Bedeutung oder -$ZR_{22}$ für Halogen und $R_{25}$ für $C_1$-$C_4$-Alkyl stehen, und wenn n = 3, $R_4$ für 1,3,5-Triazin-2,4,6-triyl steht.

Solche Verbindungen der Formel (I) sind besonders bevorzugt, worin $R_1$ für Wasserstoff, $R_2$ für Wasserstoff oder Methyl, $R_3$ für 2,2,6,6-Tetramethyl-4-piperidyl, n für 1, 2 oder 3 und, wenn n 1 ist, $R_4$ für -$COR_5$, -$COOR_6$ oder

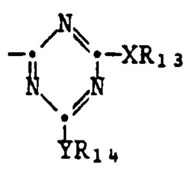

stehen, worin $R_5$ $C_1$-$C_{18}$-Alkyl, $R_6$ $C_1$-$C_{18}$-Alkyl, Cyclohexyl, 4-t-Butylcyclohexyl oder 1,2,2,6,6-Pentamethyl-4-piperidyl, $R_{13}$ und $R_{14}$ unabhängig voneinander $C_1$-$C_8$-Alkyl oder Allyl sowie X und Y unabhängig voneinander -O- oder $>$N-$R_{16}$, wobei $R_{16}$ Wasserstoff, $C_1$-$C_8$-Alkyl, Allyl oder 2,2,6,6-Tetramethyl-4-piperidyl darstellt, bedeuten und, wenn n 2 ist, $R_4$ für -COO-$R_{17}$-OOC-, -CO-$R_{18}$-CO- oder

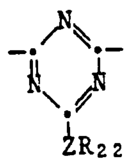

steht, worin $R_{17}$ $C_4$-$C_6$-Alkylen, $R_{18}$ eine direkte Bindung oder $C_1$-$C_8$-Alkylen, $R_{22}$ $C_1$-$C_8$-Alkyl oder Allyl und Z -O- oder $>$N-$R_{16}$ mit $R_{16}$ in der oben angegebenen Bedeutung bedeuten und, wenn n 3 ist, $R_4$ für eine 1,3,5-Triazin-2,4,6,-triylgruppe steht.

Solche Verbindungen der Formel (I) sind von Interesse, worin $R_1$ für Wasserstoff, $R_2$ für Wasserstoff oder Methyl, $R_3$ für 2,2,6,6-Tetramethyl-4-piperidyl, n für 1 oder 2 und, wenn n 1 ist, $R_4$ für -COO$R_6$ oder

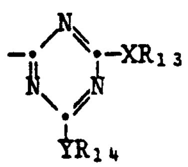

stehen, worin $R_6$ $C_4$-$C_{18}$-Alkyl, Cyclohexyl, 4-t-Butylcyclohexyl oder 1,2,2,6,6-Pentamethyl-4-piperidyl, $R_{13}$ und $R_{14}$ unabhängig voneinander $C_1$-$C_4$-Alkyl sowie X und Y $>$N-$R_{16}$, wobei $R_{16}$ für 2,2,6,6-Tetramethyl-4-piperidyl steht, bedeuten, und, wenn n 2 ist, $R_4$ für -COO-$R_{17}$-OOC- oder

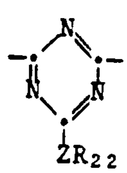

steht, worin $R_{17}$ $C_4$-$C_6$-Alkylen, $R_{22}$ $C_1$-$C_4$-Alkyl oder Allyl und Z -O- oder $>$N-$R_{16}$, wobei $R_{16}$ für Wasserstoff, $C_1$-$C_4$-Alkyl oder Allyl steht, bedeuten.

Besonders bevorzugte Beispiele für Verbindungen der Formel (I) sind:

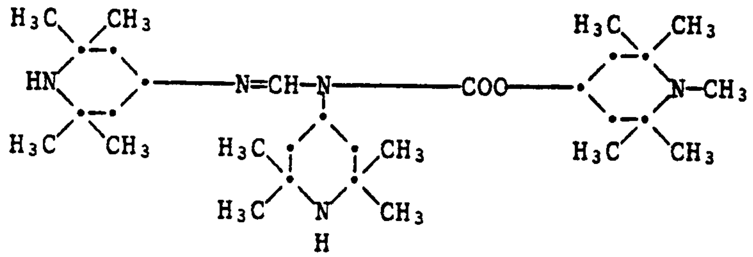

,

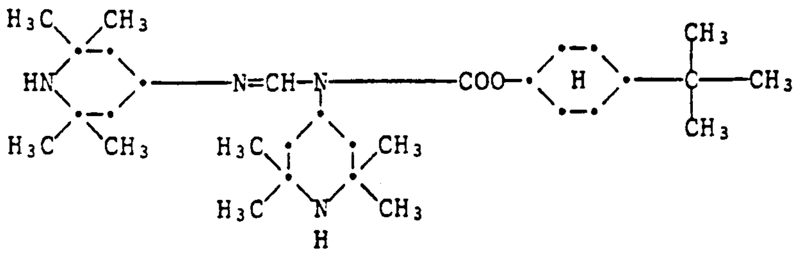

,

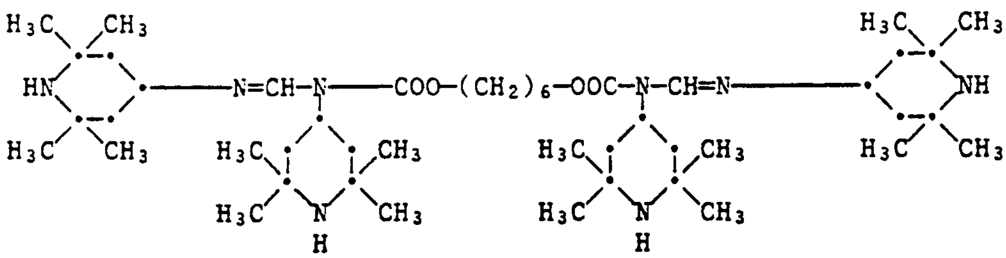

,

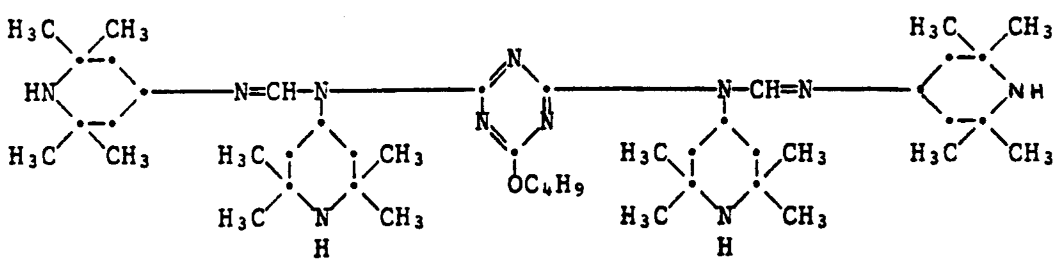

,

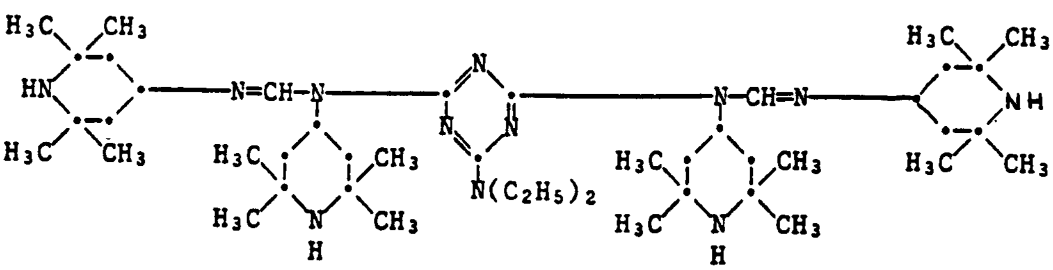

.

Die Verbindungen der Formel (I) sind auf zwei verschiedenen Wegen erhältlich, je nach der Art der Gruppe $R_3$, beispielsweise indem man zunächst die Verbindungen mit n = 1 und $R_4$ = H herstellt, aus denen die Verbindungen mit n = 1, 2, 3 oder 4 und $R_4 \neq$ H durch Umsetzung mit geeigneten Acylierungsmitteln erhältlich sind.

Ist $R_3$ eine Gruppe der Formel (II), so kann man die entsprechenden Verbindungen der Formel (I) beispielsweise gemäss Reaktionsgleichung 1 durch Umsetzung von 2 Mol eines Piperidylamins (V) mit 1 Mol eines Orthoesters (VI), worin $R'$ für $C_1$-$C_4$-Alkyl steht, herstellen, wobei sich unter Abspaltung des Alkohols $R'OH$ das Amidin (VII) bildet. Die Verbindungen der Formel (I) mit einer Gruppe der Formel (II) als $R_3$ und $R_4 \neq$ H kann man dann durch Acylierung aus den Verbindungen der Formel (VII) erhalten.

REAKTIONSGLEICHUNG 1

a)

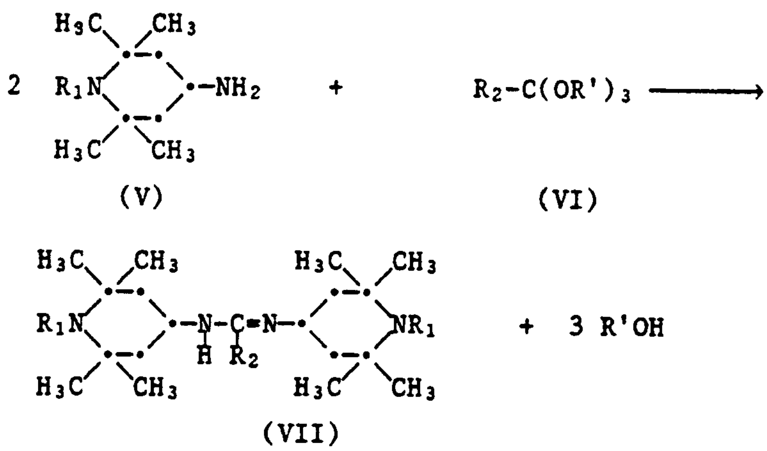

(V) (VI)

(VII)

b) (VII) $\xrightarrow{\text{Acylierungsmittel}}$ Verbindung der Formel (I) mit $R_3$ = Gruppe (II) und $R_4 \neq$ H.

Ist $R_3$ $C_6$-$C_{18}$-Aryl, so kann man die entsprechenden Verbindungen der Formel (I) beispielsweise gemäss Reaktionsgleichung 2 durch Umsetzung eines Piperidylamins (V) mit einem Alkylimidat (VIII), worin R′ die oben angegebene Bedeutung hat, herstellen, wobei sich unter Abspaltung des Alkohols R′OH das Amidin (IX) bildet.

Die Verbindungen der Formel (I) mit $R_3$ = $C_6$-$C_{18}$-Aryl und $R_4 \neq$ H lassen sich dann durch Acylierung aus den Verbindungen der Formel (IX) herstellen.

REAKTIONSGLEICHUNG 2

a)

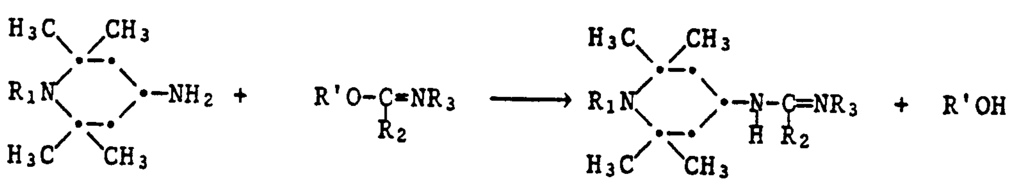

(V) (VIII) (IX)

b) (IX) $\xrightarrow{\text{Acylierungsmittel}}$ Verbindungen der Formel (I) mit $R_3$ = $C_6$-$C_{18}$-Aryl und $R_4 \neq$ H.

Die Reaktionen a) in den Gleichungen 1 und 2 lassen sich zum Beispiel mit oder ohne inertes Lösungsmittel durchführen, wobei man bei einer Temperatur von 80° bis 250°C, vorzugsweise 100° bis 180°C, unter Entfernung des während der Reaktion freigesetzten Alkohols arbeitet.

Das Verhältnis der Reaktionspartner ist nicht kritisch, und man kann einen Überschuss des einen oder anderen Reaktionspartners einsetzen.

Die nachfolgenden Acylierungsreaktionen b) in den Gleichungen 1) und 2) können vorzugsweise in einem inerten Lösungsmittel bei einer Temperatur von -20° bis 200°C, vorzugsweise 0° bis 180°C,

erfolgen; die Reaktionsbedingungen sind von der Art des verwendeten Acylierungsmittels abhängig.

Wird ein Acylhalogenid als Acylierungsmittel eingesetzt, so wird die während der Reaktion freigesetzte Halogenwasserstoffsäure vorzugsweise mit einer anorganischen Base, wie z.B. Natrium- oder Kaliumhydroxyd oder - carbonat, in einer der während der Reaktion freigesetzten Säure zumindest äquivalenten Menge neutralisiert. Die Reaktionen gemäss Gleichungen 1) und 2) können in einem einzigen Reaktor ohne Isolierung des Amidins (VII) bzw. (IX) durchgeführt werden, doch ist es ebenfalls möglich, diese Verbindungen abzutrennen und nach Reinigung in den nachfolgenden Acylierungsreaktionen einzusetzen.

Zur näheren Erläuterung der vorliegenden Erfindung sind unten einige Herstellungsbeispiele für Verbindungen der Formel (I) beschrieben; dabei dienen diese Beispiele lediglich der Erläuterung und sind in keiner Weise als Einschränkung auszulegen.

Beispiel 1: 93,76 g (0,6 Mol) 2,2,6,6-Tetramethyl-4-piperidylaminund 88,92 g (0,6 Mol) Triethylorthoformiat werden unter Entfernung des während der Reaktion freigesetzten Ethanols auf 140-150 °C bis zum Aufhören der Ethanolabspaltung erhitzt. Nach dem Abkühlen wird der gebildete Niederschlag durch Filtrieren abgetrennt, mit n-Hexan gewaschen und getrocknet. Dabei erhält man die Verbindung der Formel

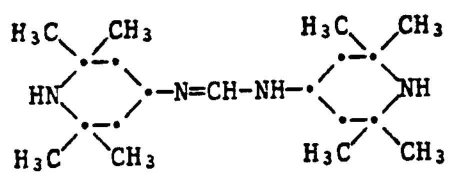

vom Schmelzpunkt 175-197 °C.

| Analyse für $C_{19}H_{38}N_4$ | | | |
|---|---|---|---|
| Berechnet: | C 70,75 %; | H 11,87 %; | N 17,37% |
| Gefunden: | C 70,78 %; | H 11,96 %; | N 17,32 % |

Beispiele 2 und 3: Man verfährt wie im Beispiel 1 unter Verwendung der entsprechenden Reagenzien zur Herstellung der folgenden Verbindungen der allgemeinen Formel

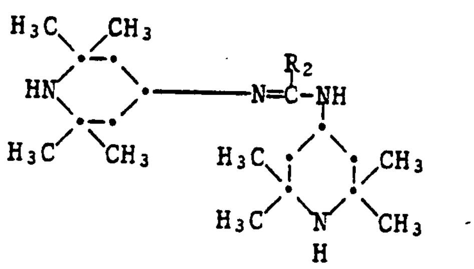

| Beispiel | $R_2$ | Schmelzpunkt (°C) |
|---|---|---|
| 2 | $CH_3$- | 93-94 |
| 3 | $C_2H_5$- | 45-46 |

Beispiel 4: Eine auf 0° bis +5 °C gekühlte Suspension von 32,25 g (0,1 Mol) N,N'-Bis-(2,2,6,6-tetramethyl-4-piperidyl)-formamidin (gemäss Beispiel 1 hergestellt) in 150 ml Dichlormethan versetzt man langsam mit einer Lösung von 11,39 g (0,105 Mol) Chlorameisensäureethylester in 30 ml Dichlormethan,

wobei man die Temperatur unter 10 ° C hält. Nach beendeter Zugabe rührt man zwei Stunden lang, lässt die Temperatur auf 20 ° C ansteigen, kühlt dann wieder auf etwa 5 ° C ab und versetzt langsam mit einer Lösung von 4,4 g (0,11 Mol) Natriumhydroxyd in 30 ml Wasser, wobei man die Temperatur unter 10 ° C hält. Nach beendeter Zugabe wird zwei Stunden lang weitergerührt, während man die Temperatur auf 20 ° C ansteigen lässt.

Die organische Phase wird abgetrennt, mit Wasser gewaschen, über $Na_2SO_4$ getrocknet und bis zur Trockne eingedampft.

Der dabei erhaltene Rückstand wird aus Essigester umkristallisiert.

Dabei erhält man die Verbindung der Formel

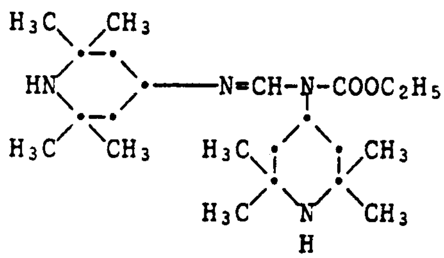

vom Schmelzpunkt 123-124 ° C.

| Analyse für $C_{22}H_{42}N_4O_2$ | | | |
|---|---|---|---|
| Berechnet: | C 66,96 %; | H 10,73 %; | N 14,20 % |
| Gefunden: | C 66,65 %; | H 10,71 %; | N 14,06 % |

Beispiele 5-28: Man verfährt wie im Beispiel 4 unter Verwendung der entsprechenden Reagenzien zur Herstellung der folgenden Verbindungen der allgemeinen Formel

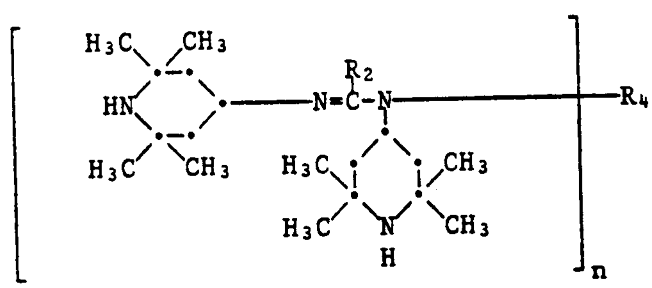

| Bsp. | n | $R_2$ | $R_4$ | Schmelzpunkt °C |
|---|---|---|---|---|
| 5 | 1 | H- | $-COOC_4H_9$ | 89-90 |
| 6 | 1 | H- | 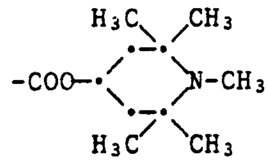 | 172-173 |
| 7 | 1 | H- | $-COC_7H_{15}$ | Öl |
| 8 | 2 | H- | $-COO-(CH_2)_4-OOC-$ | 172-173 |
| 9 | 2 | H- | $-CO-(CH_2)_8-CO-$ | 148-149 |
| 10 | 1 | $CH_3-$ | $-COOC_2H_5$ | Öl |
| 11 | 1 | $C_2H_5-$ | $-COOC_2H_5$ | Öl |
| 12 | 1 | $CH_3-$ | $-COOC_4H_9$ | 92-94 |
| 13 | 1 | H | 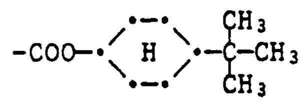 | 157-158 |
| 14 | 2 | H | $-COO-(CH_2)_6-OOC-$ | 165-167 |
| 15 | 2 | H | $-COCO-$ | 262-263 |
| 16 | 2 | H | $-CO-(CH_2)_4-CO-$ | 155-156 |
| 17 | 1 | H | $-COC_{17}H_{35}$ | Wachs |
| 18 | 2 | H | $-CO(CH_2)_2CO-$ | 238-240 |
| 19 | 1 | H | 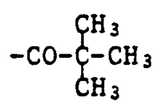 | 125-126 |
| 20 | 1 | H | $-COCH_3$ | 127-129 |
| 21 | 1 | H | 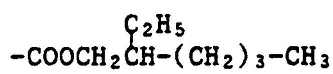 | 55-56 |
| 22 | 1 | H | $-COOCH_2CH_2OCH_3$ | 94-95 |
| 23 | 2 | H | $-COO(CH_2)_2O(CH_2)_2OOC-$ | 140-141 |
| 24 | 2 | H | 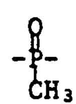 | 162-163 |
| 25 | 1 | H | $-COOC_{18}H_{37}$ | 69-70 |
| 26 | 1 | H | $-COOC_{14}H_{29}$ | 55-56 |
| 27 | 1 | H | $-COC_{11}H_{23}$ | 60-62 |
| 28 | 1 | H | $-CO(CH_2)_2COOH$ | 185-186 |

Beispiel 29: Man gibt 64,5 g (0,2 Mol) N,N'-Bis-(2,2,6,6-tetramethyl-4-piperidyl)-formamidin langsam zu einer auf 10°C gekühlten Lösung von 18,45 g (0,1 Mol) Cyanurchlorid in 500 ml Xylol, wobei man die Temperatur zwischen 10° und 15°C hält.

Nach beendeter Zugabe erhitzt man 2 Stunden lang auf 50°-55°C, gibt 53 g (0,5 Mol) wasserfreies gemahlenes $Na_2CO_3$ dazu, erhitzt 3 Stunden lang auf 70°C und filtriert dann heiss ab.

Ein weisser Feststoff fällt aus der filtrierten Lösung aus und wird durch Filtrieren abgetrennt und

getrocknet.

Dabei erhält man die Verbindung der Formel

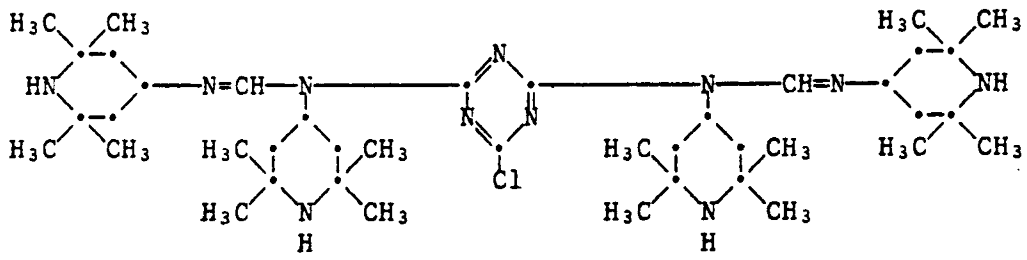

vom Schmelzpunkt 206-207 ° C.

| Analyse für $C_{41}H_{74}ClN_{11}$ | |
|---|---|
| Berechnet: | Cl = 4,69 % |
| Gefunden: | Cl = 4,71 % |

Beispiel 30: Man gibt 158 g (0,49 Mol) N,N′-Bis-(2,2,6,6-tetramethyl-4-piperidyl)-formamidin langsam zu einer Lösung von 27,7 g (0,15 Mol) Cyanurchlorid in 300 ml Trimethylbenzol, wobei man die Temperatur auf 25-30 ° C hält.

Dann erhitzt man eine Stunde lang auf 100-110 ° C, gibt 18,9 g gepulvertes Natriumhydroxyd dazu und erhitzt weitere 12 Stunden lang am Rückfluss unter azeotropischer Entfernung des Reaktionswassers.

Man verdünnt mit 100 ml Trimethylbenzol, kühlt ab und filtriert.

Der nach dem Trocknen erhaltene Rückstand wird mit Wasser und dann mit Methylethylketon gewaschen und wiederum getrocknet.

Dabei erhält man die Verbindung der Formel

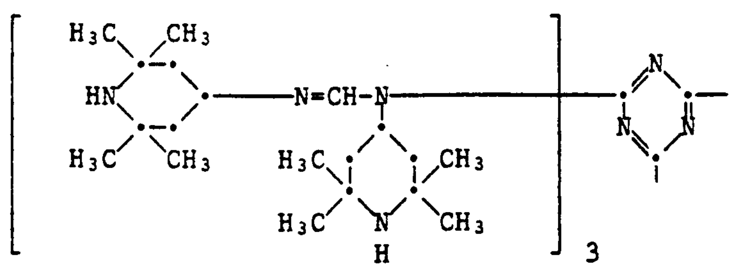

vom Schmelzpunkt 295-296 ° C.

| Analyse für $C_{60}H_{111}N_{15}$ | | | |
|---|---|---|---|
| Berechnet: | C 69,12 %; | H 10,73 %; | N 20,15 % |
| Gefunden: | C 68,77 %; | H 10,72 %; | N 20,01 % |

Beispiele 31-43: Man verfährt wie in Beispiel 30 unter Verwendung der entsprechenden Reagenzien zur Herstellung der folgenden Verbindungen der allgemeinen Formel

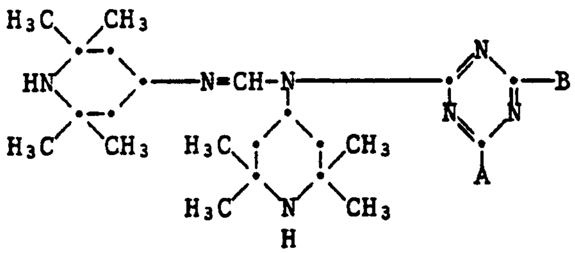

| Beispiel | A | B | Schmelzpunkt °C |
|---|---|---|---|
| 31 | 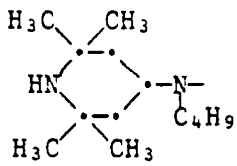 | 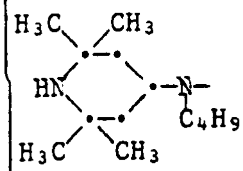 | 224-225 |
| 32 | 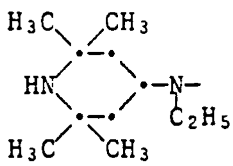 | 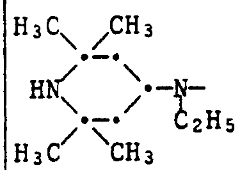 | 176-177 |
| 33 | 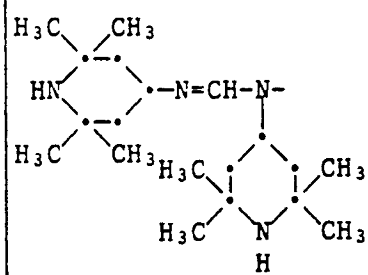 | $C_4H_9O-$ | 236-237 |
| 34 | 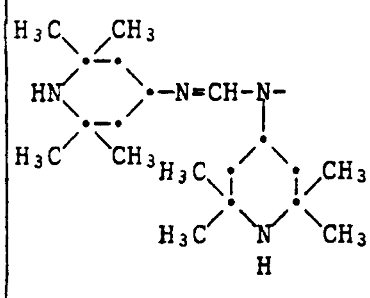 | $(C_2H_5)_2-N-$ | 232-234 |
| 35 | 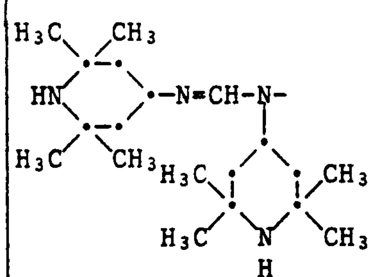 | $-NHCH_2CH=CH_2$ | 246-248 |
| 36 | $-N(CH_2CH=CH_2)_2$ | $-N(CH_2CH=CH_2)_2$ | 198-200 |
| 37 | $-NHCH_2CH=CH_2$ | $-NHCH_2CH=CH_2$ | 159-160 |

| Beispiel | A | B | Schmelzpunkt °C |
|---|---|---|---|
| 38 | 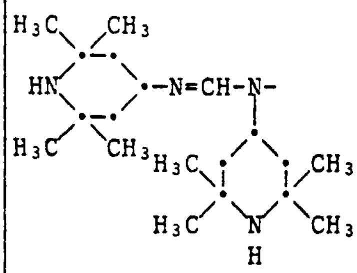 | $-OC_3H_7iso$ | 226-227 |
| 39 | 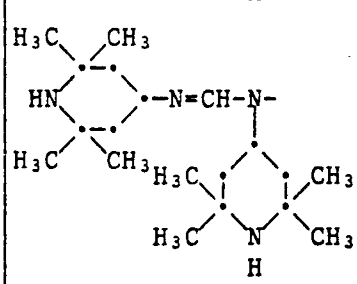 | $-N(CH_3)_2$ | 205-206 |
| 40 | 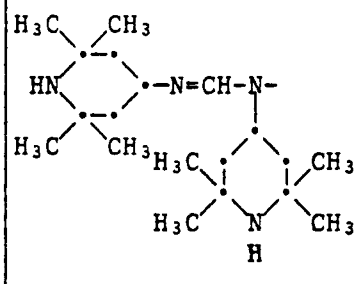 | $-N(C_4H_9)_2$ | 199-200 |
| 41 | 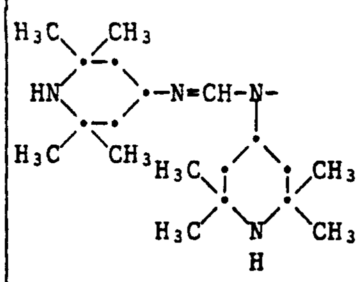 | $-N(CH_2CH{=}CH_2)_2$ | 215-216 |
| 42 | 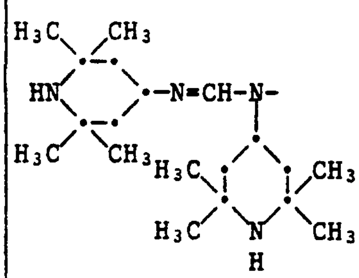 | $-N(CH_2CH_2OH)_2$ | 210-212 |
| 43 | $-N(C_2H_5)_2$ | $-N(C_2H_5)_2$ | 121-122 |

Beispiel 44: Man erhitzt 165,2 g (1 Mol) 4-Aminobenzoesäureethylester und 296,4 g (2 Mol) Orthoameisensäuretriethylester 2 Stunden lang unter Abtrennung des während der Reaktion freigesetzten Ethanols auf 110 °C. Der überschüssige Orthoameisensäuretriethylester wird durch Erhitzen im Vakuum (24 mbar) auf 110 °C entfernt. Man kühlt ab, verdünnt mit 600 ml n-Hexan, erhitzt 30 Minuten lang am Rückfluss und lässt dann abkühlen, und der dabei abgeschiedene Feststoff wird abfiltriert, mit 300 ml Hexan gewaschen und

getrocknet.

55,3 g (0,25 Mol) so erhaltenes Ethyl-(4-ethoxycarbonylphenyl)-formimidat und 39 g (0,25 Mol) 2,2,6,6-Tetramethyl-4-piperidylamin werden 6 Stunden lang auf 100 °C erhitzt. Nach dem Abkühlen versetzt man mit 200 ml Aceton und filtriert.

Das so erhaltene Produkt wird mit Aceton gewaschen und getrocknet.

Dabei erhält man eine Verbindung der Formel

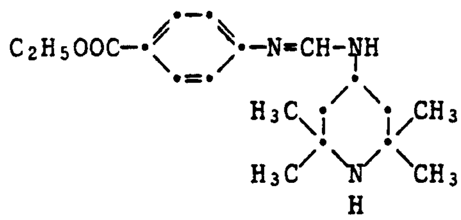

vom Schmelzpunkt 110-111 °C.

| Analyse für $C_{19}H_{29}N_3O_2$ | | | |
|---|---|---|---|
| Berechnet: | C 68,85 %; | H 8,82 %; | N 12,68 % |
| Gefunden: | C 68,89 %; | H 8,90 %; | N 12,80 % |

Beispiel 45: Man gibt eine Lösung von 8,58 g (0,051 Mol) Hexamethylendiisocyanat in 20 ml Toluol langsam zu 33,15 g (0,1 Mol) in 100 ml Toluol gelöstem N-(2,2,6,6-Tetramethyl-4-piperidyl)-N′-(4-ethoxycarbonylphenyl)-formamidin (gemäss Beispiel 44 hergestellt). Dann erhitzt man 4 Stunden lang auf 80 °C und dampft bei vermindertem Druck (26,7 mbar) bis zur Trockne ein. Der so erhaltene Feststoff wird mit Aceton umkristallisiert.

Dabei erhält man die Verbindung der Formel

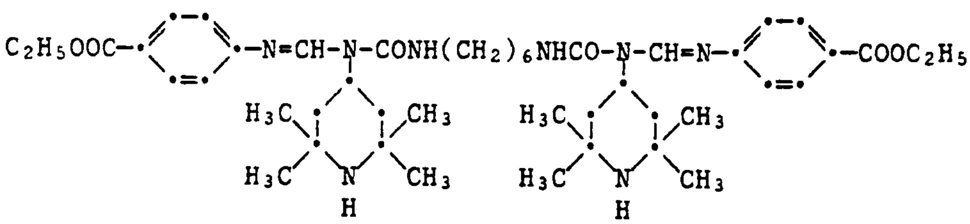

vom Schmelzpunkt 151-152 °C.

| Analyse für $C_{46}H_{70}N_8O_6$ | | | |
|---|---|---|---|
| Berechnet: | C 69,15 %; | H 7,81 %; | N 12,40 % |
| Gefunden: | C 69,01 %; | H 7,86 %; | N 12,28 % |

Beispiele 46-48: Man verfährt wie in Beispiel 45 unter Verwendung der entsprechenden Reagenzien zur Herstellung der folgenden Verbindungen:

| Bsp. | Formel | Schmelzpunkt °C |
| --- | --- | --- |
| 46 | 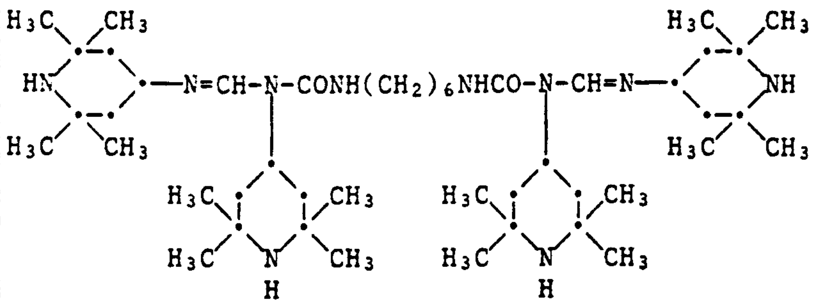 | 172-173 |
| 47 | 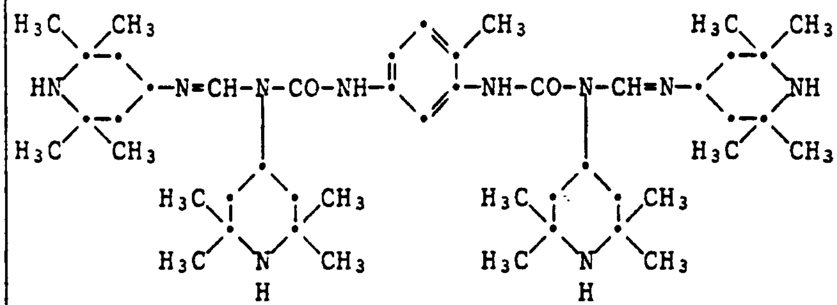 | 199-200 |
| 48 | 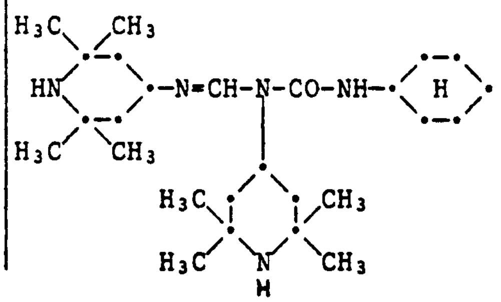 | 168-169 |

Beispiel 49: Eine auf 0° bis 5°C gekühlte Suspension von 32,25 g (0,1 Mol) N,N′-Bis-(2,2,6,6-tetramethyl-4-piperidyl)-formamidin (gemäss Beispiel 1 hergestellt) in 150 ml Dichlormethan versetzt man langsam mit einer Lösung von 14,24 g (0,105 Mol) Diethylcarbamoylchlorid in 30 ml Dichlormethan, wobei man die Temperatur unter 10°C hält.

Nach der Zugabe rührt man und erhitzt 8 Stunden lang am Rückfluss.

Dann kühlt man auf etwa 5°C ab und gibt langsam eine Lösung von 4,4 g (0,11 Mol) Natriumhydroxyd in 30 ml Wasser dazu, wobei man die Temperatur unter 10°C hält.

Nach der Zugabe wird eine Stunde lang weitergerührt, wobei man die Temperatur bis auf 20°C ansteigen lässt.

Die organische Phase wird abgetrennt, mit Wasser gewaschen und über $Na_2SO_4$ getrocknet. Das Lösungsmittel wird abgedampft und der erhaltene Rückstand mit n-Hexan umkristallisiert.

Dabei erhält man die Verbindung der Formel

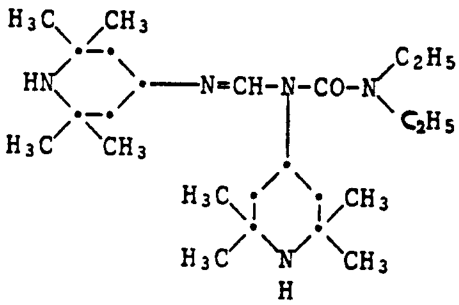

vom Schmelzpunkt 90-91 ° C.

| Analyse für $C_{24}H_{47}N_5O$ | | | |
|---|---|---|---|
| Berechnet:<br>Gefunden: | C 68,36 %;<br>C 68,01 %; | H 11,23 %;<br>H 11,20 %; | N 16,61 %<br>N 16,33 % |

Beispiel 50: Zu einer auf 0° bis 5° C gekühlten Suspension von 80,60 g (0,25 Mol) N,N′-Bis-(2,2,6,6-tetramethyl-4-piperidyl)-formamidin (gemäss Beispiel 1 hergestellt) in 600 ml Dichlormethan gibt man langsam eine Lösung von 43,10 g (0,25 Mol) Diethylchlorphosphat in 100 ml Dichlormethan, wobei man die Temperatur unter 10° C hält. Nach der Zugabe rührt man 2 Stunden lang, wobei man die Temperatur bis auf 20° C ansteigen lässt, und kühlt dann wieder auf etwa 5° C.

Eine Lösung von 10,8 g (0,27 Mol) Natriumhydroxyd in 100 ml Wasser wird langsam zugesetzt, wobei man die Temperatur unter 10° C hält.

Nach der Zugabe wird 2 Stunden lang weitergerührt, wobei man die Temperatur bis auf 20° C ansteigen lässt.

Die organische Phase wird abgetrennt, mit Wasser gewaschen, über $Na_2SO_4$ getrocknet und bis zur Trockne eingedampft.

Der so erhaltene Rückstand wird mit Octan umkristallisiert.

Dabei erhält man die Verbindung der Formel

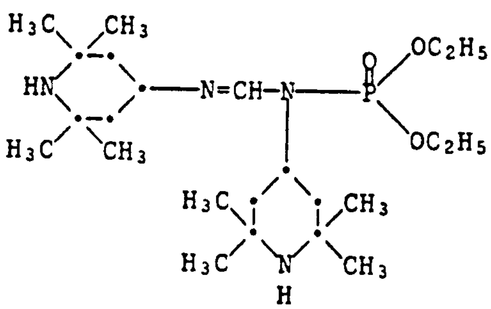

vom Schmelzpunkt 104-106° C.

| Analyse für $C_{23}H_{47}N_4O_3P$-Monohydrat | | | |
|---|---|---|---|
| Berechnet:<br>Gefunden: | C 57,7 %;<br>C 57,37 %; | H 10,4 %;<br>H 10,37 %; | N 11,7 %<br>N 11,6 % |

Beispiel 51: Man verfährt wie in Beispiel 4 unter Verwendung der entsprechenden Reagenzien zur Herstellung der Verbindung der Formel

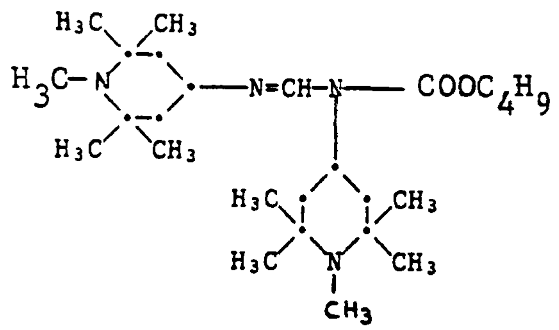

Siedepunkt: 186-188 ° C bei 0,067 mbar.

Wie eingangs erwähnt sind die Verbindungen der Formel (I) bei der Verbesserung der Lichtstabilität, Wärmestabilität und Oxidationsstabilität organischer Materialien, vorzugsweise synthetische Polymere und insbesondere Polyolefine, sehr wirksam.

Organische Materialien, die mit Verbindungen der Formel (I) stabilisiert werden können, sind beispielsweise:

Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

Mischungen der obengenannten Polymere, z.B. Mischungen von Polypropylen mit Polyisobutylen.

Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Vinylacetat-Copolymere, Ethylen-Alkylacrylat-Copolymere, Ethylen-Alkylmethacrylat-Copolymere oder Ethylen-Acrylsäure-Copolymere und deren Salze (Ionomere), sowie Terpolymere von Ethylen mit Propylen und einem Dien, wie Hexadien, Dicyclo-pentadien oder Ethylidennorbornen.

Polystyrol, Poly-(p-methylstyrol).

Copolymere von Styrol oder $\alpha$-Methylstyrol mit Dienen oder Acrylderivaten, wie z.B. Styrol-Butadien, Styrol-Acrylnitril, Styrol-Ethylmethacrylat, Styrol-Butadien-Ethylacrylat, Styrol-Acrylnitril-Methylacrylat; Mischungen von hoher Schlagzähigkeit aus Styrol-Copolymeren und anderen Polymeren, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

Pfropfcopolymere von Styrol, wie z.B. Styrol auf Polybutadien, Styrol und Acrylnitril auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den im vorigen Absatz genannten Copolymeren, wie sie z.B. als sogenannte ABS-, MBS-, ASA- oder AES-Polymere bekannt sind.

Halogenhaltige Polymere, wie z.B. Polychloropren, Chlorkautschuk, chloriertes oder chlorsulfoniertes Polyethylen, Epichlorhydrinhomo- und -copolymere, Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

Polymere, die sich von $\alpha,\beta$-ungesättigten Säuren und deren Derivaten ableiten, wie Polyacrylate und Polymethacrylate, Polyacrylamide und Polyacrylnitril.

Copolymere der im vorigen Absatz genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin.

Homo- und Copolymere von cyclischen Ethern, wie Polyalkylenglykole, Polyethylenoxid, Polypropylenoxid oder deren Copolymere mit Bisglycidylethern.

Polyacetale, wie Polyoxymethylen, sowie solche Polyoxymethylene, die Comonomere, wie z.B. Ethylenoxid, enthalten.

Polyphenylenoxide und -sulfide und deren Mischungen mit Styrolpolymeren.

Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte (Polyisocyanate, Polyole oder Praepolymere).

Polyamide und Copolyamide, die sich von Diaminen und Dicarbonsäuren und/oder Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylenisophthalamid, sowie deren Block-Copolymere mit Polyethern, wie z.B. mit Polyethylenglykol, Polypropylenglykol oder Polytetramethylenglykol.

Polyharnstoffe, Polyimide und Polyamid-imide.

Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-[2,2-(4-hydroxyphenyl)-propan]-terephthalat, Polyhydroxybenzoat, sowie Block-Polyether-ester, die sich von Polyethern mit Hydroxylendgruppen ableiten.

Polycarbonate und Polyestercarbonate.

Polysulfone, Polyethersulfone und Polyetherketone.

Vernetzte Polymere, die sich von Aldehyden einerseits und Phenolen, Harnstoffen oder Melaminen andererseits ableiten, wie Phenol-Formaldehyd-, Harnstoff-Formaldehyd- und Melamin-Formaldehydharze.

Trocknende und nicht-trocknende Alkydharze.

Ungesättigte Polyesterharze, die sich von Copolyestern gesättigter und ungesättigter Dicarbonsäuren mit mehrwertigen Alkoholen sowie Vinylverbindungen als Vernetzungsmittel ableiten, wie auch deren halogenhaltige, schwerbrennbare Modifikationen.

Vernetzbare Acrylharze, die sich von substituierten Acrylsäureestern ableiten, wie z.B. von Epoxyacrylaten, Urethan-acrylaten oder Polyester-acrylaten.

Alkydharze, Polyesterharze und Acrylatharze, die mit Melaminharzen, Harnstoffharzen, Polyisocyanaten oder Epoxidharzen vernetzt sind.

Vernetzte Epoxidharze, die sich von Polyepoxiden ableiten, z.B. von Bis-glycidylethern oder von cycloaliphatischen Diepoxiden.

Natürliche Polymere, wie Cellulose, Naturkautschuk, Gelatine, sowie deren polymerhomolog chemisch abgewandelte Derivate, wie Celluloseacetate, -propionate und -butyrate, bzw. Celluloseether, wie Methylcellulose.

Mischungen (Polyblends) der vorgenannten Polymeren, wie z.B. PP/EPDM, Polyamid 6/EPDM oder ABS, PVC/EVA, PVC/ABS, PVC/MBS, PC/ABS, PBTP/ABS.

Die Verbindungen der Formel (I) sind insbesondere zur Stabilisierung von Polyethylen und Polypropylen verwendbar.

Die Verbindungen der Formel (I) kann man je nach der Art des Polymeren, dem Anwendungszweck und der Gegenwart weiterer Zusatzstoffe in verschiedenen Verhältnissen mit dem organischen Material vermischen.

Im allgemeinen werden die Verbindungen der Formel (I) in Konzentrationen von 0,01 bis 5 Gew.%, bezogen auf das Gewicht des organischen Materials, vorzugsweise 0,05 bis 1 Gew.%, eingesetzt.

Die Verbindungen der Formel (I) lassen sich nach verschiedenen Methoden in die polymeren Materialien einarbeiten, wie durch Vermischen in trockener Pulverform oder nasses Vermischen in Form von Lösungen oder Suspensionen oder als Masterbatch; dabei kann man das Polymere in der Form von Pulver, Granulat, einer Lösung, einer Suspension oder als Latex einsetzen.

Die mit den Produkten der Formel (I) stabilisierten Polymeren sind zur Herstellung von Formkörpern, Folien, Bändern, Fasern, Endlosfäden, Lacken und dergleichen verwendbar.

Falls gewünscht können die erfindungsgemäss stabilisierten Zusammensetzungen auch noch z.B. etwa 0,05 bis 5 Gew.%, vorzugsweise etwa 0,1 bis 2,5 Gew.%, verschiedener üblicher Zusatzstoffe wie Antioxidantien, UV-Absorber, Nickelstabilisatoren, Pigmente, Füllstoffe, Weichmacher, Antistatika, Flammschutzmittel, Gleitmittel, Korrosionsschutzmittel, Metalldesaktivatoren u.a. enthalten.

Beispiele für Zusatzstoffe, die mit den Verbindungen der Formel (I) vermischt werden können, sind insbesondere:

Antioxidantien aus den folgenden Klassen:

Alkylierte Monophenole, z.B. 2,6-Di-t-butyl-4-methylphenol, 2-t-Butyl-4,6-dimethylphenol, 2,6-Di-t-butyl-4-ethylphenol, 2,6-Di-t-butyl-4-n-butylphenol, 2,6-Di-t-butyl-4-i-butylphenol, 2,6-Di-cyclopentyl-4-methylphenol,

2-(α-methylcyclohexyl)-4,6-dimethylphenol, 2,6-Di-octadecyl-4-methylphenol, 2,4,6-Tri-cyclohexylphenol und 2,6-Di-t-butyl-4-methoxymethylphenol.
Alkylierte Hydrochinone, z.B. 2,6-Di-t-butyl-4-methoxyphenol, 2,5-Di-t-butyl-hydrochinon, 2,5-Di-t-amyl-hydrochinon und 2,6-Diphenyl-4-octadecyloxyphenol.
Thiobisphenole, z.B. 2,2′-Thio-bis-(6-t-butyl-4-methylphenol), 2,2′-Thio-bis-(4-octylphenol), 4,4′-Thio-bis-(6-t-butyl-3-methylphenol) und 4,4′-Thio-bis-(6-t-butyl-2-methylphenol).
Alkyliden-Bisphenole, z.B. 2,2′-Methylen-bis-(6-t-butyl-4-methylphenol), 2,2′-Methylen-bis-(6-t-butyl-4-ethylphenol), 2,2′-Methylen-bis-[4-methyl-6-(α-methylcyclohexyl)-phenol], 2,2′-Methylen-bis-(4-methyl-6-cyclohexyl-phenol), 2,2′-Methylen-bis-(6-nonyl-4-methylphenol), 2,2′-Methylen-bis-[6-(α-methylbenzyl)-4-nonylphenol], 2,2′-Methylen-bis-[6-(α,α-dimethylbenzyl)-4-nonylphenol], 2,2′-Methylen-bis-(4,6-di-t-butylphenol), 2,2′-Ethyliden-bis-(4,6-di-t-butylphenol), 2,2′-Ethyliden-bis-(6-t-butyl-4-isobutylphenol), 4,4′-Methylen-bis-(2,6-di-t-butylphenol), 4,4′-Methylen-bis-(6-t-butyl-2-methylphenol), 1,1-Bis-(5-t-butyl-4-hydroxy-2-methylphenyl)-butan, 2,6-Bis-(3-t-butyl-5-methyl-2-hydroxybenzyl)-4-methylphenol, 1,1,3-Tris-(5-t-butyl-4-hydroxy-2-methylphenyl)-butan, 1,1-Bis-(5-t-butyl-4-hydroxy-2-methylphenyl)-3-n-dodecylmercaptobutan, Ethylenglykol-bis-[3,3-bis-(3′-t-butyl-4′-hydroxyphenyl)-butyrat], Bis-(3-t-butyl-4-hydroxy-5-methylphenyl)-dicyclopentadien und Bis-[2-(3′-t-butyl-2′-hydroxy-5′-methylbenzyl)-6-t-butyl-4-methylphenyl]-terephthalat.
Benzylverbindungen, z.B. 1,3,5-Tris-(3,5-di-t-butyl-4-hydroxybenzyl)-2,4,6-trimethylbenzol, Bis-(3,5-di-t-butyl-4-hydroxybenzyl)-sulfid, 3,5-Di-t-butyl-4-hydroxybenzyl-mercaptoessigsäure-isooctylester, Bis-(4-t-butyl-3-hydroxy-2,6-dimethylbenzyl)-dithiolterephthalat, 1,3,5-Tris-(3,5-di-t-butyl-4-hydroxybenzyl)-isocyanurat, 1,3,5-Tris-(4-t-butyl-3-hydroxy-2,6-dimethylbenzyl)-isocyanurat, 3,5-Di-t-butyl-4-hydroxybenzyl-phosphonsäure-dioctadecylester und 3,5-Di-t-butyl-4-hydroxybenzyl-phosphonsäure-monoethylester-calciumsalz.
Acylaminophenole, z.B. 4-Hydroxy-laurinsäureanilid, 4-Hydroxystearinsäureanilid, 2,4-Bis-octylmercapto-6-(3,5-di-t-butyl-4-hydroxyanilino)-s-triazin und N-(3,5-Di-t-butyl-4-hydroxyphenyl)-carbaminsäure-octylester.
Ester der β-(3,5-Di-t-butyl-4-hydroxyphenyl)-propionsäure mit ein- oder mehrwertigen Alkoholen, wie z.B. mit Methanol, Octadecanol, 1,6-Hexandiol, Neopentylglykol, Thiodiethylenglykol, Diethylenglykol, Triethylenglykol, Pentaerythrit, Trishydroxyethyl-isocyanurat und N,N′-Bis-hydroxyethyl-oxamid.
Ester der β-(5-t-Butyl-4-hydroxy-3-methylphenyl)-propionsäure mit ein- oder mehrwertigen Alkoholen, wie z.B. mit Methanol, Octadecanol, 1,6-Hexandiol, Neopentylglykol, Thiodiethylenglycol, Diethylenglykol, Triethylenglykol, Pentaerythrit, Trishydroxyethyl-isocyanurat und N,N′-Bis-hydroxyethyl-oxamid.
Amide der β-(3,5-Di-t-butyl-4-hydroxyphenyl)-propionsäure, wie z.B. N,N′-Bis-(3,5-di-t-butyl-4-hydroxyphenylpropionyl)-hexamethylendiamin, N,N′-Bis-(3,5-di-t-butyl-4-hydroxyphenylpropionyl)-trimethylendiamin und N,N′-Bis-(3,5-di-t-butyl-4-hydroxyphenylpropionyl)-hydrazin.

UV-Absorber und Lichtschutzmittel aus den folgenden Klassen:

2-(2′-Hydroxyphenyl)-benztriazole, wie z.B. das 5′-Methyl-, 3′,5′-Di-t-butyl-, 5′-t-Butyl-, 5′-(1,1,3,3-Tetramethylbutyl)-, 5-Chlor-3′,5′-di-t-butyl-, 5-Chlor-3′-t-butyl-5′-methyl-, 3′-sec.Butyl-5′-t-butyl, 4′-Octoxy-, 3′,5′-Di-t-amyl-und 3′,5′-Bis-(α,α-dimethylbenzyl)-Derivat.
2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2′,4′-Trihydroxy- und 2′-Hydroxy-4,4′-dimethoxy-Derivat.
Ester von verschiedenen substituierten Benzoesäuren, wie z.B. Phenylsalicylat, 4-t-Butylphenylsalicylat, Octylphenylsalicylat, Dibenzoylresorcin, Bis-(4-t-butylbenzoyl)-resorcin, Benzoylresorcin, 3,5-Di-t-butyl-4-hydroxybenzoesäure-2,4-di-t-butylphenylester, 3,5-Di-t-butyl-4-hydroxybenzoesäurehexadecylester und N-Ethyl-N-phenyl-N′-(p-ethoxycarbonylphenyl)-formamidin.
Acrylate, wie z.B. α-Cyan-β,β-diphenylacrylsäureethylester bzw. -isooctylester, α-Carbomethoxy-zimtsäuremethylester, α-Cyan-β-methyl-p-methoxy-zimtsäuremethylester bzw. -butylester, α-Carbomethoxy-p-methoxy-zimtsäuremethylester und N-(β-Carbomethoxy-β-cyanvinyl)-2-methyl-indolin.
Nickelverbindungen, wie z.B. Nickelkomplexe des 2,2′-Thio-bis-[4-(1,1,3,3-tetramethylbutyl)-phenols], wie der 1:1 oder der 1:2 Komplex, gegebenenfalls mit zusätzlichen Liganden, wie n-Butylamin, Triethanolamin oder N-Cyclohexyldiethanolamin, Nickeldibutyldithiocarbamat, Nickelsalze von 4-Hydroxy-3,5-di-t-butylbenzylphosphonsäure-monoalkylestern, wie vom Methyl-, Ethyl- oder Butylester und Nickelkomplexe von Ketoximen, wie von 2-Hydroxy-4-methylphenylundecylketoxim und Nickelkomplexe des 1-Phenyl-4-lauroyl-5-hydroxy-pyrazols, gegebenenfalls mit zusätzlichen Liganden.
Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-t-butyl-4-hydroxybenzyl-malonsäure-bis-(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N′-Bis-(2,2,6,6-tetramethyl-4-piperidyl)-hexamethylendiamin und 4-t-Octylamino-2,6-dichlor-1,3,5-s-triazin, Tris-(2,2,6,6-tetramethylpiperidyl)-nitrilotriacetat, Tetrakis-(2,2,6,6-tetramethylpiperidyl)-1,2,3,4-butan-tetracarbonsäure-ester und 1,1′-(1,2-Ethandiyl)-bis-(3,3,5,5-tetramethylpiperazinon).

Oxalsäurediamide, wie z.B. 4,4′-Di-octyloxy-oxanilid, 2,2′-Dioctyloxy-5,5′-di-t-butyl-oxanilid, 2,2′-Didodecyloxy-5,5′-di-t-butyl-oxanilid, 2-Ethoxy-2′-ethyl-oxanilid, N,N′-Bis-(3-dimethylaminopropyl)-oxalamid, 2-Ethoxy-5-t-butyl-2′-ethyloxanilid und dessen Gemisch mit 2-Ethoxy-2′-ethyl-5,4′-di-t-butyl-oxanilid und Gemische von o- und p-Methoxy- sowie von o- und p-Ethoxy-disubstituierten Oxaniliden.

Metalldesaktivatoren, wie z.B. N,N′-Diphenyloxamid, N-Salicylal-N′-salicyloylhydrazin, N,N′-Bis-salicyloylhydrazin, N,N′-Bis-(3,5-di-t-butyl-4-hydroxyphenylpropionyl)-hydrazin, 3-Salicyloylamino-1,2,4-triazol und Bis-benzyliden-oxalsäuredihydrazid.

Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-t-butylphenyl)-phosphit, Diisodecylpentaerythritdiphosphit, Di-(2,4-di-t-butylphenyl)-pentaerythritdiphosphit, Tristearyl-sorbit-triphosphit und Tetrakis-(2,4-di-t-butylphenyl)-4,4′-Biphenylen-diphosphonit.

Peroxidzerstörende Verbindungen, wie z.B. Ester der β-Thio-dipropionsäure, beispielsweise der Lauryl-, Stearyl-, Myristyl- oder Tridecylester, Mercaptobenzimidazol oder das Zinksalz des 2-Mercaptobenzimidazols, Zink-dibutyl-dithiocarbamat, Dioctadecyldisulfid und Pentaerythrit-tetrakis-(β-dodecylmercapto)-propionat.

Polyamidstabilisatoren, wie z.B. Kupfersalze in Kombination mit Jodiden und/oder Phosphorverbindungen und Salze des zweiwertigen Mangans.

Basische Co-stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinkbrenzcatechinat.

Nukleierungsmittel, wie z.B. 4-t-Butylbenzoesäure, Adipinsäure und Diphenylessigsäure.

Füllstoffe und Verstärkungsmittel, wie z.B. Calciumcarbonat, Silikate, Glasfasern, Asbest, Talk, Kaolin, Glimmer, Bariumsulfat, Metalloxyde und -hydroxyde, Russ und Graphit.

Sonstige Zusätze, wie z.B. Weichmacher, Gleitmittel, Emulgatoren, Pigmente, optische Aufheller, Flammschutzmittel, Antistatika und Treibmittel.

Die Wirksamkeit der erfindungsgemässen Verbindungen als Stabilisatoren wird in den nachfolgenden Beispielen erläutert, bei denen einige in den Herstellungsbeispielen erhaltene Produkte zur Stabilisierung von Polypropylenplatten und Bändern verwendet werden.

Beispiel 52: Je 1 g der in Tabelle 1 angegebenen Verbindungen, 0,5 g Tris-(2,4-di-t-butylphenyl)-phosphit, 0,5 g Pentaerythrit-tetrakis-[3-(3,5-di-t-butyl-4-hydroxyphenyl)-propionat], 1 g Phthalocyaninblau, 1 g Calciumstearat und 1 000 g Polypropylenpulver (Schmelzindex = 2,4 g/10 Min.; gemessen bei 230 °C und 2,16 kg ) werden in einem langsamen Mischer innig vermischt. Die so erhaltenen Gemische werden bei einer Temperatur von 200-220 °C zu Polymergranulat extrudiert, das dann durch Spritzgiessen bei 190-220 °c in 2 mm dicke Platten umgewandelt wird.

Die dabei erhaltenen Plattenwerden in einem Weather-O-Meter Modell 65 WR (ASTM G26-77) bei einer Temperatur der schwarzen Tafel von 63 °C bis zum Beginn oberflächlicher Versprödung (Kreidung) belichtet.

Zum Vergleich wird eine unter den gleichen Bedingungen wie oben, aber ohne Zusatz der erfindungsgemässen Verbindungen hergestellte Polypropylenplatte belichtet. Die Tabelle 1 zeigt die Belichtungszeiten (in Stunden) bis zum Beginn oberflächlicher Versprödung.

Tabelle 1

| Stabilisator | Versprödungszeit (Stunden) |
|---|---|
| Ohne | 500 |
| Verbindung aus Beispiel 4 | 4 300 |
| Verbindung aus Beispiel 5 | 4 800 |
| Verbindung aus Beispiel 6 | 4 000 |
| Verbindung aus Beispiel 8 | 3 600 |
| Verbindung aus Beispiel 13 | 3 800 |
| Verbindung aus Beispiel 21 | 3 750 |
| Verbindung aus Beispiel 49 | 3 900 |

Beispiel 53: Je 1 g der in Tabelle 2 angegebenen Verbindungen, 0,5 g Tris-(2,4-di-t-butylphenyl)-phosphit, 0,5 g Pentaerythrit-tetrakis-[3-(3,5-di-t-butyl-4-hydroxyphenyl)-propionat] und 1 g Calciumstearat werden in einem langsamen Mischer mit 1 000 g Polypropylenpulver (Schmelzindex = 3 g/10 Min.; gemessen bei 230 ° C und 2,16 kg) vermischt.

Die so erhaltenen Gemische werden bei einer Temperatur von 200-220 ° C zu Polymergranulat extrudiert, das dann auf einem Technikumsapparat (Leonard, Sumirago (VA)-Italien) unter den folgenden Arbeitsbedingungen in 50 µm dicke und 2,5 mm breite gereckte Bänder umgewandelt wird:

Extrudertemperatur: 220-240 ° C
Kopftemperatur: 260 ° C
Streckverhältnis: 1:6

Die so hergestellten Bänder werden, an einer weissen Karte befestigt, in einem Weather-O-Meter Modell 65 WR (ASTM G26-77) bei einer Temperatur der schwarzen Tafel von 63 ° C belichtet.

Die verbliebene Reissfestigkeit wird mittels einer Zerreissmaschine mit konstanter Geschwindigkeit an nach verschiedenen Belichtungszeiten gezogenen Proben gemessen; dann wird die zur Halbierung der ursprünglichen Reissfestigkeit erforderliche Belichtungszeit in Stunden ($T_{50}$) berechnet.

Zum Vergleich werden unter den gleichen Bedingungen wie oben, aber ohne Zusatz der erfindungsgemässen Verbindungen hergestellte Bänder belichtet. Die dabei erhaltenden Ergebnisse sind in Tabelle 2 angegeben.

Tabelle 2

| Stabilisator | $T_{50}$ (Stunden) |
|---|---|
| Ohne | 190 |
| Verbindung aus Beispiel 10 | 2 710 |
| Verbindung aus Beispiel 13 | 2 150 |
| Verbindung aus Beispiel 14 | 2 500 |
| Verbindung aus Beispiel 18 | 2 200 |
| Verbindung aus Beispiel 20 | 2 100 |
| Verbindung aus Beispiel 21 | 2 400 |
| Verbindung aus Beispiel 33 | 2 800 |
| Verbindung aus Beispiel 34 | 3 150 |
| Verbindung aus Beispiel 46 | 2 450 |
| Verbindung aus Beispiel 49 | 2 300 |

## Patentansprüche

1. Verbindungen der Formel (I),

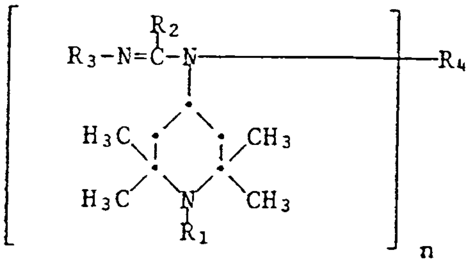

(I)

worin $R_1$ Wasserstoff, 0•, CN, NO, Cyanomethyl, $C_1$-$C_{12}$-Alkyl, $C_3$-$C_{12}$-Alkenyl oder $C_3$-$C_{12}$-Alkinyl unter der Voraussetzung, dass das an das Stickstoffatom gebundene Kohlenstoffatom primär ist, $C_7$-$C_{12}$-Aralkyl, Methylbenzyl, t-Butylbenzyl, Hydroxybenzyl, $C_1$-$C_{12}$-Acyl, 2,3-Epoxypropyl, OH-monosubstituiertes $C_2$-$C_6$-Alkyl oder 2,3-Dihydroxypropyl ist, $R_2$ für Wasserstoff, $C_1$-$C_{18}$-Alkyl, $C_5$-$C_{18}$-Cycloalkyl, Methylcyclohexyl, Trimethylcyclohexyl, t-Butylcyclohexyl, $C_6$-$C_{18}$-Aryl, Methylphenyl, Dimethylphenyl, Trimethylphenyl, t-Butylphenyl, Methoxyphenyl, Ethoxyphenyl, Hydroxyphenyl, 3,5-Di-t-butyl-4-hydroxyphenyl, $C_7$-$C_{18}$-Aralkyl, Methylbenzyl, Hydroxybenzyl, 3,5-Di-t-butyl-4-hydroxybenzyl oder 2-(3,5-Di-t-butyl-4-hydroxyphenyl)-ethyl, $R_3$ für $C_6$-$C_{18}$-Aryl, Methylphenyl, Dimethylphenyl, Trimethylphenyl, Ethylphenyl, Diethylphenyl, Hydroxyphenyl, 3,5-Di-t-butyl-4-hydroxyphenyl, Methoxyphenyl, Ethoxyphenyl, 4-Ethoxycarbonylphenyl, 4-(2,2,6,6-Tetramethyl-4-piperidyloxycarbonyl)-phenyl, 4-(1,2,2,6,6-Pentamethyl-4-piperidyloxycarbonyl)-phenyl oder eine Gruppe der Formel (II),

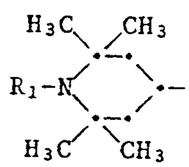

(II)

worin $R_1$ die oben angegebene Bedeutung hat, n für eine ganze Zahl von 1 bis 4 und, wenn n 1 ist, $R_4$ Wasserstoff,
CN, $-COR_5$, $-COOR_6$, $-CO-R_7-COOR_8$,

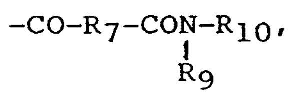 , 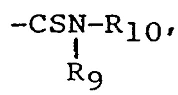 ,

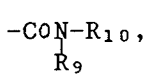 , $-SO_2R_{11}$, 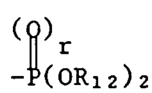 oder 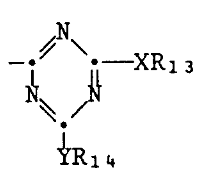 ist,

worin $R_5$ Wasserstoff, $C_1$-$C_{18}$-Alkyl, $C_5$-$C_{18}$-Cycloalkyl, Methylcyclohexyl, t-Butylcyclohexyl, $C_2$-$C_{18}$-Alkenyl, $C_6$-$C_{18}$-Aryl, Methylphenyl, Dimethylphenyl, Trimethylphenyl, Ethylphenyl, t-Butylphenyl, Methoxyphenyl, Ethoxyphenyl, Hydroxyphenyl, 3,5-Di-t-butyl-4-hydroxyphenyl, $C_7$-$C_{18}$-Aralkyl, 3,5-Di-t-butyl-4-hydroxybenzyl, 2-(3,5-Di-t-butyl-4-hydroxyphenyl)ethyl oder durch $C_1$-$C_{18}$-Alkoxy, $C_2$-$C_{18}$-Dialkylamino oder eine Gruppe der Formel (III)

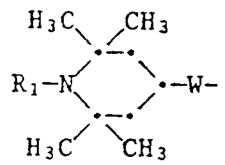

(III)

substituiertes $C_1$-$C_{10}$-Alkyl bedeutet, wobei $R_1$ die oben angegebene Bedeutung hat und W -O- oder $>N$-$R_{15}$ darstellt, worin $R_{15}$ für Wasserstoff, $C_1$-$C_{18}$-Alkyl, $C_5$-$C_{18}$-Cycloalkyl, Methylcyclohexyl, Dimethylcyclohexyl, Trimethylcyclohexyl, $C_3$-$C_{18}$-Alkenyl, $C_6$-$C_{18}$-Aryl, Methylphenyl, Dimethylphenyl, Trimethylphenyl, t-Butylphenyl, Methoxyphenyl, Ethoxyphenyl, Hydroxyphenyl, 3,5-Di-t-butyl-4-hydroxyphenyl, $C_7$-$C_{18}$-Aralkyl, Methylbenzyl, Hydroxybenzyl, 3,5-Di-t-butyl-4-hydroxybenzyl oder eine Gruppe der Formel (II) steht, $R_6$ $C_1$-$C_{18}$-Alkyl, durch OH, $C_1$-$C_{18}$-Alkoxy oder $C_2$-$C_{18}$-Dialkylamino substituiertes $C_2$-$C_6$-Alkyl, $C_5$-$C_{18}$-Cycloalkyl, Methylcyclohexyl, Dimethylcyclohexyl, Trimethylcyclohexyl, t-Butylcyclohexyl, $C_3$-$C_{18}$-Alkenyl, $C_6$-$C_{18}$-Aryl, Methylphenyl, Dimethylphenyl, Trimethylphenyl, Ethylphenyl, Isopropylphenyl, t-Butylphenyl, Di-t-butylphenyl, 2,6-Di-t-butyl-4-methylphenyl, Methoxyphenyl, Ethoxyphenyl, $C_7$-$C_{18}$-Aralkyl, Methylbenzyl, 3,5-Di-t-butyl-4-hydroxybenzyl oder eine Gruppe der Formel (II) und $R_7$ eine direkte Bindung, $C_1$-$C_{18}$-Alkylen, $C_2$-$C_{20}$-Alkyliden, $C_7$-$C_{20}$-Aralkyliden, $C_6$-$C_{10}$-Cycloalkylen, Methylcyclohexylen, $C_2$-$C_{18}$-Alkenylen oder $C_6$-$C_{10}$-Arylen bedeuten, $R_8$ die für $R_6$ angegebene Bedeutung hat oder Wasserstoff oder ein Alkalimetall darstellt, $R_9$ und $R_{10}$, die gleich oder verschieden sind, die für $R_{15}$ angegebene Bedeutung haben, oder $R_9$ und $R_{10}$ zusammen mit dem Stickstoffatom, an das sie gebunden sind, 1-Pyrrolidinyl, 1-Piperidyl, 4-Morpholinyl, 1-Hexahydroazepinyl oder 4-Methyl-1-piperazinyl bilden, $R_{11}$ $C_1$-$C_{12}$-Alkyl, $C_6$-$C_{12}$-Aryl, Methylphenyl, Dimethylphenyl, Trimethylphenyl, Methoxyphenyl oder Ethoxyphenyl, r 0 oder 1 und $R_{12}$ $C_1$-$C_{12}$-Alkyl oder eine Gruppe der Formel (II) bedeuten, oder die beiden Gruppen $R_{12}$ zusammen $C_2$-$C_{12}$-Alkylen oder eine Gruppe

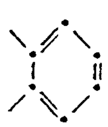 , 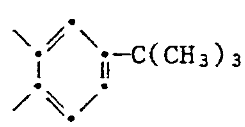 oder 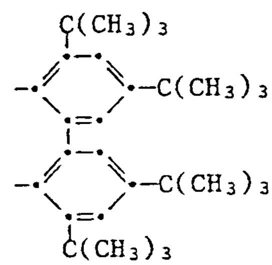

darstellen und zusammen mit dem Phosphoratom und den beiden Sauerstoffatomen einen 5- bis 7-gliedrigen Ring bilden, $R_{13}$ und $R_{14}$, die gleich oder verschieden sind, die für $R_6$ angegebene Bedeutung haben oder für Wasserstoff stehen, und X und Y, die gleich oder verschieden sind, eine direkte Bindung, -O- oder $>N$-$R_{16}$ bedeuten, wobei $R_{16}$ die für $R_{15}$ angegebene Bedeutung hat oder OH-monosubstituiertes $C_2$-$C_6$-Alkyl darstellt, oder die Gruppen $R_{13}X$- und $R_{14}Y$-, die gleich oder verschieden sind, 1-Pyrrolidinyl, 1-Piperidyl, 4-Morpholinyl, 1-Hexahydroazepinyl oder 4-Methyl-1-piperazinyl darstellen, und wenn n = 2,
$R_4$ -CO-, $-SO_2$-, -COO-$R_{17}$-OOC-, -COCOO-$R_{17}$-OOCCO-, -CO-$R_{18}$CO-,

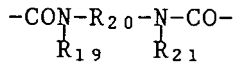 , 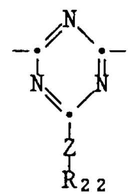 , 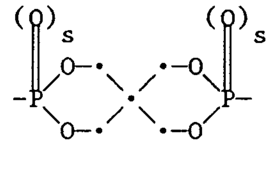 ,

$(-CO-R_7-COO)_2Me$ oder 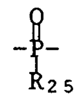 ist,

worin $R_{17}$ $C_2$-$C_{18}$-Alkylen, in der Kette durch 1 oder 2 Sauerstoffatome substituiertes $C_4$-$C_{12}$-Alkylen, $C_6$-$C_{18}$-Cycloalkylen, Cyclohexylendimethylen, Methylendicyclohexylen, Isopropylidendicyclohexylen, $C_4$-$C_8$-Alkenylen, $C_6$-$C_{18}$-Arylen, $C_8$-$C_{12}$-Aralkylen oder eine Gruppe der Formel (IV),

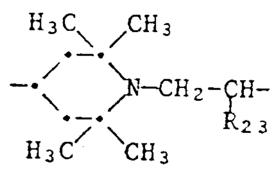 (IV)

worin $R_{23}$ für Wasserstoff, $C_1$-$C_4$-Alkyl oder Phenyl steht, und $R_{18}$ eine direkte Bindung, $C_1$-$C_{18}$-Alkylen, $C_2$-$C_{20}$-Alkyliden, $C_7$-$C_{20}$-Aralkyliden, $C_6$-$C_{10}$-Cycloalkylen, Methylcyclohexylen, $C_6$-$C_{10}$-Arylen oder in der Kette durch 1 oder 2 Sauerstoffatome oder 1 oder 2 Gruppen $>$N-$R_{24}$ wobei $R_{24}$ die für $R_{15}$ angegebene Bedeutung hat, substituiertes $C_2$-$C_{12}$-Alkylen bedeuten, $R_{19}$ und $R_{21}$, die gleich oder verschieden sind, die für $R_{15}$ angegebene Bedeutung haben, $R_{20}$ $C_2$-$C_{18}$-Alkylen, $C_6$-$C_{18}$-Cycloalkylen, Cyclohexylendimethylen, Methylendicyclohexylen, $C_6$-$C_{18}$-Arylen oder $C_8$-$C_{12}$-Aralkylen bedeutet, $R_{22}$ die für $R_{13}$ und $R_{14}$ angegebene Bedeutung und Z die für X und Y angegebene Bedeutung haben, oder -$ZR_{22}$ 1-Pyrrolidinyl, 1-Piperidyl, 4-Morpholinyl, 1-Hexahydroazepinyl, 4-Methyl-1-piperazinyl oder Halogen darstellt, $R_{25}$ für $C_1$-$C_4$-Alkyl und s für 0 oder 1 stehen, $R_7$ die oben angegebene Bedeutung hat, Me für Ba, Ca, Co", Mg, Mn", Ni", Sn" oder Zn steht, und wenn n = 3,

$R_4$ aliphatisches $C_4$-$C_{18}$-Triacyl, ein Triacylrest, welcher sich von Zitronensäure ableitet, durch ein Stickstoffatom substituiertes aliphatisches $C_6$-$C_{18}$-Triacyl, ein Triacylrest, welcher sich von 1,2,4-Benzoltricarbonsäure oder 1,3,5-Benzoltricarbonsäure ableitet, eine Gruppe

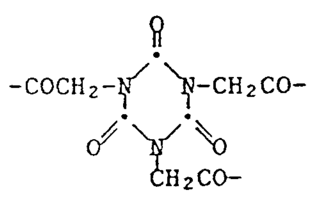 , 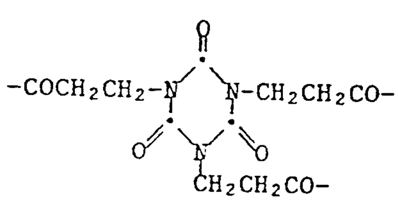 ,

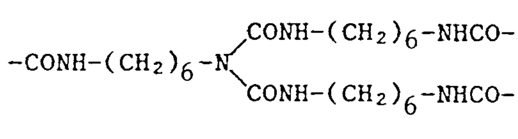 oder 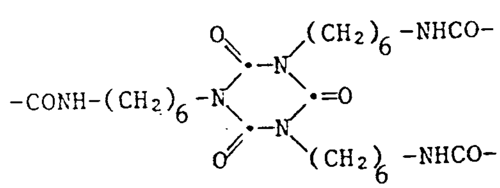

oder eine 1,3,5-Triazin-2,4,6-triylgruppe, eine ≡P-Gruppe, eine ≡PO-Gruppe oder eine Gruppe (-CO-$R_7$-COO)$_3$Al mit $R_7$ in der oben angegebenen Bedeutung ist, und wenn n = 4, $R_4$ aliphatisches $C_6$-$C_{18}$-Tetraacyl, durch zwei Stickstoffatome substituiertes aliphatisches $C_{10}$-$C_{18}$-Tetraacyl, aromatisches $C_{10}$-$C_{18}$-Tetraacyl, cycloaliphatisches $C_{10}$-$C_{22}$-Tetraacyl oder eine Gruppe (-CO-$R_7$-COO)$_4$Sn$^{IV}$ mit $R_7$ in der angegebenen Bedeutung ist.

**2.** Verbindungen nach Anspruch 1, worin $R_1$ für Wasserstoff, Methyl, Allyl, Benzyl oder Acetyl, $R_2$ für Wasserstoff, $C_1$-$C_{12}$-Alkyl, $C_6$-$C_9$-Cycloalkyl oder $C_6$-$C_9$-Aryl, $R_3$ für $C_6$-$C_{12}$-Aryl oder eine Gruppe der Formel (II), n für 1, 2 oder 3 und, wenn n 1 ist, $R_4$ für Wasserstoff oder eine der Gruppen
-$COR_5$, -$COOR_6$, -CO-$R_7$-$COOR_8$,

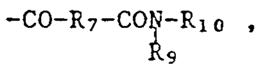 , 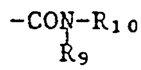 ,

-$SO_2R_{11}$ , 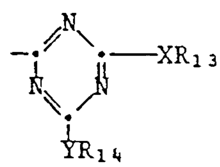

stehen, worin $R_5$ Wasserstoff, $C_1$-$C_{18}$-Alkyl, $C_6$-$C_9$-Cycloalkyl, $C_2$-$C_4$-Alkenyl, $C_6$-$C_8$-Aryl, $C_7$-$C_{16}$-Aralkyl oder durch eine Gruppe der Formel (III), wobei W -O- oder >N-$R_{15}$ und $R_{15}$ Wasserstoff, $C_1$-$C_{12}$-Alkyl oder $C_6$-$C_9$-Cycloalkyl darstellen, substituiertes $C_1$-$C_3$-Alkyl, $R_6$ $C_1$-$C_{12}$-Alkyl, $C_6$-$C_{12}$-Cycloalkyl oder eine Gruppe der Formel (II), $R_7$ eine direkte Bindung, $C_1$-$C_8$-Alkylen, $C_6$-$C_8$-Cycloalkylen, Vinylen oder Phenylen, $R_8$ $C_1$-$C_{12}$-Alkyl, $C_6$-$C_9$-Cycloalkyl, Allyl oder eine Gruppe der Formel (II) sowie $R_9$ und $R_{10}$, die gleich oder verschieden sind, Wasserstoff, $C_1$-$C_{12}$-Alkyl, $C_6$-$C_9$-Cycloalkyl, Allyl oder eine Gruppe der Formel (II) bedeuten oder zusammen mit dem Stickstoffatom, an das sie gebunden sind, 1-Pyrrolidinyl, 1-Piperidyl, 4-Morpholinyl, 1-Hexahydroazepinyl oder 4-Methyl-1-piperazinyl bilden, $R_{11}$ Methyl, Ethyl oder $C_6$-$C_9$-Aryl, $R_{13}$ und $R_{14}$, die gleich oder verschieden sind, Wasserstoff, $C_1$-$C_{12}$-Alkyl, $C_6$-$C_9$-Cycloalkyl, Allyl oder eine Gruppe der Formel (II) sowie X und Y, die gleich oder verschieden sind, -O- oder >N-$R_{16}$, wobei $R_{16}$ Wasserstoff, $C_1$-$C_{12}$-Alkyl, $C_6$-$C_9$-Cycloalkyl, Allyl oder eine Gruppe der Formel (II) darstellt, bedeuten oder die Gruppen $R_{13}$X- und $R_{14}$Y-, die gleich oder verschieden sind, eine 1-Pyrrolidinyl-, 1-Piperidyl-, 4-Morpholinyl- oder 1-Hexahydroazepinylgruppe bedeuten, oder, wenn n 2 ist, $R_4$ für eine -CO-Gruppe oder eine der Gruppen
-COO-$R_{17}$-OOC-, -CO-$R_{18}$-CO-,

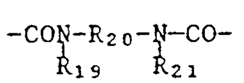 , 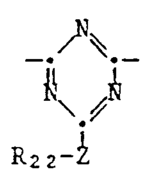

steht, worin $R_{17}$ $C_2$-$C_{10}$-Alkylen, $C_6$-$C_{15}$-Cycloalkylen, 2-Buten-1,4-diyl oder eine Gruppe der Formel (IV), wobei $R_{23}$ für Wasserstoff oder Methyl steht, $R_{18}$ eine direkte Bindung, $C_1$-$C_{12}$-Alkylen, $C_2$-$C_{12}$-Alkyliden oder in der Kette durch eine oder zwei Gruppen >N-$R_{24}$, wobei $R_{24}$ für $C_1$-$C_8$-Alkyl, $C_6$-$C_9$-Cycloalkyl oder eine Gruppe der Formel (II) steht, substituiertes $C_2$-$C_8$-Alkylen, $R_{19}$ und $R_{21}$, die gleich oder verschieden sind, Wasserstoff, $C_1$-$C_8$-Alkyl, $C_6$-$C_9$-Cycloalkyl oder eine Gruppe der Formel (II), $R_{20}$ $C_2$-$C_{10}$-Alkylen, $C_6$-$C_{15}$-Cycloalkylen, $C_6$-$C_{15}$-Arylen oder Xylylen, $R_{22}$ Wasserstoff, $C_1$-$C_{12}$-Alkyl, $C_6$-$C_9$-Cycloalkyl, Allyl oder eine Gruppe der Formel (II) und Z -O- oder >N-$R_{16}$ mit $R_{16}$ wie oben definiert bedeuten, oder, wenn n 3 ist, $R_4$ für aliphatisches $C_4$-$C_8$-Triacyl, eine Gruppe N($CH_2$CO-)$_3$, einen Triacylrest, welcher sich von 1,2,4-Benzoltricarbonsäure oder 1,3,5-Benzoltricarbonsäure ableitet,

oder für eine Gruppe

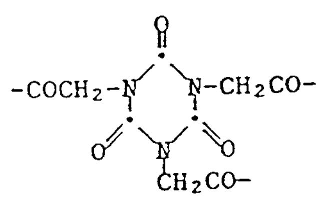

oder

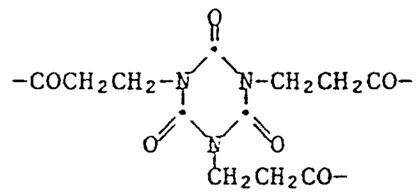

oder eine 1,3,5-Triazin-2,4,6-triylgruppe steht.

3. Verbindungen nach Anspruch 1, worin $R_1$ für Wasserstoff oder Methyl, $R_2$ für Wasserstoff oder Methyl, $R_3$ für 2,2,6,6-Tetramethyl-4-piperidyl oder 1,2,2,6,6-Pentamethyl-4-piperidyl, n für 1, 2 oder 3 und, wenn n 1 ist, $R_4$ für Wasserstoff oder eine der Gruppen
$-COR_5$, $-COOR_6$, $-COCOOR_8$,

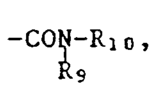

,

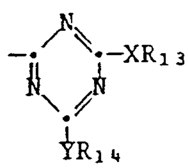

stehen, worin $R_5$ $C_1$-$C_{17}$-Alkyl, Cyclohexyl oder Phenyl, $R_6$ $C_2$-$C_{18}$-Alkyl, $C_6$-$C_{10}$-Cycloalkyl oder eine Gruppe der Formel (II), wobei $R_1$ für Wasserstoff oder Methyl steht, $R_8$ $C_2$-$C_{12}$-Alkyl, Cyclohexyl oder eine Gruppe der Formel (II), wobei $R_1$ für Wasserstoff oder Methyl steht, $R_9$ Wasserstoff, $R_{10}$ $C_4$-$C_{12}$-Alkyl oder Cyclohexyl, $R_{13}$ und $R_{14}$, die gleich oder verschieden sind, $C_1$-$C_{12}$-Alkyl, Cyclohexyl, Allyl oder eine Gruppe der Formel (II), wobei $R_1$ für Wasserstoff oder Methyl steht, sowie X und Y, die gleich oder verschieden sind, eine Gruppe $>N-R_{16}$, wobei $R_{16}$ Wasserstoff, $C_1$-$C_{12}$-Alkyl, Cyclohexyl, Allyl oder eine Gruppe der Formel (II) darstellt, wobei $R_1$ für Wasserstoff oder Methyl steht, bedeuten und, wenn n 2 ist, $R_4$ für eine der Gruppen
$-COO-R_{17}-OOC-$, $-CO-R_{18}-CO-$,

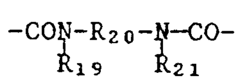

,

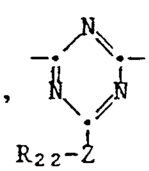

steht, worin $R_{17}$ $C_4$-$C_{10}$-Alkylen, $C_6$-$C_8$-Cycloalkylen oder eine Gruppe der Formel (IV), wobei $R_{23}$ für Wasserstoff steht, $R_{18}$ eine direkte Bindung, $C_1$-$C_8$-Alkylen, $C_2$-$C_8$-Alkyliden, Cyclohexylen oder Phenylen, $R_{19}$ und $R_{21}$ Wasserstoff, $R_{20}$ $C_6$-$C_9$-Alkylen oder $C_6$-$C_{13}$-Arylen, $R_{22}$ $C_1$-$C_{12}$-Alkyl, Cyclohexyl, Allyl, oder eine Gruppe der Formel (II), wobei $R_1$ für Wasserstoff oder Methyl steht, und Z -O- oder $>N-R_{16}$ mit $R_{16}$ wie oben definiert bedeuten und, wenn n 3 ist, $R_4$ für eine 1,3,5-Triazin-2,4,6-triylgruppe steht.

4. Verbindungen nach Anspruch 1, worin $R_1$ für Wasserstoff, $C_1$-$C_4$-Alkyl, Allyl, Benzyl oder Acetyl steht.

5. Verbindungen nach Anspruch 1, worin $R_2$ für Wasserstoff, $C_1$-$C_4$-Alkyl, Cyclohexyl, Phenyl oder Benzyl und $R_3$ für Phenyl, 4-Ethoxycarbonylphenyl oder eine Gruppe der Formel (II), worin $R_1$ die in Anspruch 1 angegebene Bedeutung hat, stehen.

6. Verbindungen nach Anspruch 1, worin $R_1$ für Wasserstoff oder Methyl, $R_2$ für Wasserstoff oder $C_1$-$C_4$-Alkyl und $R_3$ für 4-Ethoxycarbonylphenyl oder eine Gruppe der Formel (II), worin $R_1$ die oben angegebene Bedeutung hat, stehen.

7. Verbindungen nach Anspruch 1, worin n 1 oder 2 ist.

8. Verbindungen nach Anspruch 1, worin n 1 ist und $R_4$ für $-COR_5$, $-COOR_6$ oder

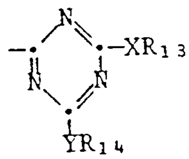

steht.

9. Verbindungen nach Anspruch 1, worin n 2 ist und $R_4$ für $-COO-R_{17}-OOC-$, $-CO-R_{18}-CO-$ oder

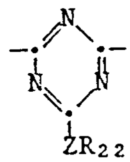

steht.

10. Verbindungen nach Anspruch 1, worin n 1, 2 oder 3 ist, und, wenn n = 1, $R_4$ Wasserstoff, $-COR_5$, $-COOR_6$, $-CO-R_7-COOR_8$,

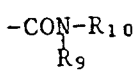 oder 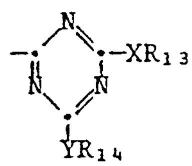 ist,

worin $R_5$ $C_1$-$C_{18}$-Alkyl, $R_6$ $C_1$-$C_{18}$-Alkyl, durch $C_1$-$C_4$-Alkoxy substituiertes $C_2$-$C_6$-Alkyl, Cyclohexyl, Methylcyclohexyl, Dimethylcyclohexyl, Trimethylcyclohexyl, t-Butylcyclohexyl oder eine Gruppe der Formel (II),
worin $R_1$ die in Anspruch 1 angegebene Bedeutung hat, $R_7$ $C_1$-$C_{10}$-Alkylen, $R_8$ Wasserstoff, $R_9$ und $R_{10}$ unabhängig voneinander Wasserstoff, $C_1$-$C_4$-Alkyl oder Cyclohexyl, $R_{13}$ und $R_{14}$ unabhängig voneinander Wasserstoff, $C_1$-$C_8$-Alkyl, Allyl oder eine Gruppe der Formel (II), worin $R_1$ die in Anspruch 1 angegebene Bedeutung hat, sowie X und Y unabhängig voneinander -O- oder $>N-R_{16}$ bedeuten, wobei $R_{16}$ für Wasserstoff, $C_1$-$C_8$-Alkyl, OH-monosubstituiertes $C_2$-$C_4$-Alkyl, Allyl oder eine Gruppe der Formel (II) steht, worin $R_1$ die in Anspruch 1 angegebene Bedeutung hat,
wenn n = 2, $R_4$ $-COO-R_{17}-OOC-$, $-CO-R_{18}-CO-$,

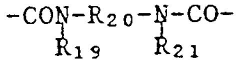, 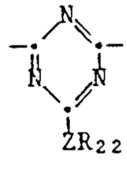 oder 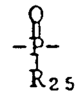 ist,

worin $R_{17}$ $C_2$-$C_{12}$-Alkylen oder in der Kette durch 1 oder 2 Sauerstoffatome substituiertes $C_4$-$C_{12}$-Alkylen, $R_{18}$ eine direkte Bindung oder $C_1$-$C_{10}$-Alkylen, $R_{19}$ und $R_{21}$ Wasserstoff, $R_{20}$ $C_2$-$C_{10}$-Alkylen, Cyclohexylen, $R_{22}$ $C_1$-$C_8$-Alkyl, OH-monosubstituiertes $C_2$-$C_4$-Alkyl oder Allyl, Z -O- oder $>N-R_{16}$ mit $R_{16}$ in der oben angegebenen Bedeutung oder $-ZR_{22}$ Halogen und $R_{25}$ $C_1$-$C_4$-Alkyl bedeuten, und

wenn n = 3, $R_4$ für 1,3,5-Triazin-2,4,6-triyl steht.

**11.** Verbindungen nach Anspruch 1, worin $R_1$ für Wasserstoff, $R_2$ für Wasserstoff oder Methyl, $R_3$ für 2,2,6,6-Tetramethyl-4-piperidyl, n für 1, 2 oder 3 und, wenn n = 1, $R_4$ für $-COR_5$, $-COOR_6$ oder

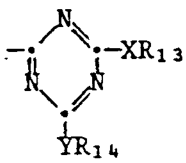

stehen, worin $R_5$ $C_1$-$C_{18}$-Alkyl, $R_6$ $C_1$-$C_{18}$-Alkyl, Cyclohexyl, 4-t-Butylcyclohexyl oder 1,2,2,6,6-Pentamethyl-4-piperidyl, $R_{13}$ und $R_{14}$ unabhängig voneinander $C_1$-$C_8$-Alkyl oder Allyl sowie X und Y unabhängig voneinander -O- oder $>N-R_{16}$, wobei $R_{16}$ Wasserstoff, $C_1$-$C_8$-Alkyl, Allyl oder 2,2,6,6-Tetramethyl-4-piperidyl darstellt, bedeuten und, wenn n = 2, $R_4$ für
$-COO-R_{17}-OOC-$ , $CO-R_{18}-CO-$ oder

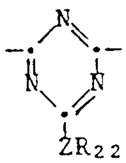

steht, worin $R_{17}$ $C_4$-$C_6$-Alkylen, $R_{18}$ eine direkte Bindung oder $C_1$-$C_8$-Alkylen, $R_{22}$ $C_1$-$C_8$-Alkyl oder Allyl und Z -O- oder $>N-R_{16}$ mit $R_{16}$ wie oben definiert bedeuten und, wenn n 3 ist, $R_4$ für eine 1,3,5-Triazin-2,4,6-triylgruppe steht.

**12.** Verbindungen nach Anspruch 1, worin $R_1$ für Wasserstoff, $R_2$ für Wasserstoff oder Methyl, $R_3$ für 2,2,6,6-Tetramethyl-4-piperidyl, n für 1 oder 2 und, wenn n = 1, $R_4$ für $-COOR_6$ oder

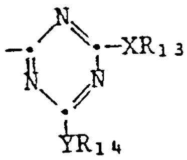

stehen, worin $R_6$ $C_4$-$C_{18}$-Alkyl, Cyclohexyl, 4-t-Butylcyclohexyl oder 1,2,2,6,6-Pentamethyl-4-piperidyl, $R_{13}$ und $R_{14}$ unabhängig voneinander $C_1$-$C_4$-Alkyl sowie X und Y $>N-R_{16}$ mit $R_{16}$ als 2,2,6,6-Tetramethyl-4-piperidyl bedeuten, und, wenn n = 2, $R_4$ für $-COO-R_{17}-OOC-$ oder

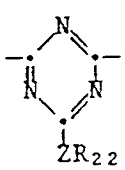

steht, worin $R_{17}$ $C_4$-$C_6$-Alkylen, $R_{22}$ $C_1$-$C_4$-Alkyl oder Allyl und Z -O- oder $>N-R_{16}$, wobei $R_{16}$ Wasserstoff, $C_1$-$C_4$-Alkyl oder Allyl darstellt, bedeuten.

**13.** Die Verbindungen

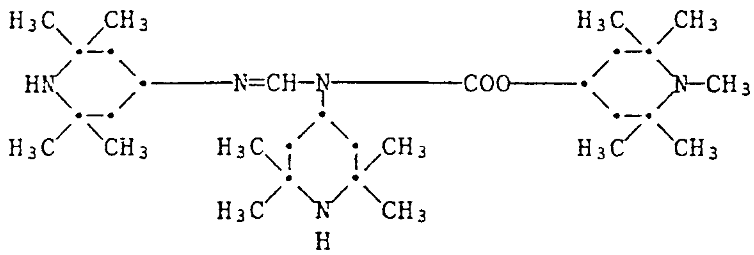

,

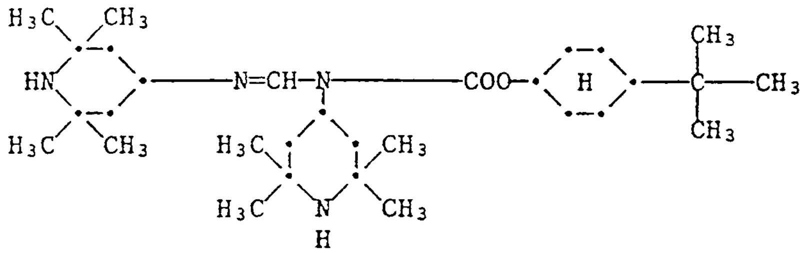

,

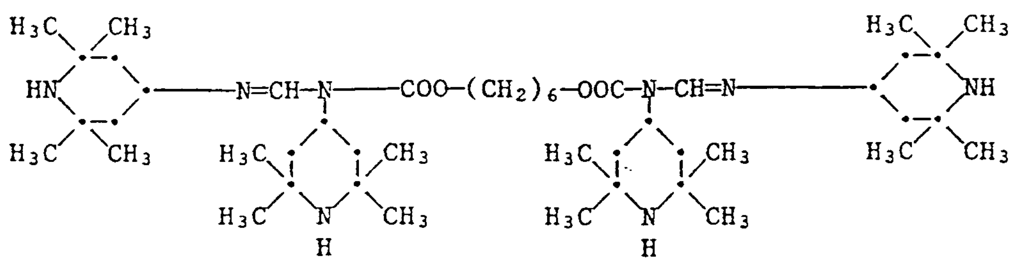

,

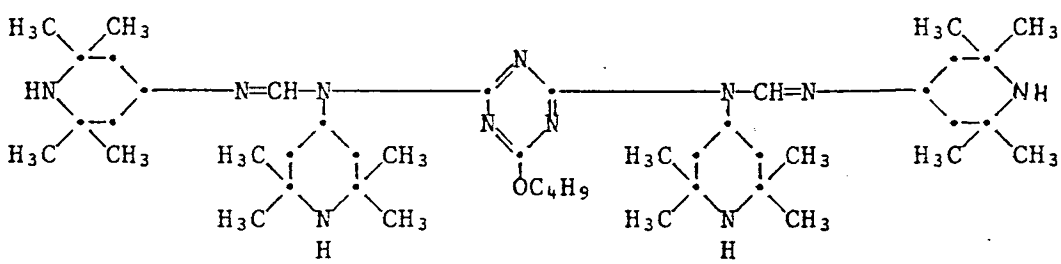

und

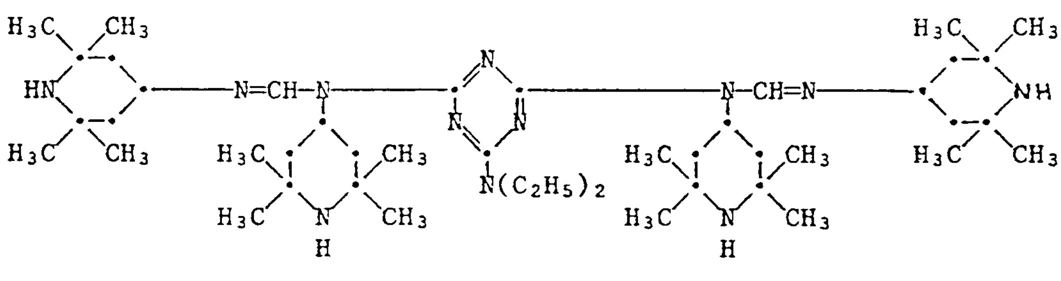

als Verbindungen nach Anspruch 1.

**14.** Zusammensetzung enthaltend ein gegen oxidativen, thermischen und / oder licht-induzierten Abbau empfindliches organisches Material und mindestens eine Verbindung nach Anspruch 1.

**15.** Zusammensetzung nach Anspruch 14, worin als organisches Material ein synthetisches Polymer vorliegt.

**16.** Zusammensetzung nach Anspruch 14, worin als organisches Material ein Polyolefin vorliegt.

**17.** Zusammensetzung nach Anspruch 16, worin als Polyolefin Polyethylen oder Polypropylen vorliegt.

**18.** Zusammensetzung nach Anspruch 14, die neben der Verbindung der Formel (I) ferner weitere übliche Zusatzstoffe für synthetische Polymere enthält.

**19.** Verwendung von Verbindungen nach Anspruch 1 zum Stabilisieren von organischem Material gegen thermischen, oxidativen und / oder licht-induzierten Abbau.

## Claims

**1.** A compound of the formula (I)

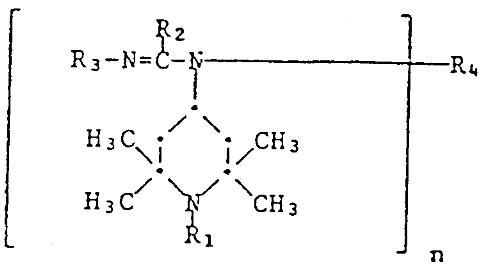

(I)

in which $R_1$ is hydrogen, O•, CN, NO, cyanomethyl, $C_1$-$C_{12}$alkyl, $C_3$-$C_{12}$alkenyl or $C_3$-$C_{12}$alkynyl subject to the proviso that the carbon atom attached to the nitrogen atom is a primary carbon atom, $C_7$-$C_{12}$aralkyl, methylbenzyl, t-butylbenzyl, hydroxybenzyl, $C_1$-$C_{12}$acyl, 2,3-epoxypropyl, OH-monosubstituted $C_2$-$C_6$alkyl or 2,3-dihydroxypropyl, $R_2$ is hydrogen, $C_1$-$C_{18}$alkyl, $C_5$-$C_{18}$cycloalkyl, methylcyclohexyl, trimethylcyclohexyl, t-butylcyclohexyl, $C_6$-$C_{18}$aryl, methylphenyl, dimethylphenyl, trimethylphenyl, t-butylphenyl, methoxyphenyl, ethoxyphenyl, hydroxyphenyl, 3,5-di-t-butyl-4-hydroxyphenyl, $C_7$-$C_{18}$aralkyl, methylbenzyl, hydroxybenzyl, 3,5-di-t-butyl-4-hydroxybenzyl or 2-(3,5-di-t-butyl-4-hydroxyphenyl)-ethyl, $R_3$ is $C_6$-$C_{18}$aryl, methylphenyl, dimethylphenyl, trimethylphenyl, ethylphenyl, diethylphenyl, hydroxyphenyl, 3,5-di-t-butyl-4-hydroxyphenyl, methoxyphenyl, ethoxyphenyl, 4-ethoxycarbonylphenyl, 4-(2,2,6,6-tetramethyl-4-piperidyloxycarbonyl)-phenyl, 4-(1,2,2,6,6-pentamethyl-4-piperidyloxycarbonyl)-phenyl or a group of the formula (II)

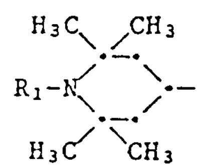

(II)

in which $R_1$ is as defined above, n is an integer from 1 to 4 and, if n = 1, $R_4$ is hydrogen, CN, $-COR_5$, $-COOR_6$, $-CO-R_7-COOR_8$,

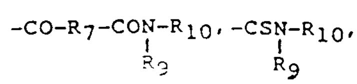

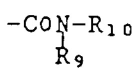, $-SO_2R_{11}$, 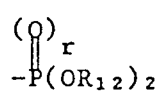 or 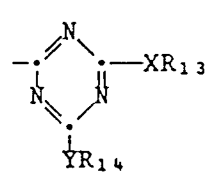

in which $R_5$ is hydrogen, $C_1$-$C_{18}$alkyl, $C_5$-$C_{18}$cycloalkyl, methylcyclohexyl, t-butylcyclohexyl, $C_2$-$C_{18}$alkenyl, $C_6$-$C_{18}$aryl, methylphenyl, dimethylphenyl, trimethylphenyl, ethylphenyl, t-butylphenyl, methoxyphenyl, ethoxyphenyl, hydroxyphenyl, 3,5-di-t-butyl-4-hydroxyphenyl, $C_7$-$C_{18}$aralkyl, 3,5-di-t-butyl-4-hydroxybenzyl, 2-(3,5-di-t-butyl-4-hydroxyphenyl)-ethyl or $C_1$-$C_{10}$alkyl substituted by $C_1$-$C_{18}$alkoxy, by $C_2$-$C_{18}$dialkylamino or by a group of the formula (III)

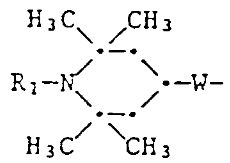 (III)

where $R_1$ is as defined above and W is -O- or $>$N-$R_{15}$ in which $R_{15}$ is hydrogen, $C_1$-$C_{18}$alkyl, $C_5$-$C_{18}$cycloalkyl, methylcyclohexyl, dimethylcyclohexyl, trimethylcyclohexyl, $C_3$-$C_{18}$alkenyl, $C_6$-$C_{18}$aryl, methylphenyl, dimethylphenyl, trimethylphenyl, t-butylphenyl, methoxyphenyl, ethoxyphenyl, hydroxyphenyl, 3,5-di-t-butyl-4-hydroxyphenyl, $C_7$-$C_{18}$aralkyl, methylbenzyl, hydroxybenzyl, 3,5-di-t-butyl-4-hydroxybenzyl or a group of the formula (II), $R_6$ is $C_1$-$C_{18}$alkyl, $C_2$-$C_6$alkyl substituted by OH, by $C_1$-$C_{18}$alkoxy or by $C_2$-$C_{18}$dialkylamino, $C_5$-$C_{18}$cycloalkyl, methylcyclohexyl, dimethylcyclohexyl, trimethylcyclohexyl, t-butylcyclohexyl, $C_3$-$C_{18}$alkenyl, $C_6$-$C_{18}$aryl, methylphenyl, dimethylphenyl, trimethylphenyl, ethylphenyl, isopropylphenyl, t-butylphenyl, di-t-butylphenyl, 2,6-di-t-butyl-4-methylphenyl, methoxyphenyl, ethoxyphenyl, $C_7$-$C_{18}$aralkyl, methylbenzyl, 3,5-di-t-butyl-4-hydroxybenzyl or a group of the formula (II), $R_7$ is a direct bond, $C_1$-$C_{18}$alkylene, $C_2$-$C_{20}$alkylidene, $C_7$-$C_{20}$aralkylidene, $C_6$-$C_{10}$cycloalkylene, methylcyclohexylene, $C_2$-$C_{18}$alkenylene or $C_6$-$C_{10}$arylene, $R_8$ is as defined for $R_6$ or is hydrogen or an alkali metal, $R_9$ and $R_{10}$, which are identical or different, are as defined for $R_{15}$, or $R_9$ and $R_{10}$, together with the nitrogen atom to which they are bonded, form 1-pyrrolidinyl, 1-piperidyl, 4-morpholinyl, 1-hexahydroazepinyl or 4-methyl-1-piperazinyl, $R_{11}$ is $C_1$-$C_{12}$alkyl, $C_6$-$C_{12}$aryl, methylphenyl, dimethylphenyl, trimethylphenyl, methoxyphenyl or ethoxyphenyl, r is 0 or 1, $R_{12}$ is $C_1$-$C_{12}$alkyl or a group of the formula (II),

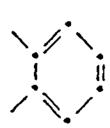 , 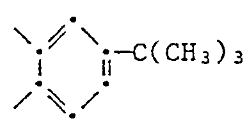 or 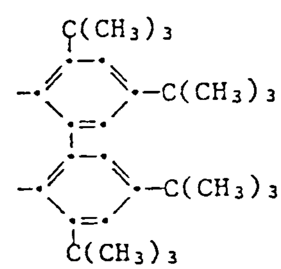

or the two groups $R_{12}$ together are $C_2$-$C_{12}$alkylene or a group and together with the phosphorus atom and the two oxygen atoms form a 5-membered to 7-membered ring, $R_{13}$ and $R_{14}$, which are identical or different, are as defined for $R_6$ or are hydrogen, and X and Y, which are identical or different, are a direct bond, -O- or $>$N-$R_{16}$, where $R_{16}$ is as defined for $R_{15}$ or is OH-monosubstituted $C_2$-$C_6$alkyl, or the groups $R_{13}$X- and $R_{14}$Y-, which are identical or different, are 1-pyrrolidinyl, 1-piperidyl, 4-morpholinyl, 1-hexahydroazepinyl or 4-methyl-1-piperazinyl, and if n = 2, $R_4$ is -CO-, -$SO_2$-, -COO-$R_{17}$-OOC-, -COCOO-$R_{17}$-OOCCO, -CO-$R_{18}$-CO-,

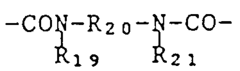 , 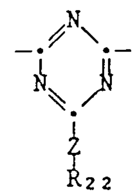 , 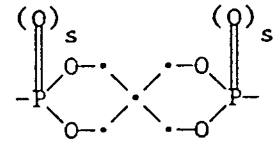 ,

$(-CO-R_7-COO)_2Me$ or 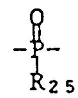

in which $R_{17}$ is $C_2$-$C_{18}$alkylene, $C_4$-$C_{12}$alkylene substituted in the chain by 1 or 2 oxygen atoms, $C_6$-$C_{18}$cycloalkylene, cyclohexylenedimethylene, methylenedicylcohexylene, isopropylidenedicyclohexylene, $C_4$-$C_8$alkenylene, $C_6$-$C_{18}$arylene, $C_8$-$C_{12}$aralkylene or a group of the formula (IV)

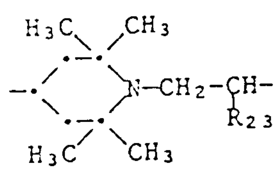 (IV)

in which $R_{23}$ is hydrogen, $C_1$-$C_4$alkyl or phenyl, $R_{18}$ is a direct bond, $C_1$-$C_{18}$alkylene, $C_2$-$C_{20}$alkylidene, $C_7$-$C_{20}$aralkylidene, $C_6$-$C_{10}$cycloalkylene, methylcylcohexylene, $C_6$-$C_{10}$arylene or $C_2$-$C_{12}$alkylene substituted in the chain by 1 or 2 oxygen atoms or by 1 or 2 groups $>$N-$R_{24}$, where $R_{24}$ is as defined for $R_{15}$, $R_{19}$ and $R_{21}$, which are identical or different, are as defined for $R_{15}$, $R_{20}$ is $C_2$-$C_{18}$alkylene, $C_6$-$C_{18}$cycloalkylene, cyclohexylenedimethylene, methylenedicyclohexylene, $C_6$-$C_{18}$arylene or $C_8$-$C_{12}$aralkylene, $R_{22}$ is as defined for $R_{13}$ and $R_{14}$, Z is as defined for X and Y, or -$ZR_{22}$ is 1-pyrrolidinyl, 1-piperidyl, 4-morpholinyl, 1-hexahydroazepinyl, 4-methyl-1-piperazinyl, or halogen, $R_{25}$ is $C_1$-$C_4$alkyl, s is 0 or 1, $R_7$ is as defined above and Me is Ba, Ca, Co", Mg, Mn", Ni", Sn" or Zn,

and if n = 3, $R_4$ is aliphatic $C_4$-$C_{18}$triacyl, a triacyl radical derived from citric acid, aliphatic $C_6$-$C_{18}$triacyl substituted by a nitrogen atom, a triacyl radical derived from 1,2,4-benzenetricarboxylic acid or 1,3,5-benzenetricarboxylic acid, a group

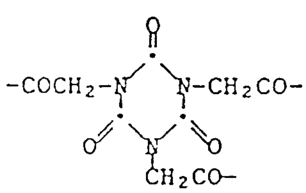 , 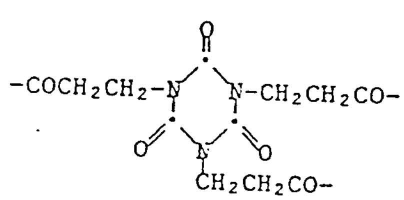 ,

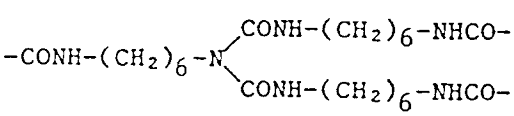 or 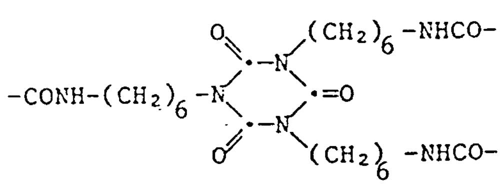

or a 1,3,5-triazine-2,4,6-triyl group, a ≡P group, a ≡PO group or a group $(-CO-R_7-COO)_3Al$ with $R_7$ as defined above,

and if n = 4, $R_4$ is aliphatic $C_6$-$C_{18}$tetraacyl, aliphatic $C_{10}$-$C_{18}$tetraacyl substituted by two nitrogen atoms, aromatic $C_{10}$-$C_{18}$tetraacyl, cycloaliphatic $C_{10}$-$C_{22}$tetraacyl or a group $(-CO-R_7COO)_4Sn^{IV}$, where

$R_7$ is as defined above.

**2.** A compound according to claim 1, in which $R_1$ is hydrogen, methyl, allyl, benzyl or acetyl, $R_2$ is hydrogen, $C_1$-$C_{12}$alkyl, $C_6$-$C_9$cycloalkyl or $C_6$-$C_9$aryl, $R_3$ is $C_6$-$C_{12}$aryl or a group of the formula (II), n is 1, 2 or 3 and, if n is 1, $R_4$ is hydrogen or one of the groups
$-COR_5$, $-COOR_6$, $-CO-R_7-COOR_8$,

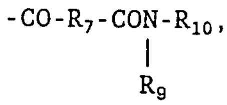, 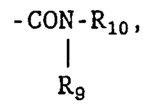,

$-SO_2R_{11}$, 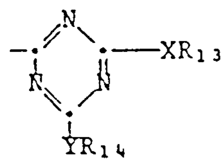

in which $R_5$ is hydrogen, $C_1$-$C_{18}$alkyl, $C_6$-$C_9$cycloalkyl, $C_2$-$C_4$alkenyl, $C_6$-$C_8$aryl, $C_7$-$C_{16}$aralkyl or $C_1$-$C_3$alkyl substituted by a group of the formula (III) where W is -O- or $>N-R_{15}$ and $R_{15}$ is hydrogen, $C_1$-$C_{12}$alkyl or $C_6$-$C_9$cycloalkyl, $R_6$ is $C_1$-$C_{12}$alkyl, $C_6$-$C_{12}$cycloalkyl or a group of the formula (II), $R_7$ is a direct bond, $C_1$-$C_8$alkylene, $C_6$-$C_8$cycloalkylene, vinylene or phenylene, $R_8$ is $C_1$-$C_{12}$alkyl, $C_6$-$C_9$cycloalkyl, allyl or a group of the formula (II), $R_9$ and $R_{10}$, which are identical or different, are hydrogen, $C_1$-$C_{12}$alkyl, $C_6$-$C_9$cycloalkyl, allyl or a group of the formula (II), or, together with the nitrogen atom to which they are bonded, form 1-pyrrolidinyl, 1-piperidyl, 4-morpholinyl, 1-hexahydroazepinyl or 4-methyl-1-piperazinyl, $R_{11}$ is methyl, ethyl, or $C_6$-$C_9$aryl, $R_{13}$ and $R_{14}$, which are identical or different, are hydrogen, $C_1$-$C_{12}$alkyl, $C_6$-$C_9$cycloalkyl, allyl or a group of the formula (II), X and Y, which are identical or different, are -O- or $>N-R_{16}$, where $R_{16}$ is hydrogen, $C_1$-$C_{12}$alkyl, $C_6$-$C_9$cycloalkyl, allyl or a group of the formula (II), or the groups $R_{13}X$- and $R_{14}Y$-, which are identical or different, are a 1-pyrrolidinyl, 1-piperidyl, 4-morpholinyl or 1-hexahydroazepinyl group or, if n is 2, $R_4$ is a -CO- group or one of the groups
$-COO-R_{17}-OOC-$, $-CO-R_{18}-CO-$,

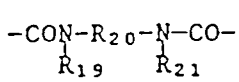 , 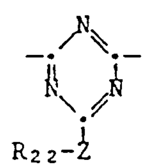

in which $R_{17}$ is $C_2$-$C_{10}$alkylene, $C_6$-$C_{15}$cycloalkylene, 2-butene-1,4-diyl or a group of the formula (IV) in which $R_{23}$ is hydrogen or methyl, $R_{18}$ is a direct bond, $C_1$-$C_{12}$alkylene, $C_2$-$C_{12}$alkylidene, or $C_2$-$C_8$alkylene substituted in the chain by one or two groups $>N-R_{24}$ where $R_{24}$ is $C_1$-$C_8$alkyl, $C_6$-$C_9$cycloalkyl or a group of the formula (II), $R_{19}$ and $R_{21}$, which are identical or different, are hydrogen, $C_1$-$C_8$alkyl, $C_6$-$C_9$cycloalkyl or a group of the formula (II), $R_{20}$ is $C_2$-$C_{10}$alkylene, $C_6$-$C_{15}$cycloalkylene, $C_6$-$C_{15}$arylene or xylylene, $R_{22}$ is hydrogen, $C_1$-$C_{12}$alkyl, $C_6$-$C_9$cycloalkyl, allyl or a group of the formula (II) and Z is -O- or $>N-R_{16}$ with $R_{16}$ as defined above, or, if n is 3, $R_4$ is aliphatic $C_4$-$C_8$triacyl, a group $N(CH_2CO-)_3$, a triacyl radical derived from 1,2,4-benzenetricarboxylic acid or 1,3,5-benzenetricarboxylic acid, a group

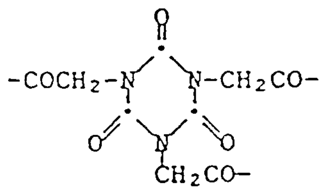 or 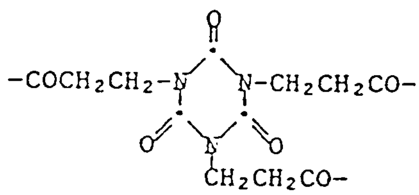

or a 1,3,5-triazine-2,4,6-triyl group.

**3.** A compound according to claim 1, in which $R_1$ is hydrogen or methyl, $R_2$ is hydrogen or methyl, $R_3$ is 2,2,6,6-tetramethyl-4-piperidyl or 1,2,2,6,6-pentamethyl-4-piperidyl, n is 1, 2 or 3, and, if n is 1, $R_4$ is hydrogen or one of the groups
$-COR_5$, $-COOR_6$, $-COCOOR_8$,

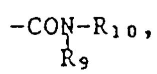 , 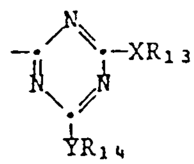

in which $R_5$ is $C_1$-$C_{17}$alkyl, cyclohexyl or phenyl, $R_6$ is $C_2$-$C_{18}$alkyl, $C_6$-$C_{10}$cycloalkyl or a group of the formula (II) with $R_1$ being hydrogen or methyl, $R_8$ is $C_2$-$C_{12}$alkyl, cyclohexyl or a group of the formula (II) with $R_1$ being hydrogen or methyl, $R_9$ is hydrogen, $R_{10}$ is $C_4$-$C_{12}$alkyl or cyclohexyl, $R_{13}$ and $R_{14}$, which are identical or different, are $C_1$-$C_{12}$alkyl, cyclohexyl, allyl or a group of the formula (II) with $R_1$ being hydrogen or methyl, and X and Y, which are identical or different, are a group $>N-R_{16}$ where $R_{16}$ is hydrogen, $C_1$-$C_{12}$alkyl, cyclohexyl, allyl or a group of the formula (II) with $R_1$ being hydrogen or methyl and, if n is 2, $R_4$ is one of the groups
$-COO-R_{17}-OOC-$, $-CO-R_{18}-CO-$,

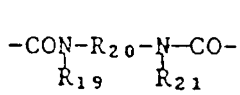 , 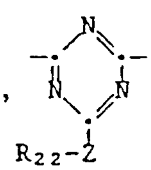

in which $R_{17}$ is $C_4$-$C_{10}$alkylene, $C_6$-$C_8$cycloalkylene or a group of the formula (IV) in which $R_{23}$ is hydrogen, $R_{18}$ is a direct bond, $C_1$-$C_8$alkylene, $C_2$-$C_8$alkylidene, cyclohexylene or phenylene, $R_{19}$ and $R_{21}$ are hydrogen, $R_{20}$ is $C_6$-$C_9$alkylene or $C_6$-$C_{13}$arylene, $R_{22}$ is $C_1$-$C_{12}$alkyl, cyclohexyl, allyl or a group of the formula (II) with $R_1$ being hydrogen or methyl, and Z is -O- or $>N-R_{16}$ with $R_{16}$ being as defined above, and, if n is 3, $R_4$ is a 1,3,5-triazine-2,4,6-triyl group.

**4.** A compound according to claim 1 in which $R_1$ is hydrogen, $C_1$-$C_4$alkyl, allyl, benzyl or acetyl.

**5.** A compound according to claim 1 in which $R_2$ is hydrogen, $C_1$-$C_4$alkyl, cyclohexyl, phenyl or benzyl and $R_3$ is phenyl, 4-ethoxycarbonylphenyl or a group of the formula (II) in which $R_1$ is as defined in claim 1.

**6.** A compound according to claim 1 in which $R_1$ is hydrogen or methyl, $R_2$ is hydrogen or $C_1$-$C_4$alkyl and $R_3$ is 4-ethoxycarbonylphenyl or a group of the formula (II) in which $R_1$ is as defined above.

**7.** A compound according to claim 1 in which n is 1 or 2.

**8.** A compound according to claim 1 in which n is 1 and $R_4$ is $-COR_5$, $-COOR_6$ or

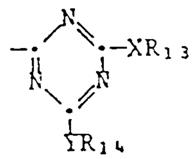

**9.** A compound according to claim 1 which n is 2 and $R_4$ is $-COO-R_{17}-OOC-$, $-CO-R_{18}-CO-$ or

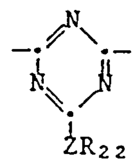

**10.** A compound according to claim 1 in which n is 1, 2 or 3 and, if n = 1, $R_4$ is hydrogen, $-COR_5$, $-COOR_6$, $-CO-R_7-COOR_8$,

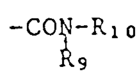

or

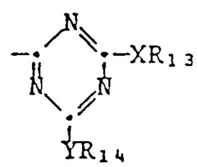

in which $R_5$ is $C_1$-$C_{18}$alkyl, $R_6$ is $C_1$-$C_{18}$alkyl, $C_2$-$C_6$alkyl substituted by $C_1$-$C_4$alkoxy, cyclohexyl, methylcyclohexyl, dimethylcyclohexyl, trimethylcyclohexyl, t-butylcyclohexyl or a group of the formula (II) in which $R_1$ is as defined in claim 1, $R_7$ is $C_1$-$C_{10}$alkylene, $R_8$ is hydrogen, $R_9$ and $R_{10}$ are independently of one another hydrogen, $C_1$-$C_4$alkyl or cyclohexyl, $R_{13}$ and $R_{14}$ are independently of one another hydrogen, $C_1$-$C_8$alkyl, allyl or a group of the formula (II) in which $R_1$ is as defined in claim 1, and X and Y are independently of one another -O- or $>N-R_{16}$, where
$R_{16}$ is hydrogen, $C_1$-$C_8$alkyl, OH-monosubstituted $C_2$-$C_4$alkyl, allyl or a group of the formula (II) in which $R_1$ is as defined in claim 1,
if n = 2, $R_4$ is $-COO-R_{17}-OOC-$, $-CO-R_{18}-CO-$, $-CO-R_{18}-CO-$,

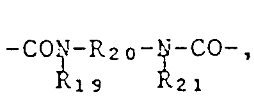

,

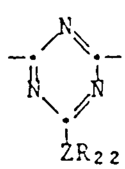

or

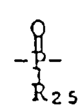

in which $R_{17}$ is $C_2$-$C_{12}$alkylene or $C_4$-$C_{12}$alkylene substituted in the chain by 1 or 2 oxygen atoms,
$R_{18}$ is a direct bond or $C_1$-$C_{10}$alkylene, $R_{19}$ and $R_{21}$ are hydrogen, $R_{20}$ is $C_2$-$C_{10}$alkylene or cyclohexylene,
$R_{22}$ is $C_1$-$C_8$alkyl, OH-monosubstituted $C_2$-$C_4$alkyl or allyl, Z is -O- or $>N-R_{16}$ with $R_{16}$ being as defined above, or $-ZR_{22}$ is halogen, $R_{25}$ is $C_1$-$C_4$alkyl and,
if n = 3, $R_4$ is 1,3,5-triazine-2,4,6-triyl.

**11.** A compound according to claim 1 in which $R_1$ is hydrogen, $R_2$ is hydrogen or methyl, $R_3$ is 2,2,6,6-tetramethyl-4-piperidyl, n is 1, 2 or 3, and if n = 1, $R_4$ is $-COR_5$, $-COOR_6$ or

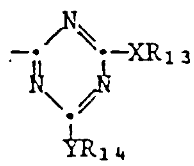

in which $R_5$ is $C_1$-$C_{18}$alkyl, $R_6$ is $C_1$-$C_{18}$alkyl, cyclohexyl, 4-t-butyl-cyclohexyl or 1,2,2,6,6-pentamethyl-4-piperidyl, $R_{13}$ and $R_{14}$ are independently of one another $C_1$-$C_8$alkyl or allyl, and X and Y are independently of one another -O- or $>$N-$R_{16}$ with $R_{16}$ being hydrogen, $C_1$-$C_8$alkyl, allyl or 2,2,6,6-tetramethyl-4-piperidyl and, if n = 2, $R_4$ is
-COO-$R_{17}$-OOC-, -CO-$R_{18}$-CO- or

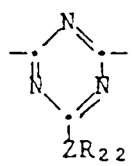

in which $R_{17}$ is $C_4$-$C_6$alkylene, $R_{18}$ is a direct bond or $C_1$-$C_8$alkylene, $R_{22}$ is $C_1$-$C_8$alkyl or allyl and Z is -O- or $>$N-$R_{16}$ with $R_{16}$ being as defined above and, if n is 3, $R_4$ is a 1,3,5-triazine-2,4,6-triyl group.

**12.** A compound according to claim 1 in which $R_1$ is hydrogen, $R_2$ is hydrogen or methyl, $R_3$ is 2,2,6,6-tetramethyl-4-piperidyl, n is 1 or 2 and, if n = 1, $R_4$ is -COO$R_6$ or

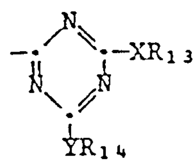

in which $R_6$ is $C_4$-$C_{18}$alkyl, cyclohexyl, 4-t-butylcyclohexyl or 1,2,2,6,6-pentamethyl-4-piperidyl, $R_{13}$ and $R_{14}$ are independently of one another $C_1$-$C_4$alkyl, and X and Y are $>$N-$R_{16}$ with $R_{16}$ being 2,2,6,6-tetramethyl-4-piperidyl and, if n = 2, $R_4$ is -COO-$R_{17}$-OOC- or

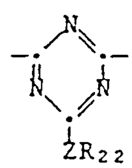

in which $R_{17}$ is $C_4$-$C_6$alkylene, $R_{22}$ is $C_1$-$C_4$alkyl or allyl and Z is -O- or $>$N-$R_{16}$ with $R_{16}$ being hydrogen, $C_1$-$C_4$alkyl or allyl.

**13.** The compounds

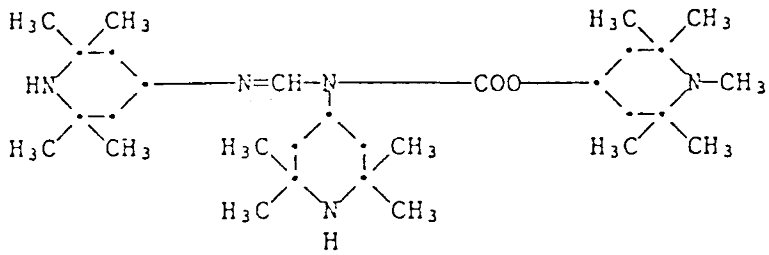

,

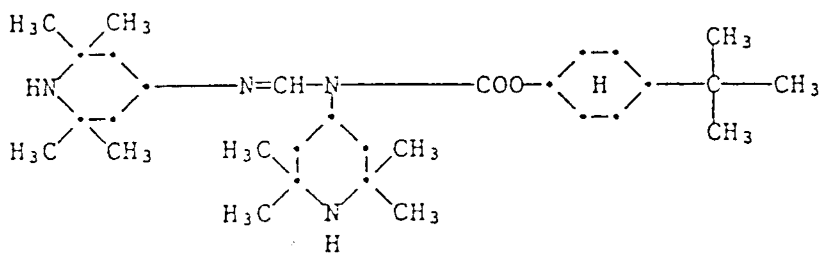

,

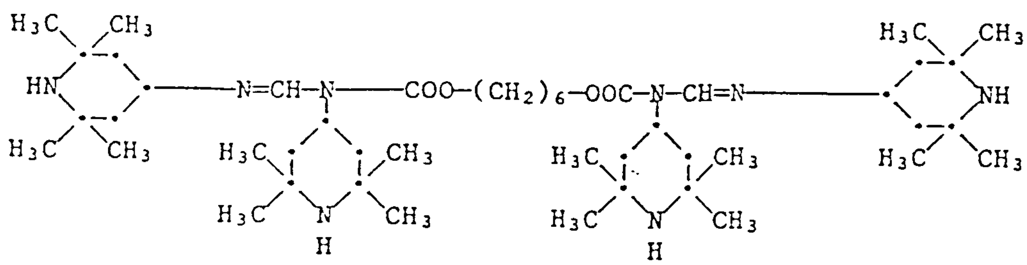

,

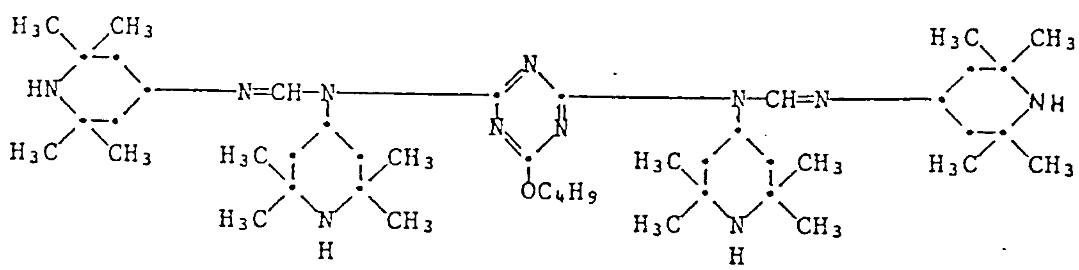

and

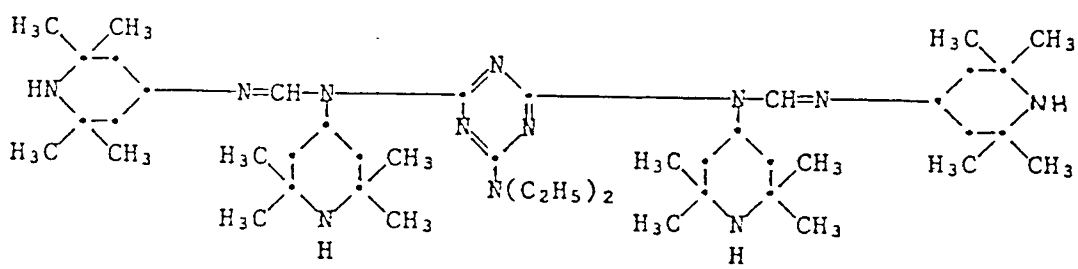

as compounds according to claim 1.

**14.** A composition comprising an organic material subject to oxidative thermal and/or light-induced degradation and at least one compound according to claim 1.

**15.** A composition according to claim 14, in which the organic material is a synthetic polymer.

**16.** A composition according to claim 14, in which the organic material is a polyolefin.

**17.** A composition according to claim 16, in which the polyolefin is polyethylene or polypropylene.

18. A composition according to claim 14, which, in addition to the compound of the formula (I), also comprises other conventional additives for synthetic polymers.

19. Use of a compound according to claim 1 for stabilising an organic material against thermal, oxidative and/or light-induced degradation.

**Revendications**

1. Composés de formule I :

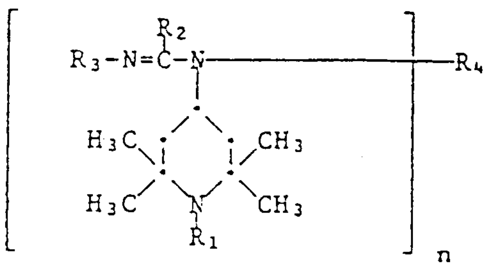

(I)

dans laquelle $R_1$ désigne l'hydrogène, O•, CN, NO, un cyanométhyle, $C_1$-$C_{12}$-alkyle, $C_3$-$C_{12}$-alcényle ou $C_3$-$C_{12}$-alcynyle si l'atome de carbone qui est lié à l'atome d'azote est un atome de carbone primaire, un $C_7$-$C_{12}$-aralkyle, méthylbenzyle, t-butylbenzyle, hydroxybenzyle, $C_1$-$C_{12}$-acyle, 2,3-époxypropyle, un $C_2$-$C_6$-alkyle avec un groupe OH ou un 2,3-dihydroxypropyle, $R_2$ est l'hydrogène, un $C_1$-$C_{18}$-alkyle, $C_5$-$C_{18}$-cycloalkyle, méthylcyclohexyle, triméthylcyclohexyle, t-butylcyclohexyle, $C_6$-$C_{18}$-aryle, méthylphényle, diméthylphényle, triméthylphényle, t-butylphényle, méthoxyphényle, éthoxyphényle, hydroxyphényle, 3,5-di-tert-butyl-4-hydroxyphényle, $C_7$-$C_{18}$-aralkyle, méthylbenzyle, hydroxybenzyle, 3,5-di-tert-butyl-4-hydroxybenzyle ou 2-(3,5-di-tert-butyl-4-hydroxyphényl)-éthyle, $R_3$ un groupe $C_6$-$C_{18}$-aryle, méthylphényle, diméthylphényle, triméthylphényle, éthylphényle, diéthylphényle, hydroxyphényle, 3,5-di-tert-butyl-4-hydroxyphényle, méthoxyphényle, éthoxyphényle, 4-éthoxycarbonylphényle, 4-(2,2,6,6-tétraméthyl-4-pipéridyloxycarbonyl)-phényle, 4-(1,2,2,6,6-pentaméthyl-4-pipéridyloxycarbonyl)-phényle ou un groupe de formule II

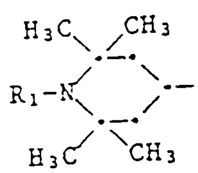

(II)

$R_1$ ayant la signification ci-dessus, n est un entier de 1 à 4 et, si n = 1, $R_4$ est l'hydrogène ou un groupe
CN, $-COR_5$, $-COOR_6$, $-CO-R_7-COOR_8$,

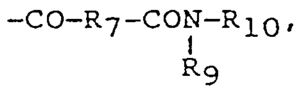, 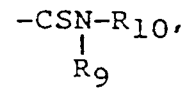,

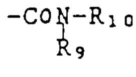, $-SO_2R_{11}$, 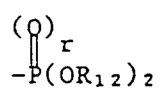 ou 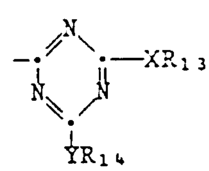

$R_5$ étant l'hydrogène ou un groupe $C_1$-$C_{18}$-alkyle, $C_5$-$C_{18}$-cycloalkyle, méthylcyclohexyle, t-butylcyclohexyle, $C_2$-$C_{18}$-alcényle, $C_6$-$C_{18}$-aryle, méthylphényle, diméthylphényle, triméthylphényle, éthylphényle, t-butylphényle, méthoxyphényle, éthoxyphényle, hydroxyphényle, 3,5-di-tert-butyl-4-hydroxyphényle, $C_7$-$C_{18}$-aralkyle, 3,5-di-tert-butyl-4-hydroxybenzyle, 2-(3,5-di-tert-butyl-4-hydroxyphényl)-éthyle ou bien un $C_1$-$C_{10}$-alkyle avec comme substituant un $C_1$-$C_{18}$-alcoxy, un $C_2$-$C_{18}$-dialkylamino ou un groupe de formule III :

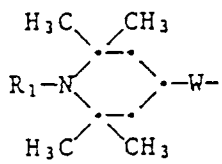

(III)

$R_1$ ayant la signification ci-dessus indiquée et W représentant -O- ou $>$N-$R_{15}$, $R_{15}$ étant l'hydrogène ou un groupe $C_1$-$C_{18}$-alkyle, $C_5$-$C_{18}$-cycloalkyle, méthylcyclohexyle, diméthylcyclohexyle, triméthylcyclohexyle, $C_3$-$C_{18}$-alcényle, $C_6$-$C_{18}$-aryle, méthylphényle, diméthylphényle, triméthylphényle, t-butylphényle, méthoxyphényle, éthoxyphényle, hydroxyphényle, 3,5-di-tert-butyl-4-hydroxyphényle, $C_7$-$C_{18}$-aralkyle, méthylbenzyle, hydroxybenzyle, 3,5-di-tert-butyl-4-hydroxybenzyle ou un groupe de formule II, $R_6$ est un $C_1$-$C_{18}$-alkyle, un $C_2$-$C_6$-alkyle substitué par un groupe OH, $C_1$-$C_{18}$-alcoxy ou $C_2$-$C_{18}$-dialkylamino, un $C_5$-$C_{18}$-cycloalkyle, méthylcyclohexyle, diméthylcyclohexyle, triméthylcyclohexyle, t-butylcyclohexyle, $C_3$-$C_{18}$-alcényle, $C_6$-$C_{18}$-aryle, méthylphényle, diméthylphényle, éthylphényle, isopropylphényle, t-butylphényle, di-t-butylphényle, 2,6-di-tert-butyl-4-méthylphényle, méthoxyphényle, éthoxyphényle, $C_7$-$C_{18}$-aralkyle, méthylbenzyle, 3,5-di-tert-butyl-4-hydroxybenzyle ou un groupe de formule II et $R_7$ représente une liaison directe, un $C_1$-$C_{18}$-alkylène, $C_2$-$C_{20}$-alkylidène, $C_7$-$C_{20}$-aralkylidène, $C_6$-$C_{10}$-cycloalkylène, méthylcyclohexylène, $C_2$-$C_{18}$-alcénylène ou $C_6$-$C_{10}$-arylène, $R_8$ a l'une des significations indiquées pour $R_6$ ou bien désigne l'hydrogène ou un métal alcalin, $R_9$ et $R_{10}$, qui peuvent être identiques ou différents l'un de l'autre, ont l'une des significations indiquées pour $R_{15}$, ou bien forment ensemble et avec l'atome d'azote auquel ils sont liés un groupe 1-pyrrolidinyle, 1-pipéridyle, 4-morpholinyle, 1-hexahydroazépinyle ou 4-méthyl-1-pipérazinyle, $R_{11}$ est un groupe $C_1$-$C_{12}$-alkyle, $C_6$-$C_{12}$-aryle, méthylphényle, diméthylphényle, triméthylphényle, méthoxyphényle ou éthoxyphényle, r = 0 ou 1 et $R_{12}$ est un alkyle en $C_1$-$C_{12}$ ou un groupe de formule II ci-dessus ou bien les deux groupes $R_{12}$ forment ensemble un alkylène en $C_2$-$C_{12}$ ou un groupe

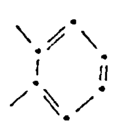 , 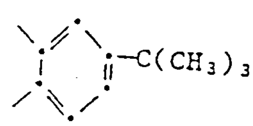 ou 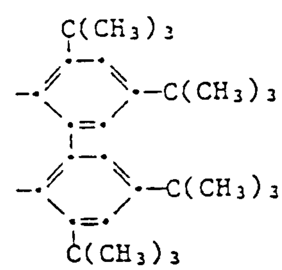

et avec l'atome de phosphore et les deux atomes d'oxygène un cycle pentagonal à heptagonal,
$R_{13}$ et $R_{14}$, qui peuvent être identiques ou différents l'un de l'autre, ont l'une des significations qui ont été données pour $R_6$ ou bien représentent chacun l'hydrogène, et X et Y, qui peuvent être également identiques ou différents l'un de l'autre, représentent une liaison directe, un atome d' oxygène ou un groupe $>$N-$R_{16}$, $R_{16}$ ayant l'une des significations indiquées pour $R_{15}$ ou étant un $C_2$-$C_6$-alkyle avec un groupe OH, ou bien les groupes $R_{13}$X- et $R_{14}$Y-, qui peuvent être identiques ou différents l'un de l'autre, sont chacun un groupe 1-pyrrolidinyle, 1-pipéridyle, 4-morpholinyle, 1-hexahydroazépinyle ou 4-méthyl-1-pipérazinyle,
si n = 2,
$R_4$ est un groupe -CO-, $-SO_2$-, -COO-$R_{17}$-OOC-, -COCOO-$R_{17}$-OOCCO-, -CO-$R_{18}$-CO-,

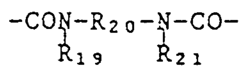 , 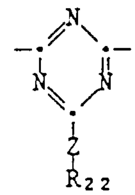 , 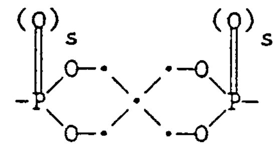 ,

$(-CO-R_7-COO)_2Me$ ou 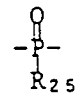 ,

$R_{17}$ étant un $C_2$-$C_{18}$-alkylène, un $C_4$-$C_{12}$-alkylène dont la chaîne est interrompue par un ou deux atomes d'oxygène, un $C_6$-$C_{18}$-cycloalkylène, cyclohexylène-diméthylène, méthylènedicyclohexylène, isopropylidène-dicyclohexylène, $C_4$-$C_8$-alcénylène,$C_6$-$C_{18}$-arylène, $C_8$-$C_{12}$-aralkylène ou encore un groupe de formule IV :

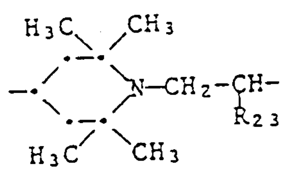

(IV)

$R_{23}$ désignant l'hydrogène, un $C_1$-$C_4$-alkyle ou un phényle, et $R_{18}$ représentant une liaison directe ou un $C_1$-$C_{18}$-alkylène, $C_2$-$C_{20}$-alkylidène, $C_7$-$C_{20}$-aralkylène, $C_6$-$C_{10}$-cycloalkylène, méthylcyclohexylène, $C_6$-$C_{10}$-arylène ou un $C_2$-$C_{12}$-alkylène dont la chaîne est interrompue par un ou deux atomes d'oxygène ou par un ou deux groupes $>N-R_{24}$, $R_{24}$ ayant l'une des significations qui ont été indiquées pour $R_{15}$, $R_{19}$ et $R_{21}$, qui peuvent être identiques ou différents l'un de l'autre, ont l'une des significations indiquées pour $R_{15}$, $R_{20}$ est un $C_2$-$C_{18}$-alkylène, $C_6$-$C_{18}$-cycloalkylène, cyclohexylène-diméthylène, méthylènedicyclohexylène, $C_6$-$C_{18}$-arylène ou $C_8$-$C_{12}$-aralkylène, $R_{22}$ a l'une des significations qui ont été indiquées pour $R_{13}$ et $R_{14}$ et Z l'une des significations indiquées pour X et Y ou bien $-ZR_{22}$ représente un groupe 1-pyrrolidinyle, 1-pipéridyle, 4-morpholinyle, 1-hexahydroazépinyle ou 4-méthyl-1-pipérazinyle ou un atome d'halogène, $R_{25}$ est un $C_1$-$C_4$-alkyle et s le nombre 0 ou 1, $R_7$ a la signification précédemment indiquée, Me désigne Ba, Ca, Co", Mg, Mn", Ni", Sn" ou Zn,

si n = 3, $R_4$ est un triacyle aliphatique en $C_4$-$C_{18}$, un triacyle de l'acide citrique, un triacyle aliphatique en $C_6$-$C_{18}$ avec un atome d'azote ou encore un triacyle de l'acide 1,2,4-benzène-tricarboxylique ou 1,3,5-benzènetricarboxylique, un groupe

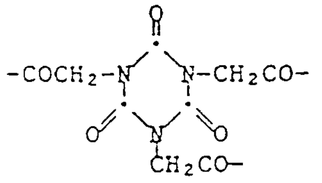 ,

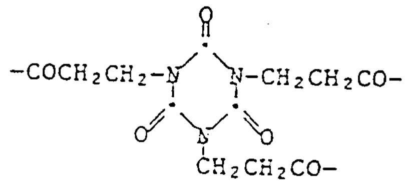 ,

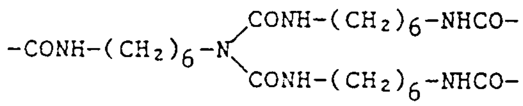 ou

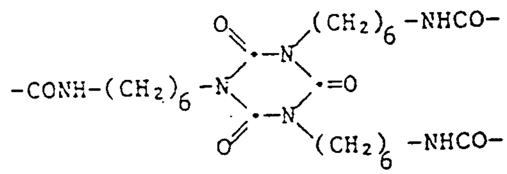

ou un groupe 1,3,5-triazine-2,4,6-triyle, ≡P-, ≡PO- ou $(-CO-R_7-COO)_3Al$, $R_7$ ayant la signification précédemment donnée,
et enfin si n = 4, $R_4$ est un tétraacyle aliphatique en $6_6$-$C_{18}$, un tétraacyle aliphatique en $C_{10}$-$C_{18}$ portant deux atomes d'azote, un tétraacyle aromatique en $C_{10}$-$C_{18}$, un tétraacyle cycloaliphatique en $C_{10}$-$C_{22}$ ou un groupe $(-CO-R_7-COO)_4Sn^{IV}$, $R_7$ ayant la signification précédemment donnée.

**2.** Composés selon la revendication 1 dans lesquels $R_1$ est l'hydrogène ou le groupe méthyle, allyle, benzyle ou acétyle, $R_2$ l'hydrogène, un $C_1$-$C_{12}$-alkyle, un $C_6$-$C_9$-cycloalkyle ou un $C_6$-$C_9$-aryle, $R_3$ un un $C_6$-$C_{12}$-aryle ou un groupe de formule II ci-dessus, n le nombre 1, 2 ou 3, et si n = 1, $R_4$ est l'hydrogène ou un groupe $-COR_5$, $-COOR_6$, $-CO-R_7-COOR_8$,

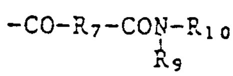 , 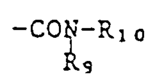 ,

$-SO_2R_{11}$ , ou 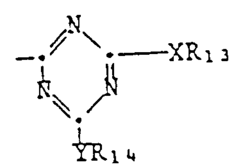

$R_5$ désignant l'hydrogène, un $C_1$-$C_{18}$-alkyle, $C_6$-$C_9$-cycloalkyle, $C_2$-$C_4$-alcényle, $C_6$-$C_8$-aryle, $C_7$-$C_{16}$-aralkyle ou bien un $C_1$-$C_3$-alkyle substitué par un groupe de formule III ci-dessus, W désignant l'oxygène ou un groupe $>N-R_{15}$ et $R_{15}$ l'hydrogène, un $C_1$-$C_{12}$-alkyle ou un $C_6$-$C_9$-cycloalkyle, $R_6$ étant un $C_1$-$C_{18}$-alkyle ou un $C_2$-$C_6$-alkyle substitué par un $C_1$-$C_4$-alcoxy ou encore un $C_6$-$C_{12}$-cycloalkyle ou un groupe de formule II, $R_7$ représentant une liaison directe, un $C_1$-$C_{10}$-alkylène, un $C_6$-$C_8$-cycloalkylène ou le groupe vinylène ou phénylène, $R_8$ l'hydrogène, un $C_1$-$C_{12}$-alkyle, un $C_6$-$C_9$-cycloalkyle, un allyle ou un groupe de formule II, $R_9$ et $R_{10}$, identiques ou différents l'un de l'autre, étant l'hydrogène, un $C_1$-$C_{12}$-alkyle, un $C_6$-$C_9$-cycloalkyle, un allyle ou un groupe de formule II ou bien formant ensemble et avec l'atome d'azote auquel ils sont liés un groupe 1-pyrrolidinyle, 1-pipéridyle, 4-morpholinyle, 1-hexahydroazépinyle ou 4-méthyl-1-pipérazinyle, $R_{11}$ étant le groupe méthyle ou un $C_6$-$C_9$-aryle, $R_{13}$ et $R_{14}$, identiques ou différents l'un de l'autre, l'hydrogène, un $C_1$-$C_{12}$-alkyle, un $C_6$-$C_9$-cycloalkyle, le groupe allyle ou un groupe de formule II, et X et Y, identiques ou différents l'un de l'autre, représentent un atome d'oxygène ou un groupe $>N-R_{16}$, $R_{16}$ étant l'hydrogène, un $C_1$-$C_{12}$-alkyle, un $C_2$-$C_4$-alkyle avec un groupe OH, un $C_6$-$C_9$-cycloalkyle, un allyle ou un groupe de formule II, ou bien les groupes $R_{13}X$- et $R_{14}Y$-, identiques ou différents l'un de l'autre, sont un groupe 1-pyrrolidinyle, 1-pipéridyle, 4-morpholinyle ou 1-hexahydroazépinyle,
si n = 2, $R_4$ est un groupe -CO-, $COO-R_{17}-OOC-$, $-CO-R_{18}-CO-$,

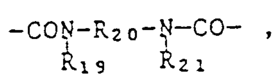 , ou 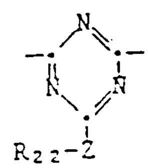

$R_{17}$ étant un $C_2$-$C_{12}$-alkylène, un $C_4$-$C_{12}$-alkylène à chaîne interrompue par un ou deux atomes d'oxygène, un $C_6$-$C_{15}$-cycloalkylène, le groupe 2-butène-1,4-diyle ou un groupe de formule IV, $R_{23}$ étant l'hydrogène ou le groupe méthyle, $R_{18}$ représentant une liaison directe, un $C_1$-$C_{12}$-alkylène, un $C_2$-$C_{12}$-alkylidène, un $C_2$-$C_8$-alkylène à chaîne interrompue par un ou deux groupes $>N-R_{24}$, $R_{24}$ désignant un $C_1$-$C_8$-alkyle, un $C_6$-$C_9$-cycloalkyle ou un groupe de formule II, $R_{19}$ et $R_{21}$, identiques ou différents l'un de l'autre, étant l'hydrogène, un $C_1$-$C_8$-alkyle, un $C_6$-$C_9$-cycloalkyle ou un groupe de formule II, $R_{20}$ un $C_2$-$C_{10}$-alkylène, un $C_6$-$C_{15}$-cycloalkylène, un $C_6$-$C_{15}$-arylène ou un xylylène, $R_{22}$ l'hydrogène, un $C_1$-$C_{12}$-alkyle, $C_2$-$C_4$-alkyle avec un groupe OH, un $C_6$-$C_9$-cycloalkyle, un allyle ou un groupe de formule II, et Z désignant l'oxygène ou un groupe $>N-R_{16}$, $R_{16}$ ayant la signification ci-dessus, ou bien -$ZR_{22}$ désignant un halogène, et $R_{25}$ étant un $C_1$-$C_4$-alkyle, et

si n = 3, $R_4$ est un $C_4$-$C_8$-triacyle aliphatique, un groupe $N(CH_2CO-)_3$, un triacyle provenant de l'acide 1,2,4-benzène-tricarboxylique ou 1,3,5-benzène-tricarboxylique ou encore un groupe

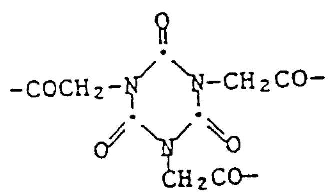 ou 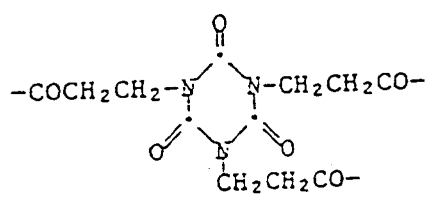

ou un groupe 1,3,5-triazine-2,4,6-triyle.

**3.** Composés selon la revendication 1 dans lesquels $R_1$ et $R_2$ sont chacun l'hydrogène ou le groupe méthyle, $R_3$ est un groupe 2,2,6,6-tétraméthyl-4-pipéridyle ou 1,2,2,6,6-pentaméthyl-4-pipéridyle, n est le nombre 1, 2 ou 3 et, si n = 1, $R_4$ est l'hydrogène ou l'un des groupes -$COR_5$, -$COOR_6$, -$COCOOR_8$,

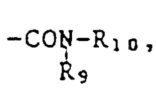 , 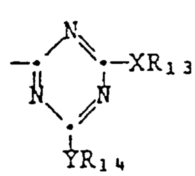

$R_5$ étant un $C_1$-$C_{17}$-alkyle, un cyclohexyle ou un phényle, $R_6$ étant un $C_2$-$C_{18}$-alkyle, un $C_6$-$C_{10}$-cycloalkyle ou un groupe de formule II dans lequel $R_1$ est l'hydrogène ou le groupe méthyle, $R_8$ un $C_2$-$C_{12}$-alkyle, un cyclohexyle ou un groupe de formule II dans lequel $R_1$ est l'hydrogène ou le groupe méthyle, $R_9$ l'hydrogène, $R_{10}$ un $C_{14}$-$C_{12}$-alkyle ou un cyclohexyle, $R_{13}$ et $R_{14}$, qui peuvent être identiques ou différents l'un de l'autre, un $C_1$-$C_{12}$-alkyle, un cyclohexyle, un allyle ou un groupe de formule II dans lequel $R_1$ est l'hydrogène ou le groupe méthyle, et X et Y, qui peuvent être identiques ou différents, étant chacun un groupe $>N-R_{16}$, dans lequel $R_{16}$ est l'hydrogène, un $C_1$-$C_{12}$-alkyle, un cyclohexyle, un allyle ou un groupe de formule II, dans lequel $R_1$ est l'hydrogène ou le groupe méthyle,

si n = 2, $R_4$ est l'un des groupes -$COO-R_{17}-OOC$-, -$CO-R_{18}-CO$-,

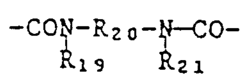 , 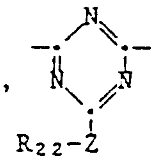

$R_{17}$ étant un $C_4$-$C_{10}$-alkylène, un $C_6$-$C_8$-cycloalkylène ou un groupe de formule IV dont $R_{23}$ est l'hydrogène, $R_{18}$ une liaison directe, un $C_1$-$C_8$-alkylène, un $C_2$-$C_8$-alkylidène, un cyclohexylène ou un phénylène, $R_{19}$ et $R_{21}$ étant l'hydrogène, $R_{20}$ un $C_6$-$C_9$-alkylène ou un $C_6$-$C_{13}$-arylène, $R_{22}$ un $C_1$-$C_{12}$-alkyle, un cyclohexyle, un allyle ou un groupe de formule II dans lequel $R_1$ est l'hydrogène ou le groupe méthyle, et Z désignant un atome d'oxygène ou un groupe $>$N-$R_{16}$, dont $R_{16}$ a la signification précédemment donnée, et si n = 3, $R_4$ est un groupe 1,3,5-triazine-2,4,6-triyle.

**4.** Composés selon la revendication 1 dans lesquels $R_1$ est l'hydrogène, un alkyle en $C_1$-$C_4$ ou le groupe allyle, benzyle ou acétyle.

**5.** Composés selon la revendication 1 dans lesquels $R_2$ est l'hydrogène, un alkyle en $C_1$-$C_4$, un cyclohexyle, un phényle ou un benzyle et $R_3$ un phényle, un groupe 4-éthoxycarbonylphényle ou un groupe de formule II indiquée à la revendication 1, dont $R_1$ a la signification donnée à cette revendication.

**6.** Composés selon la revendication 1 dans lesquels $R_1$ est l'hydrogène ou le groupe méthyle, $R_2$ est l'hydrogène ou un alkyle en $C_1$-$C_4$ et $R_3$ un groupe 4-éthoxycarbonylphényle ou un groupe de formule II indiquée à la revendication 1, $R_1$ ayant la signification indiquée.

**7.** Composés selon la revendication 1 dans lesquels n est le nombre 1 ou 2.

**8.** Composés selon la revendication 1 dans lesquels n = 1 et $R_4$ est un groupe -$COR_5$, -$COOR_6$ ou

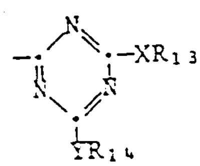

**9.** Composés selon la revendication 1 dans lesquels n = 2 et $R_4$ est un groupe -COO-$R_{17}$-OOC, -CO-$R_{18}$-CO- ou

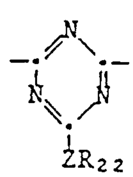

**10.** Composés selon la revendication 1 dans lesquels n est le nombre 1, 2 ou, 3 si n = 1, $R_4$ est l'hydrogène ou un groupe -$COR_5$, -$COOR_6$, -CO-$R_7$-$COOR_8$,

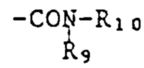 ou 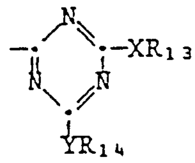

$R_5$ étant un $C_1$-$C_{18}$-alkyle, $R_6$ un $C_1$-$C_{18}$-alkyle, un $C_2$-$C_6$-alkyle substitué par un $C_1$-$C_4$-alcoxy, un cyclohexyle, un méthylcyclohexyle, un diméthylcyclohexyle, un triméthylcyclohexyle, un t-butylcyclohexyle ou un groupe de formule II, $R_7$ un $C_1$-$C_{10}$-alkylène, $R_8$ l'hydrogène, $R_9$ et $R_{10}$, identiques ou différents l'un de l'autre, étant l'hydrogène, un $C_1$-$C_4$-alkyle ou un cyclohexyle, $R_{13}$ et $R_{14}$, identiques ou différents l'un de l'autre, étant l'hydrogène, un $C_1$-$C_8$-alkyle, un allyle ou un groupe de formule II, et X et Y, identiques ou différents l'un de l'autre, étant un atome d'oxygène ou un groupe $>$N-$R_{16}$, $R_{16}$ désignant l'hydrogène, un $C_1$-$C_8$-alkyle, un $C_2$-$C_4$-alkyle avec un groupe OH, un allyle ou un groupe de formule II, si n = 2, $R_4$ est un groupe -CO-, COO-$R_{17}$-OOC-, -CO-$R_{18}$-CO-,

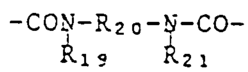, 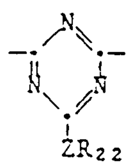 ou 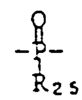

$R_{17}$ étant un $C_2$-$C_{12}$-alkylène ou un $C_4$-$C_{12}$-alkylène dont la chaîne est interrompue par un ou deux atomes d'oxygène, $R_{18}$ une liaison directe ou un $C_1$-$C_{10}$-alkylène, $R_{19}$ et $R_{21}$ l'hydrogène, $R_{20}$ un $C_2$-$C_{10}$-alkylène ou un cyclohexylène, $R_{22}$ un $C_1$-$C_8$-alkyle, un $C_2$-$C_4$-alkyle avec un groupe OH ou un allyle et Z un atome d'oxygène ou un groupe $>$N-$R_{16}$, dont $R_{16}$ a la signification précédemment indiquée, ou encore -Z$R_{22}$ étant un halogène, et $R_{25}$ étant un $C_1$-$C_4$-alkyle, alors que si n = 3, $R_4$ est un groupe 1,3,5-triazine-2,4,6-triyle.

**11.** Composés selon la revendication 1 dans lesquels $R_1$ est l'hydrogène, $R_2$ l'hydrogène ou le groupe méthyle, $R_3$ le groupe 2,3,6,6-tétraméthyl-4-pipéridyle, n le nombre 1, 2 ou 3 et si n = 1, $R_4$ est un groupe -CO$R_5$, -COO$R_6$ ou

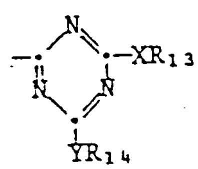

$R_5$ étant un $C_1$-$C_{18}$-alkyle, $R_6$ un $C_1$-$C_{18}$-alkyle, un cyclohexyle, le groupe 4-t-butylcyclohexyle ou 2,2,6,6-pentaméthyl-4-pipéridyle, $R_{13}$ et $R_{14}$, identiques ou différents l'un de l'autre, sont un $C_1$-$C_8$-alkyle ou un allyle et X et Y, identiques ou différents l'un de l'autre, un atome d'oxygène ou un groupe $>$N-$R_{16}$, dont $R_{16}$ est l'hydrogène, un $C_1$-$C_8$-alkyle, un allyle ou le groupe 2,3,6,6-tétraméthyl-4-pipéridyle, alors que si n = 2, $R_4$ est un groupe COO-$R_{17}$-OOC-, -CO-$R_{18}$-CO- ou

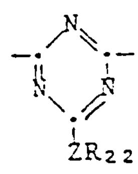

$R_{17}$ étant un $C_4$-$C_6$-alkylène, $R_{18}$ une liaison directe ou un $C_1$-$C_8$-alkylène, $R_{22}$ un $C_1$-$C_8$-alkyle ou un allyle et Z un atome d'oxygène ou un groupe $>$N-$R_{16}$, $R_{16}$ ayant l'une des significations précédemment indiquées, et si n = 3, $R_4$ est un groupe 1,3,5-triazine-2,4,6-triyle.

**12.** Composés selon la revendication 1 dans lesquels $R_1$ est l'hydrogène, $R_2$ l'hydrogène ou le groupe méthyle, $R_3$ le groupe 2,2,6,6-tétraméthyl-4-pipéridyle, n le nombre 1 ou 2 et si n = 1, $R_4$ est un groupe $-COOR_6$ ou

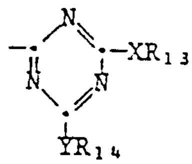

$R_6$ étant un $C_4$-$C_{18}$-alkyle, un cyclohexyle, le groupe 4-t-butylcyclohexyle ou un 2,2,6,6-pentaméthyl-4-pipéridyle, $R_{13}$ et $R_{14}$, indépendamment l'un de l'autre, sont un $C_1$-$C_4$-alkyle, et X et Y un groupe $>$N-$R_{16}$, dont $R_{16}$ est le groupe 2,2,6,6-tétraméthyl-4-pipéridyle, alors que si n = 2, $R_4$ est un groupe COO-$R_{17}$-OOC- ou

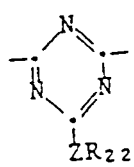

$R_{17}$ étant un $C_4$-$C_6$-alkylène, $R_{22}$ un $C_1$-$C_4$-alkyle ou un allyle et Z un atome d'oxygène ou $>$N-$R_{16}$, $R_{16}$ désignant l'hydrogène, un $C_1$-$C_4$-alkyle ou un allyle.

**13.** Les composés suivants

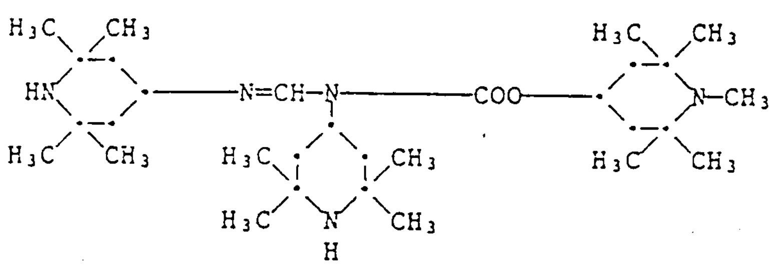

,

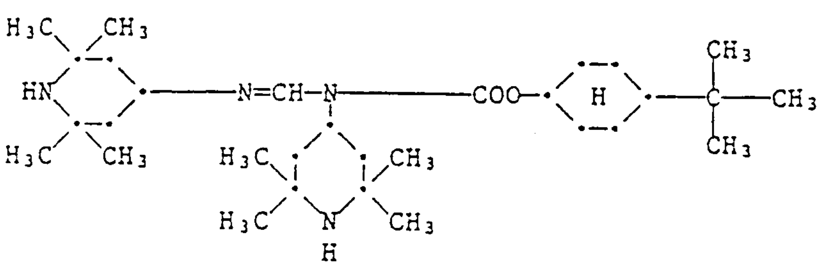

,

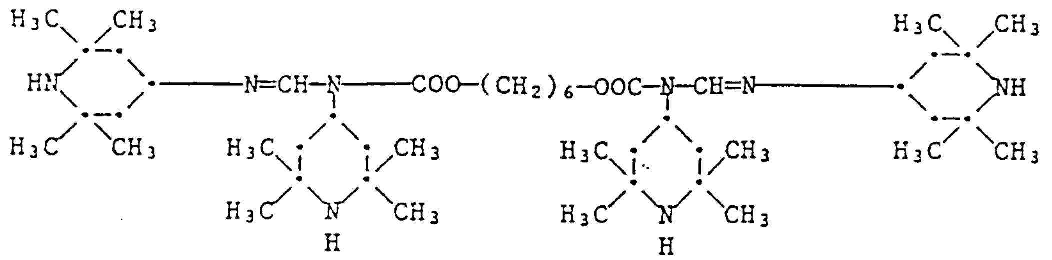

,

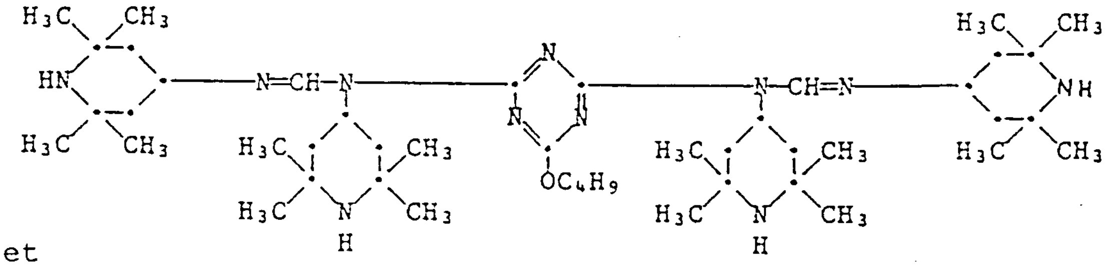

et

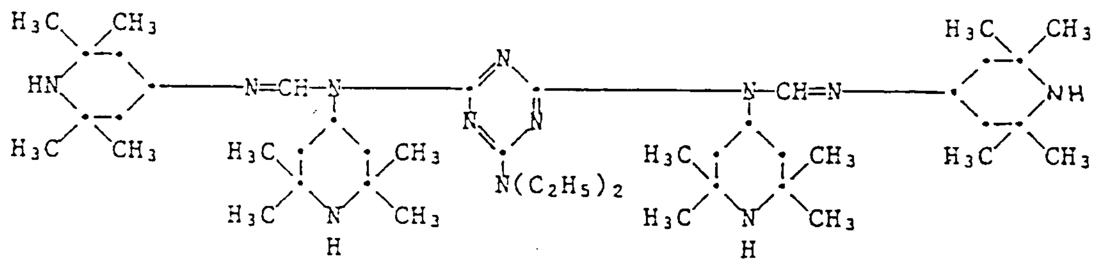

à titre de composés selon la revendication 1.

**14.** Composition comprenant une matière organique sujette à se dégrader par oxydation ou sous l'action de la chaleur et/ou de la lumière, avec un ou plusieurs composés de la revendication 1.

**15.** Composition selon la revendication 14 dont la matière organique est une matière polymère synthétique.

**16.** Composition selon la revendication 14 dont la matière organique est une polyoléfine.

**17.** Composition selon la revendication 16 dans laquelle la polyoléfine est du polyéthylène ou du polypropylène.

**18.** Composition selon la revendication 14 contenant en outre d'autres additifs courants pour des matières polymères synthétiques.

**19.** L'emploi de composés de la revendication 1 pour stabiliser et protéger des matières organiques contre les dégradations sous l'action de la chaleur et/ou de la lumière ou par oxydation.